(19) Europäisches Patentamt
European Patent Office
Office européen des brevets

(11) EP 1 539 996 B1

(12) **EUROPEAN PATENT SPECIFICATION**

(45) Date of publication and mention of the grant of the patent:
**09.03.2011 Bulletin 2011/10**

(21) Application number: **03735170.7**

(22) Date of filing: **02.07.2003**

(51) Int Cl.:
***C12Q 1/68*** *(2006.01)*

(86) International application number:
**PCT/AU2003/000854**

(87) International publication number:
**WO 2004/005555 (15.01.2004 Gazette 2004/03)**

# (54) METHOD OF PRODUCING PLANTS HAVING ENHANCED TRANSPIRATION EFFICIENCY AND PLANTS PRODUCED THEREFROM

VERFAHREN ZUR PRODUKTION VON PFLANZEN MIT VERBESSERTER TRANSPIRATIONSEFFIZIENZ SOWIE DAVON PRODUZIERTE PFLANZEN

PROCEDE DE PRODUCTION DE PLANTES PRESENTANT UNE EFFICACITE DE TRANSPIRATION AMELIOREE ET PLANTES OBTENUES AU MOYEN DE CE PROCEDE

(84) Designated Contracting States:
**AT BE BG CH CY CZ DE DK EE ES FI FR GB GR HU IE IT LI LU MC NL PT RO SE SI SK TR**

(30) Priority: **02.07.2002 AU PS333902**

(43) Date of publication of application:
**15.06.2005 Bulletin 2005/24**

(73) Proprietor: **THE AUSTRALIAN NATIONAL UNIVERSITY**
**Canberra, ACT 2601 (AU)**

(72) Inventors:
- **MASLE, Josette**
**Queanbeyan, New South Wales 2620 (AU)**
- **FARQUHAR, Graham, Douglas**
**Queanbeyan, New South Wales 2620 (AU)**
- **GILMORE, Scott, Robert**
**Downer, Australian Capital Territory 260 (AU)**

(74) Representative: **Dempster, Robert Charles et al**
**D Young & Co LLP**
**120 Holborn**
**London EC1N 2DY (GB)**

(56) References cited:
**WO-A-01/02541 US-A1- 2002 040 489**

- **THUMMA B. R. ET AL: "Identification of causal relationships among traits related to drought resistance in Stylosanthes scabra using QTL analysis" JOURNAL OF EXPERIMENTAL BOTANY, vol. 52, no. 355, February 2001 (2001-02), pages 203-214, XP002397857**
- **HERVÉ D ET AL.: "QTL analysis of photosynthesis and water status traits in sunflower (Helianthus annuus L.) under greenhouse conditions" JOURNAL OF EXPERIMENTAL BOTANY, vol. 52, no. 362, September 2001 (2001-09), pages 1857-1864, XP002397858**
- **DATABASE GENBANK [Online] 16 October 2002 HAINEY C.F. ET AL., XP003003454 Database accession no. (AY106598)**
- **RAUH B.L. ET AL.: 'Quantitative trait loci analysis of growth response to varying nitrogen soueces in arabidopsis thaliana' THEOR. APPL. GENET. vol. 104, 2002, pages 743 - 750, XP008046246**
- **TORIL K.E. ET AL.: 'The arabidopsis ERECTA gene encodes a putative receptor protein kinase with extracellular leucine-rich repeats' THE PLANT CELL vol. 8, 1996, pages 735 - 746, XP008046352**
- **DOUGLAS S.J. ET AL.: 'KNATI and ERECTA regulate inflorescence architecture in arabidopsis' THE PLANT CELL vol. 14, March 2002, pages 547 - 558, XP002217925**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to the field of plant breeding and the production of genetically engineered plants. More specifically, the invention described herein provides genes that are capable of enhancing the transpiration efficiency of a plant when expressed therein. These genes are particularly useful for the production of plants having enhanced transpiration efficiency, by both traditional plant breeding and genetic engineering approaches. The invention further extends to plants produced by the methods described herein.

### BACKGROUND TO THE INVENTION

#### 1. General

This specification contains nucleotide and amino acid sequence information prepared using PatentIn Version 3.1, presented herein after the claims. Each nucleotide sequence is identified in the sequence listing by the numeric indicator <210> followed by the sequence identifier (e.g. <210>1, <210>2, etc). The length and type of sequence (DNA, protein (PRT), etc), and source organism for each nucleotide sequence, are indicated by information provided in the numeric indicator fields <211>, <212> and <213>, respectively. Nucleotide sequences referred to in the specification are defined by the term "SEQ ID NO:", followed by the sequence identifier (eg. SEQ ID NO: 1 refers to the sequence in the sequence listing designated as <400>1).

The designation of nucleotide residues referred to herein are those recommended by the IUPAC-IUB Biochemical Nomenclature Commission, wherein A represents Adenine, C represents Cytosine, G represents Guanine, T represents thymine, Y represents a pyrimidine residue, R represents a purine residue, M represents Adenine or Cytosine, K represents Guanine or Thymine, S represents Guanine or Cytosine, W represents Adenine or Thymine, H represents a nucleotide other than Guanine, B represents a nucleotide other than Adenine, V represents a nucleotide other than Thymine, D represents a nucleotide other than Cytosine and N represents any nucleotide residue. As used herein the term "derived from" shall be taken to indicate that a specified integer is obtained from a particular source albeit not necessarily directly from that source.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

The present invention is not to be limited in scope by the specific embodiments described herein, which are intended for the purposes of exemplification only. Functionally equivalent products, compositions and methods are clearly within the scope of the invention, as described herein.

#### 2. Description of the related art

It is well known that virtually all plants require a certain quantity of water for proper growth and development, because $CO_2$ fixation and photosynthate assimilation by plants cost water. A significant quantity of water absorbed by plants from the soil returns to the atmosphere via plant transpiration.

Transpiration efficiency is a measure of the amount of dry matter produced by a plant per unit of water transpired, or, in other words, carbon gain relative to water lost through transpiration.

For plants having low transpiration efficiency, or when water is in short supply, the loss of water through transpiration can limit key metabolic processes associated with plant growth and development. For example, during drought, or when plants having low transpiration efficiency are grown in arid and semi-arid environments, plant productivity as determined by dry matter production or photosynthetic rate, is considerably reduced. Accordingly, the production of plants having enhanced water use efficiency or transpiration efficiency is highly desirable for their adaptation to arid or semi-arid conditions, or to enhance their drought resistance.

The enhancement of water use efficiency or transpiration efficiency by plants is also highly desirable in consideration of global climatic change and increasing pressure on world water resources. The inefficient utilization of agricultural water is known to impact adversely upon the supply of navigable water, potable water, and water for industrial or recreational use. Accordingly, the production of plants having enhanced transpiration efficiency is highly desirable for reducing the pressure on these water resources. It is also desirable for increasing plant productivity under well-watered conditions.

By enhancing transpiration efficiency, carbon gain rates are enhanced per unit of water transpired, thereby stimulating plant growth under well-watered conditions, or alternatively, under mild or severe drought conditions. This is achieved by enhancing carbon gain more than transpiration rate, or by reducing the amount of water lost at any particular rate of carbon fixation. Those skilled in the art also consider that for a given growth rate plants having enhanced transpiration efficiency dry out soils more slowly, and use less water, than less efficient near-isogenic plants.

Several chemical as well as environmental pre-treatments have been described for enhancing the ability of plant seedlings to survive drought, either by reducing transpiration or by reducing the amount of water that is actually lost to the atmosphere.

Known environmental treatments largely involve the use of physical barriers. Whilst placing a physical barrier over plant stomata is known to reduce water loss via transpiration, the procedure is not always desirable or practicable for field-grown crops. For example, physical barriers over plant stomata may inhibit certain gas- exchange processes of the plant. It is more desirable to enhance actual transpiration efficiency or water use efficiency of the plant through manipulation of intrinsic plant function.

Chemical agents are typically the so-called "anti-transpirant" or "anti-desiccant" agents, both of which are applied to the leaves. Anti-transpirants are typically films or metabolic anti-transpirants.

These products form a film on leaves, thereby either blocking stomatal pores, or coating leaf epidermal cells with a water-proof film. Typical film anti-transpirants include waxes, wax-oil emulsions, higher alcohols, silicones, plastics, latexes and resins. For example, Elmore, United States Patent No. 4,645,682 disclosed an anti-transpirant consisting of an aqueous paste wax; Cushman et al., United States Patent Nos. 3,791,839 and 3,847,641 also disclosed wax emulsions for controlling transpiration in plants; and Petrucco et al., United States Patent No. 3,826,671, disclosed a polymer composition said to be effective for controlling transpiration in plants.

Metabolic anti-transpirants generally close stomata, thereby reducing the rate of transpiration. Typical metabolic anti-transpirants include succinic acids, phenylmercuric acetate, hydroxysulfonates, the herbicide atrazine, sodium azide, and phenylhydrazones, as well as carbon cyanide.

Compounds having plant growth regulator activity have also been shown to be useful for reducing transpiration. For example, Bliesner *et al.,* United States Patent No. 4,671,816, disclosed an acetylene compound, said to possess utility for regulating plant growth, whilst Kuznetsov *et al.* (Russian Patent No. SU 1,282,492; ., Russian Patent Application No. SU 1,253,559-Al), and Smirnov *et al* (Russian Patent No. SU 1,098,934) disclosed the use of derivatives of 2-methyl-5-hydroxybenzimidazole, and the chloride or bromide salts thereof, as anti-transpirant growth regulators. Vichnevetskaia (USSN 5,589,437 issued December 31, 1996) also describe hydroxybenzimidazole derivatives for enhancing the drought resistance of plants by reducing transpiration. Schulz et al., United States Patent No. 4,943,315, also disclosed formulations comprising an acetylene and a phenylbenzylurea compound, for reducing transpiration in plants and/or for avoiding impairment to plants caused by heat and dry conditions. Abscisic acid has also been shown to reduce or suppress transpiration in plants (eg. Helv. Chim. Acta, 71, 931, 1988; *J. Org. Chem., 54,* 681, 1989; and Japanese Patent Publication No. 184,966/1991).

Metabolic anti-transpirants are costly to produce and often exhibit phytotoxic effects or inhibit plant growth (Kozlowski (1979), In: Tree Growth and Environmental Stresses (Univ. of Washington Press, Seattle and London)), and are not practically used.

Recent studies have examined alternative methods for enhancing transpiration efficiency, particularly breeding approaches to select lines that grow more efficiently under mild drought conditions. Carbon isotope discrimination has been used to identify Arabidopsis ecotypes with contrasted transpiration efficiencies (Masle et al., In: Stable isotopes and plant carbon-water relations, Acad. Press, Physiol. Ser., pp371-386, 1993) and to assist conventional breeding of new plant varieties in a number of species (Hall et al., Plant Breeding Reviews 4, 81-113, 1994) including rice (Farquhar et al., In: Breaking the Yield Barrier, ed KG Cassman, IRRI, 95, 101) and most recently wheat (Rebetzke et al. Crop Science 42:739-745, 2002).

No single gene has been identified as being capable of enhancing transpiration efficiency when expressed *in planta.* Transpiration efficiency may well be multigenic. As a consequence, the genes and signalling pathways that regulate the photosynthetic and/or stomatal components of the transpiration efficiency mechanism in plants have not been identified or characterized.

Moreover, notwithstanding that the effect of down-regulating expression of the *Rubisco* gene, or mutation in genes involved in abscisic acid (eg. *aba, abi*), are known to modify transpiration efficiency to some extent through stomatal closure, the consequence of such modifications is not necessarily specific, resulting in pleiotropic effects.

*Arabidopsis thaliana* ecotype Landsberg *erecta* (L-*er1*) is one of the most popular ecotypes and is used widely for both molecular and genetic studies. It harbors the er1 mutation, which confers a compact inflorescence, blunt fruits, and short petioles. There are a number of *erecta* mutant alleles. Phenotypic characterization of the mutant alleles suggests a role for the wild type *ER* gene in regulating plant morphogenesis, particularly the shapes of organs that originate from the shoot apical meristem. Torii et al., The Plant Cell 8, 735, 1996, showed that the ER gene encodes a putative receptor protein kinase comprising a cytoplasmic protein kinase catalytic domain, a transmembrane region, and an extracellular domain consisting of leucine-rich repeats, which are thought to interact with other macromolecules.

Genbank database accession no. AY106598 discloses a nucleotide sequence which is a partial sequence of an ERECTA gene from *Zea mays.* US 2002/040489 discloses expressed sequences from *Arabidopsis thaliana,* including a nucleotide sequence which is a partial sequence of an ERECTA mRNA. Thumma et al., Journal of Experimental Botany 52(355):203-214 (2001) discloses a quantitative trait loci analysis method to identify genetic markers associated with transpiration efficiency in *Stylosanthes scabra.* WO 01/02541 discloses a method of increasing or decreasing transcription in a plant by modulating the ABA-activated protein kinase (AAPK) gene, which is expressed in guard cells of the plant. Torii et al., The Plant Cell 8:735-746 (1996) discloses an *Arabidopsis* ERECTA sequence and mutants of this sequence having altered stem height.

## SUMMARY OF THE INVENTION

In work leading up to the present invention, the inventors sought to elucidate the specific genetic determinants of plant transpiration efficiency. In plants, the development of molecular genetic markers, such as, for example, genetic markers that map to a region of the genome of a crop plant, such as, for example, a region of the rice genome, maize genome, barley genome, sorghum genome, or wheat genome, or a region of the tomato genome or of any Brassicaceae, assists in the production of plants having enhanced transpiration efficiency (Edwards et al., Genetics 116, 113 - 125, 1987; Paterson et al., Nature 335, 721-726, 1988).

The present inventors identified a locus that is linked to the genetic variation in transpiration efficiency in plants. To elucidate a locus associated with the transpiration efficiency of plants, the inventors established experimental conditions and sampling procedures to determine the contribution to total transpiration efficiency of the factors influencing this phenotype, and, more particularly, the genetic contribution to the total variation in transpiration efficiency. Factors influencing transpiration efficiency include, for example, genotype of the plant, environment (eg. temperature, light, humidity, boundary layer around the leaves, root growth conditions), development (eg. age and/or stage and/or posture of plants that modify gas exchange and/or carbon metabolism), and seed-specific factors (Masle et al. 1993, op. cit). The screens developed by the inventors were also used to survey mutant and wild type populations for variations in transpiration efficiency and to identify ecotypes having contrasting transpiration efficiencies including the parental lines that had been used by Lister and Dean (1993). The transpiration efficiencies of the members of Lister and Dean's (1993) Recombinant Inbred Line (RIL) mapping population were then determined, and linkage analyses were performed against genetic markers to determine the chromosome regions that are linked to genetic variation in transpiration efficiency, thereby identifying a locus conditioning transpiration efficiency. Complementation tests, wherein plants were transformed with a wild-type allele at this locus confirmed the functionality of the allele in determining a transpiration efficiency phenotype.

Accordingly the present invention provides methods and uses as defined in the appended claims. Described herein is a locus associated with transpiration efficiency of *A. thaliana,* such as, for example the *ERECTA* locus on *A. thaliana* chromosome 2, or a hybridization probe which maps to the region between about 46cM and about 50.7cM on chromosome 2 of *A. thaliana.* The inventors identified additional *ERECTA* alleles or *erecta* alleles in *A. thaliana,* rice, sorghum, wheat and maize which are structurally related to this primary *A. thaliana ERECTA* or *erecta* allele. Based upon the large number of *ERECTA*/*erecta* alleles described herein, homologs of the *A. thaliana ERECTA* locus from other plant species to those specifically exemplified, can be identified using the methods described herein.

Described herein is a genetic marker or locus associated with the genetic variation in transpiration efficiency of a plant, wherein said locus comprises a nucleotide sequence linked genetically to an *ERECTA* locus in the genome of the plant. The locus or genetic marker is useful for determining transpiration efficiency of a plant.

As used herein, the terms "genetically linked" and "map to" shall be taken to refer to a sufficient genetic proximity between a linked nucleic acid comprising a gene, allele, marker or other nucleotide sequence and nucleic acid comprising all or part of an *ERECTA* locus to permit said linked nucleic acid to be useful for determining the presence of a particular allele of said *ERECTA* locus in the genome of a plant. Those skilled in the art will be aware that for such linked nucleic acid to be used in this manner, it must be sufficiently close to said locus not to be in linkage disequilibrium or to have a high recombination frequency between said linked nucleic acid and said locus. Preferably, the linked nucleic acid and the locus are less than about 25cM apart, more preferably less than about 10cM apart, even more preferably less than about 5cM apart, still even more preferably less than about 3cM apart and still even more preferably less than about 1cM apart.

Described herein is an isolated nucleic acid associated with the genetic variation in transpiration efficiency of a plant, said nucleic acid comprising a nucleotide sequence selected from the group consisting of:

(a) the sequence of an *ERECTA* genomic gene or the 5'-UTR or 3'-UTR or protein-encoding region or an intron region thereof;
(b) the sequence of an allelic variant of (a) or the 5'-UTR or 3'-UTR or protein-encoding region or an intron region of said allelic variant;
(c) the sequence of a fragment of (a) or (b) that hybridizes specifically to nucleic acid (eg., RNA or DNA) from a plant under at least low stringency hybridization conditions; and
(d) a sequence that is complementary to (a) or (b) or (c).

Described herein is an isolated *ERECTA* gene from wheat comprising a nucleotide sequence selected from the group consisting of:

(i) the sequence set forth in SEQ ID NO: 19;
(ii) a sequence encoding the amino acid sequence set forth in SEQ ID NO: 20; and
(iii) a sequence that is complementary to (i) or (ii).

Described herein is an isolated *ERECTA* gene from maize comprising a nucleotide sequence selected from the group consisting of:

(i) the sequence set forth in SEQ ID NO: 44;
(ii) a sequence encoding the amino acid sequence set forth in SEQ ID NO: 45; and
(iii) a sequence that is complementary to (i) or (ii).

Described herein is an isolated *ERECTA* gene from rice comprising a nucleotide sequence selected from the group consisting of:

(i) the sequence set forth in SEQ ID NO: 3;
(ii) a sequence encoding the amino acid sequence set forth in SEQ ID NO: 4; and
(iii) a sequence that is complementary to (i) or (ii).

Described herein is an isolated *ERECTA* gene from *A. thaliana* comprising a nucleotide sequence selected from the group consisting of:

(i) the sequence set forth in SEQ ID NO: 1;
(ii) a sequence encoding the amino acid sequence set forth in SEQ ID NO: 2; and
(iv) a sequence that is complementary to (i) or (ii).

Described herein is an isolated *ERECTA* gene from *A. thaliana* comprising a nucleotide sequence selected from the group consisting of:

(i) the sequence set forth in SEQ ID NO: 7;
(ii) a sequence encoding the amino acid sequence set forth in SEQ ID NO: 8; and
(v) a sequence that is complementary to (i) or (ii).

Described herein is an isolated *ERECTA* gene from *A. thaliana* comprising a nucleotide sequence selected from the group consisting of:

(i) the sequence set forth in SEQ ID NO: 9;
(ii) a sequence encoding the amino acid sequence set forth in SEQ ID NO: 10; and
(vi) a sequence that is complementary to (i) or (ii).

Described herein is an isolated *ERECTA* gene from sorghum comprising a nucleotide sequence selected from the group consisting of:

(i) the sequence set forth in SEQ ID NO: 5;
(ii) a sequence encoding the amino acid sequence set forth in SEQ ID NO: 6; and
(vii) a sequence that is complementary to (i) or (ii).

Notwithstanding that an *ERECTA* or *erecta* structural gene or genomic gene or the protein encoding region thereof is particularly useful for breeding and/or mapping purposes, this aspect is not to be limited to the *ERECTA* or

*erecta* structural or genomic gene or the protein-encoding region thereof. As exemplified herein, the primary *A. thaliana ERECTA* locus can be determined using any linked nucleic acid that maps to a region in the chromosome at a genetic distance of up to about 3cM from the *ERECTA* or *erecta* allele. The skilled artisan will readily be able to utilize similar probes to identify linkage to an *ERECTA* or *erecta* allele in any other plant species, based upon the teaching provided herein that the *ERECTA* or *erecta* allele is linked to the transpiration efficiency phenotype of plants.

Preferably, all or part of the locus associated with the transpiration efficiency phenotype in a plant (ie., nucleic acid genetically linked to the *ERECTA* or *erecta* structural or genomic gene) is provided as recombinant or isolated nucleic acid, such as, for example, in the form of a gene construct (eg. a recombinant plasmid or cosmid), to facilitate germplasm screening.

The *ERECTA* locus or a gene that is linked to the *ERECTA* locus is particularly useful in a breeding program, to predict the transpiration efficiency of a plant, or alternatively, as a selective breeding marker to select plants having enhanced transpiration efficiency. Once mapped, marker-assisted selection (MAS) is used to introduce the *ERECTA* locus or markers linked thereto into a wide variety of populations. MAS has the advantage of reducing the breeding population size required, and the need for continuous recurrent testing of progeny, and the time required to develop a superior line.

Accordingly, one aspect of the present invention provides a method of selecting a plant having enhanced transpiration efficiency, comprising detecting a genetic marker for transpiration efficiency which marker comprises an *ERECTA* locus in the genome of the plant and selecting a plant that comprises or expresses the genetic marker, wherein the genetic marker comprises a nucleotide sequence having at least about 55% overall sequence identity to at least about 20 nucleotides in length of any one of SEQ ID Nos: 1, 3, 5, 7, 9, 11 to 19 or 21 to 44 or a complementary sequence thereto, including a nucleotide sequence selected from the group consisting of:

(a) a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43, SEQ ID NO: 44;
(b) a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45; and
(c) a sequence complementary to (a) or (b).

In one embodiment, the method comprises:

having enhanced transpiration efficiency, comprising:

(a) screening mutant or near-isogenic or recombinant inbred lines of plants to segregate alleles at an *ERECTA* locus; and
(b) identifying a polymorphic marker linked to said *ERECTA* locus.

The data exemplified herein for *A. thaliana* or rice can clearly be extrapolated to other plant species. For example, the evidence provided herein for the role of the *A. thaliana ERECTA* allele in determining the transpiration efficiency phenotype in those plant species has permitted the elucidation of a wide range of homologous *ERECTA* alleles in other plant species, in particular wheat, rice, sorghum and maize, that are also likely to determine the transpiration efficiency phenotype in those plants. Also described herein is a method of selecting a plant having enhanced transpiration efficiency, comprising selecting a plant that comprises or expresses a functionally equivalent homolog of a protein-encoding region of the *ERECTA* gene of *A. thaliana,* maize, wheat, sorghum or rice.

Also described herein is a method of selecting a plant having enhanced transpiration efficiency, comprising:

(a) identifying a locus on the Arabidopsis chromosome 2 (46-50.7 cM) or rice chromosome 6 associated with genetic variation in transpiration efficiency in a plant;
(b) identifying nucleic acid in a different plant species that comprises a nucleotide sequence having at least about 55% identity to the sequence of the locus at (a); and
(c) selecting a plant that comprises or expresses the identified nucleic acid at (b).

Also described herein is a method of selecting a plant having enhanced transpiration efficiency, comprising:

(a) identifying a locus on the Arabidopsis chromosome 2 (46-50.7 cM) or rice chromosome 6 associated with genetic variation in transpiration efficiency in a plant;
(b) determining the nucleotide sequence of the identified locus;
(c) identifying nucleic acid of a plant species other than *A. thaliana* or rice that comprises a nucleotide sequence having at least about 55% identity to the sequence of the locus at (a); and
(d) selecting a plant that comprises or expresses the identified nucleic acid at (b).

Preferably, the selected plant according to any one or more of the preceding embodiments is *Arabidopsis thaliana,* rice, sorghum, wheat or maize, however other species are not excluded.

Preferably, the subject selection method comprises linking the transpiration efficiency phenotype of the plant to the expression of the marker in the plant, or alternatively, linking a structural polymorphism in DNA to a transpiration efficiency phenotype in the plant, eg., by a process comprising detecting a restriction fragment length polymorphism (RFLP), amplified fragment length polymorphism (AFLP), single strand chain polymorphism (SSCP) or microsatellite analysis. As will be known to the skilled artisan, a nucleic acid probe or primer of at least about 20 nucleotides in length from any one of SEQ ID Nos: 1, 3, 5, 7, 9, 11 to 19 or 21 to 44 or a complementary sequence thereto can be hybridized to genomic DNA from the plant, and the hybridization detected using a detection means, thereby identifying the polymorphism.

It is clearly preferred that the selected plant has enhanced transpiration efficiency compared to a near-isogenic plant that does not comprise or express the genetic marker.

As exemplified herein, the inventors also identified specific genes or alleles that are linked to the *ERECTA* locus of *A. thaliana,* and rice and determined the transpiration efficiencies of those plants. More particularly, the transpiration efficiencies of near-isogenic lines, each carrying a mutation within an *ERECTA* locus, and a correlation between transpiration efficiency phenotype and *ERECTA* expression or gene copy number are determined, thereby providing the genetic contribution of genes or alleles at the *ERECTA* locus to transpiration efficiency. This analysis permits an assessment of the genetic contribution of particular alleles to transpiration efficiency, thereby determining allelic variants that are linked to a particular transpiration efficiency. Thus, the elucidation of the *ERECTA* locus for transpiration efficiency in plants facilitates the fine mapping and determination of allelic variants that modulate transpiration efficiency. The methods described herein can be applied to an assessment of the contribution of specific alleles to the transpiration efficiency phenotype for any plant species that is amenable to mutagenesis such as, for example, by transposon mutagenesis, irradiation, or chemical means. As will be known to the skilled artisan many crop species, such as, maize, wheat, and rice, are amenable to such mutagenesis.

Also described herein is a method of identifying a gene that determines the transpiration efficiency of a plant comprising:

(a) identifying a locus associated with genetic variation in transpiration efficiency in a plant;
(b) identifying a gene or allele that is linked to said locus, wherein said gene or allele is a candidate gene or allele for determining the transpiration efficiency of a plant; and
(c) determining the transpiration efficiencies of a panel of plants, wherein not all members of said panel comprise or express said gene or allele, and wherein variation in transpiration efficiency between the members of said panel indicates that said gene is involved in determining transpiration efficiency.

The method may comprise:

(a) identifying a locus associated with genetic variation in transpiration efficiency in a plant;
(b) identifying multiple alleles of a gene that is linked to said locus, wherein said gene is a candidate gene involved for determining the transpiration efficiency of a plant; and
(c) determining the transpiration efficiencies of a panel of plants, wherein each member of said panel comprises, and preferably expresses, at least one of said multiple alleles, wherein variation in transpiration efficiency between the members of said panel indicates that said gene is involved in determining transpiration efficiency.

Preferably, the identified gene or allele identified by the method described in the preceding paragraph is an *ERECTA* allele, or an *erecta* allele, from a plant selected from the group consisting of *A.* thaliana, sorghum, rice, maize and wheat, or a homolog thereof

The identified gene or allele, including any homologs from a plant other than *A. thaliana,* such as, for example, the wild-type *ERECTA* allele or a homolog thereof, is useful for the production of novel plants. Such plants are produced, for example, using recombinant techniques, or traditional plant breeding approaches such as introgression.

Also described herein is a method of modulating (i.e., enhancing or reducing) the transpiration efficiency of a plant comprising ectopically expressing in a plant an isolated *ERECTA* gene or an allelic variant thereof or the protein-encoding region of: said *ERECTA* gene or said allelic variant. Also described herein is a method of enhancing the transpiration efficiency of a plant comprising introgressing into said plant a nucleic acid comprising a nucleotide sequence that is homologous to a protein-encoding region of a gene of *A. thaliana* that maps to the *ERECTA* locus on chromosome 2.

A further embodiment of the invention provides a method of modulating the transpiration efficiency of a plant comprising introducing and preferably expressing therein, an isolated *ERECTA* gene or an allelic variant thereof or the protein-encoding region thereof to a plant and selecting a plant having a different transpiration efficiency compared to a near-isogenic plant that does not comprise the introduced *ERECTA* gene or allelic variant or protein-encoding region, wherein the *ERECTA* gene or allelic variant or protein-encoding region comprises a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto, wherein the *ERECTA* gene or allelic variant or protein-encoding region is introduced to the plant by a process comprising transforming plant material with a gene construct comprising the gene or allelic variant or protein-encoding region thereof. Preferably the *ERECTA* gene or allelic variant or protein-encoding region comprises a nucleotide sequence selected from the group consisting of:

(a) a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, (SEQ ID NO: 38; SEQ ID NO: 39, SEQ ID NO: 40, SEQ ID NO: 41, SEQ ID NO: 42, SEQ ID NO: 43 and SEQ ID NO: 44; and

(b) a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

The plant into which the gene etc is introduced is preferably selected from the group consisting of *Arabidopsis thaliana,* rice, sorghum, wheat and maize. As will be apparent from the present disclosure, the transpiration efficiency is enhanced as a consequence of the ectopic expression of an *ERECTA* allele or the protein-encoding region thereof in the plant. In contrast, the transpiration efficiency is reduced as a consequence of reduced expression of an *ERECTA* allele in the plant (eg., by expression of antisense RNA or RNAi or other inhibitory RNA).

A further aspect of the invention provides for the use of an isolated *ERECTA* gene or an allelic variant thereof or the protein-encoding region of said *ERECTA* gene or said allelic variant in the preparation of a gene construct for modulating (ie., enhancing or reducing) the transpiration efficiency of a plant, wherein the *ERECTA* gene or allelic variant or protein-encoding region comprises a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto. For example, expression of ERECTA protein in the plant can be modified by epitopic expression of an *ERECTA* allele in the plant, or alternatively, by reducing endogenous ERECTA expression using an inhibitory RNA (eg, antisense or RNAi).

Also described herein is a plant having enhanced transpiration efficiency, wherein said plant is produced by a method described herein.

Plants that have enhanced transpiration efficiency show increased levels of growth under normal growth conditions, thereby increasing their biomass. Accordingly, also described herein is a method of increasing the biomass of a plant comprising enhancing the level of expression of an *ERECTA* gene or allelic variant thereof or protein coding region thereof in said plant.

The method may further include the step of selecting a plant that has an increased biomass when compared to an unmodified plant. Methods of determining the biomass of a plant are well known to those skilled in the art and/or described herein.

The level of expression may be enhanced by genetic modification of a control sequence, for example a promoter sequence, associated with the ERECTA gene or allelic variant thereof.

The level of expression may be enhanced by introducing (eg., by classical breeding, introgression or recombinant means) an ERECTA gene or allelic variant thereof or the protein encoding region thereof to a plant. Preferably, the *ERECTA* gene or allelic variant or protein-encoding region comprises a nucleotide sequence selected from the group consisting of:

a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44; and

a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

The plant into which the gene etc is introduced is preferably selected from the group consisting of *Arabidopsis thaliana,* rice, sorghum, wheat and maize.

A further aspect of the present invention provides a method of increasing the resistance of a plant to an environmental stress comprising enhancing the level of expression of an ERECTA gene or allelic variant thereof or protein coding region thereof in said plant, wherein the *ERECTA* gene or allelic variant or protein-encoding region comprises a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto.

As used herein the term "environmental stress" shall be taken in its broadest context to mean one or more environmental conditions that reduce the ability of a plant to grow, survive and/or produce seed/grain. In one embodiment, an environmental stress that affects the ability for a plant to grow, survive and/or produce seed/grain is a condition selected from the group consisting of increased or decreased $CO_2$ levels, increased or decreased temperature, increased or decreased rainfall, increased or decreased humidity, increased salt levels in the soil, increased soil strength and compaction and drought.

In one embodiment, the method further includes the step of selecting a plant that has an altered resistance to an environmental stress when compared to an unmodified plant is selected. Methods of determining the resistance of a plant to environmental stress are well known to those skilled in the art and/or described herein.

In one embodiment, the level of expression is enhanced by genetic modification of a control sequence, for example a promoter sequence, associated with the ERECTA gene or allelic variant thereof.

In another embodiment, the level of expression is enhanced by introducing an ERECTA gene or allelic variant thereof or the protein encoding region thereof to a plant. Preferably, the *ERECTA* gene or allelic variant or protein-encoding region comprises a nucleotide sequence selected from the group consisting of:

a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44; and

a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

The plant into which the gene etc is introduced is preferably selected from the group consisting of *Arabidopsis thaliana,* rice, sorghum, wheat and maize.

Also described herein is a plant having increased resistance to environmental stress, wherein said plant is produced by a method described herein.

Both temperature and available moisture have been shown to dramatically influence pollination and grain/seed development, processes known as seed-set and grain-filling. Accordingly, a method that produces a plant that is resistant to environmental stress, ie a plant that has increased transpiration efficiency, results in increased or more efficient grain-filling and greater seed number. As ERECTA is expressed during flowering or pod development this gene or an allelic variant thereof is useful for increasing grain-filling in a plant.

Accordingly, a further aspect of the present invention provides a method of increasing seed or grain weight in a plant comprising enhancing the level of expression of an *ERECTA* gene or allelic variant thereof or protein coding region thereof in said plant, wherein the *ERECTA* gene or allelic variant or protein-encoding region comprises a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto.

In one embodiment, the method further includes the step of selecting a plant that has increased seed or grain weight when compared to an unmodified plant is selected. Methods of determining seed or grain weight are well known to those skilled in the art and/or described herein.

In one embodiment, the level of expression is enhanced by genetic modification of a control sequence, for example a promoter sequence, associated with the *ERECTA* gene or allelic variant thereof

In another embodiment, the level of expression is enhanced by introducing an ERECTA gene or allelic variant thereof or the protein encoding region thereof to a plant. Preferably, the

*ERECTA* gene or allelic variant or protein-encoding region comprises a nucleotide sequence selected from the group consisting of:

a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44; and

a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

The plant into which the gene etc is introduced is preferably selected from the group consisting *of Arabidopsis thaliana,* rice, sorghum, wheat and maize.

Also described herein is a plant having increased seed or grain weight, wherein said plant is produced by a method described herein.

Also described herein is a method of modulating the number of seeds produced by a plant comprising enhancing the level of expression of an ERECTA gene or allelic variant thereof in said plant.

The method may further include the step of selecting a plant that has an increased number of seeds when compared to an unmodified plant is selected. Methods of determining seed or grain number are well known to those skilled in the art and/or described herein.

The level of expression may be enhanced by genetic modification of a control sequence, for example a promoter sequence, associated with the ERECTA gene or allelic variant thereof

The level of expression may be enhanced by introducing (eg., by classical breeding, introgression or recombinant means) an ERECTA gene or allelic variant thereof or the protein encoding region thereof to a plant. Preferably, the *ERECTA* gene or allelic variant or protein-encoding region comprises a nucleotide sequence selected from the group consisting of:

a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44; and

a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

The plant into which the gene etc is introduced is preferably selected from the group consisting of *Arabidopsis thaliana,* rice, sorghum, wheat and maize.

Also described herein is a plant having an increased number of seeds, wherein said plant is produced by a method described herein.

BRIEF DESCRIPTION OF THE DRAWINGS

Figure 1a is a graphical representation showing the $CO_2$ assimilation rates ($\mu$mol C $m^2$ $s^{-1}$) of several genotypes of *A. thaliana.* Measurements were completed on rosette leaves during bolting and flowering stages. Plants were grown on fertilised soil. The genotypes of plants are indicated on the x-axis, and $CO_2$ assimilation rates indicated

on the ordinate. Col indicates a genetic background of the ecotype Columbia. Ld indicates a genetic background of the ecotype Landsberg. Plants expressing wild type *ERECTA* alleles were either in a Col (Col4-*ER*) or Ld (Ld-*ER*) background. Plants that were homozygous for a mutant er allele were either in a Ld background (Ld-*er*1) *or in* a Col background (Col-*er105* or Col-*er2 (line 3401 at NASC,* also named *Col-er106* by Torii and collaborators (see Lease et al. 2001, New Phytologist, 151:133-143)). Plants designated as F1 (Col-*ER* x Ld-*er*) were heterozygous *ER*/*er1.* Data indicate that, in a Col background, the *er105* mutation leads to reduced $CO_2$ assimilation rate, whilst the *er1* mutation enhances $CO_2$ assimilation rate in a Ld background.

Figure 1b is a graphical representation showing the stomatal conductance (mol $H_20$ $m^2$ $s^{-1}$) of several genotypes of *A. thaliana* (same plants as Fig.1a). The genotypes of plants are indicated on the x-axis and are the same as described in the legend to Figure 1a. Stomatal conductances are indicated on the ordinate. Data indicate that, in a Col background, the *er2*/*er106* mutation significantly enhances stomatal conductance, whilst the *er1* mutation significantly enhances stomatal conductance in a Ld background.

Figure 1c is a graphical representation showing the transpiration efficiency of (mmol C mol $H_20^{-1}$) of several genotypes of *A. thaliana,* as determined by the ratio of $CO_2$ assimilation rate to stomatal conductance. The genotypes of plants are indicated on the x-axis and are the same as described in the legend to Figure 1a. Transpiration efficiency is indicated on the ordinate. Data indicate that transpiration efficiency is enhanced in plants expressing a wild type *ER* allele relative to a mutant er allele, in both Ld and Col backgrounds. The lowest transpiration efficiency was observed for plants that are homozygous for the *er105* allele (ie. Col-*er105*), consistent with the fact that this allele inhibits *ERECTA* expression. From the data in Figures 1a-1c, it is apparent that the lower transpiration efficiency of plants expressing the *er105* allele is largely due to a reduced $CO_2$ fixation rate, whereas for both the *er2*/*er106* and *er1* alleles, reduced transpiration efficiency is largely due to enhanced stomatal conductance. The transpiration efficiency of the F1 heterozygote plant was intermediate between the transpiration efficiencies of its parents, suggesting codominance of these alleles. The F1, however, had a transpiration efficiency closer to that of the pollen donor parent, Ld-*er1.*

Figure 2a is a graphical representation showing the stomatal densities (Number of stomata $mm^{-2}$ leaf) for several genotypes of *A. thaliana* in three independent experiments. The genetic backgrounds of plants are indicated on the x-axis (Col, Columbia; Ld, Landsberg), and stomatal densities are indicated on the ordinate. Plant genotypes are indicated at the top of each bar, as follows: plants expressing wild type *ERECTA* alleles in a Col background were Col4*ER* or Col1*ER* (hatched bars); plants expressing wild type *ERECTA* alleles in a Ld background were *ER* (open bars); plants expressing mutant *erecta* alleles in a Col background were either *er105* or *er2*/*106* (Col filled boxes); and plants expressing the mutant *er1* allele in a Ld background were *er1* (Ld filled boxes). Columns designated a, b are data from two experiments where plants were grown in soil in the absence of fertiliser. The set of columns at the right of the figure are from a third experiment where the same plants were grown in soil comprising fertiliser. Data indicate that, in a Col background, the *er105* mutation and *er2*/*106* mutation enhances stomatal density, which in part accounts for the enhanced stomatal conductances and reduced transpiration efficiencies of plants expressing these alleles (Figures 1b and 1c). The general effect of these alleles is not dependent on the nutrient status of the soil. In contrast, the *er1* allele only enhanced stomatal density of Ld plants when fertiliser was absent, suggesting that in this ecotype enhanced stomatal aperture accounted for the enhanced stomatal conductances and reduced transpiration efficiencies measured in the er1 mutant under ample nutrient supply (Figures 1b, 1c) The er1 mutation therefore affects both stomatal aperture and stomatal density but the relative contributions of these effects to enhanced stomatal conductance per unit leaf area depend on environmemtal factors and plant nutrient status, and on genetic background.

Figure 2b is a graphical representation showing the epidermal cell size (surface area, $\mu m^2$) for several genotypes of *A. thaliana* in three independent experiments. The genetic backgrounds and genotypes of plants are indicated on the x-axis and at the tops of each column, respectively, as in the legend to Figure 2a. The ordinate indicates epidermal cell size. Columns designated a,b are data from two experiments where plants were grown in soil in the absence of fertiliser. The set of columns at the right of the figure are from a third experiment where the same plants were grown in soil comprising fertiliser. Data indicate that, in a Col background, the *er105* mutation and *er2*/*er106* mutation significantly reduce epidermal cell size ie increase the number of epidermal cells per unit leaf area. This reveals that the ER gene has effects on leaf histogenesis which, beyond their consequences on stomatal densities, may also directly affect leaf capacity for photosynthesis and therefore transpiration efficiency, (Figures 1b and 1c). The general effects of these alleles are not dependent on the nutrient status of the soil. In contrast, in a Ld background, the *er1* allele reduced epidermal cell size only when fertiliser was absent.

Figure 2c is a graphical representation showing the stomatal index for several genotypes of *A. thaliana* in three independent experiments. The genetic backgrounds and genotypes of plants are indicated on the x-axis and at the tops of each column, respectively, as in the legend to Figure 2a. The ordinate indicates stomatal index, as determined from the ratio of stomatal density to epidermal cell density. Columns designated a,b are data from two experiments where plants were grown in soil in the absence of fertiliser. The set of columns at the right of the figure are from a third experiment where the same plants were grown in soil comprising fertiliser. Data indicate that the er mutations tested do not significantly modify stomatal index in Col background (because increases in stomatal density are correlated to increases in epidermal cell numbers in the Col mutant plants) but does so in Landsberg background. Accordingly, the ER gene does appear to directly modify stomatal development *per se.* Taken together Figures 2a-c therefore show that the ERECTA gene has two types of effects on leaf stomatal conductance: a) developmental, b) biophysical and/or biochemical. The expression of these effects and impact on transpiration rate vary with genetic background, suggesting interactions with other genes that are polymorphic between the Col and Ld ecotypes, and also with nutrient status.

Figure 3 is a graphical representation showing carbon isotope composition (y-axis; in per mil, for vegetative rosettes) for 7 different experimental runs (numbers 1-7) carried out under growth cabinet conditions and glasshouse conditions. For each run, the lefthand side bar shows the mean value of carbon isotope composition for lines carrying the *ERECTA* allele, while the right-hand side bar shows the mean value across lines with the *erecta* allele. In all cases, $\delta^{13}C$ isotopic composition values for the er-lines are more negative then those for ER lines, indicative of lower transpiration efficiencies.

Figure 4a is a graphical representation showing *ERECTA* gene copy number and expression levels in transgenic T2 *A. thaliana* plants homozygous for an ER transgene. These lines were generated by transforming the Col-er2/106 mutant with the wild type ER gene under the 35S promoter. Effective transformation was ascertained and *ERECTA* expression levels were quantified in several independent transformants using real-time quantitative PCR (ABI PRISM 7700, Sequence Detection System User Bulletin #2. 1997). Copy number (y-axis) is indicated as a function of the plant line, following normalisation of *ERECTA* relative to the copy number of a control gene (18S ribosomal RNA gene). The expression of the 18S rRNA gene was shown independently not to be affected by changes in ER expression. Line 143 is null control (no insert). Lines 145, 165, 169 and 279 are transformed lines carrying the *ERECTA* allele. All ER transgenic lines, except line 145, show increased mRNA copy number: from 4 to 9.5 fold increase compared with the null control.

Figure 4b is a graphical representation showing *ERECTA* gene copy number and expression levels in transgenic T2 *A. thaliana* plants homozygous for an ER transgene, and generated by transformation of the Col-er105 mutant. Effective transformation was ascertained and *ERECTA* expression levels were quantified in several independent transformants using real-time quantitative PCR (ABI PRISM 7700, Sequence Detection System User Bulletin #2. 1997). Copy number (y-axis) is indicated as a function of the plant line, following normalisation of *ERECTA* relative to the copy number of a control gene (18S ribosomal RNA gene). The expression of the 18S rRNA gene was shown independently not to be affected by changes in ER expression. Line 18 is a null control line (no ER insert, ie similar to Col-er105). Lines 8, 19, 29 and 61 are transgenic lines carrying the *ERECTA* allele. All *ER* transgenic lines show increased mRNA copy number: from 10 to 170 fold increase compared with the null control.

Figure 4c is a graphical representation showing *ERECTA* gene copy number and expression levels in Col and Ld ER ecotypes and in one Ld-ER transgenic line (3-7K) generated by transformation of the Ld-er1 ecotype (NW20) with the ER wild type gene under control of the 35S promoter. Effective transformation was ascertained and *ERECTA* expression levels were quantified in several independent transformants using real-time quantitative PCR (ABI PRISM 7700, Sequence Detection System User Bulletin #2. 1997). Copy number (y-axis) is indicated as a function of the plant line, following normalisation of *ERECTA* relative to the copy number of a control gene (18S ribosomal RNA gene). The expression of the 18S rRNA gene was shown independently not to be affected by changes in ER expression. Lines 933, 1093 and 3176 are the non-transformed Columbia-*ERECTA* ecoptypes Col-4, Col-0 and Col-1.

Line 105c is a Col-er105 line (knockout for ER), used for generating transgenic lines shown in Figure 4b. Lines labelled 2c and 3401 on the X-axis describe Col- er2/106 (2 batches of seeds, used for generating transgenic lines shown in Figure 4a). Line NW20 is Ld-er1. Line 3-7K is a Ld-ER transformant, obtained from transformation of Ld-er1 with the *ERECTA* allele. Line 3177 is the Ld-ER ecotype, near-isogenic to NW20.

Figure 5a is a graphical representation of a first experiment showing copy number of the mRNA transcription product of the rice *ERECTA* gene in various plant organs/parts, cv Nipponbare. L= mature leaf blades; YL= young expanding leaves, still enclosed in sheaths of older leaves; R= mature root; YR= young root; SH= sheaths; INF= unfolded young panicle still enclosed in sheaths; 07: young panicles. Rice *ERECTA* mRNA copy numbers were determined by quantitative real-time PCR, with 18S mRNA as internal control gene for normalization of results. The values on the y-axis describe fold increases of rice *ERECTA* mRNA in various parts compared to the L sample (mature leaves) set to a value of 1 for normalization. Data show a similar expression pattern as the ERECTA gene in Arabidopsis (see Torii et al. 1996) ie preferential expression in young meristematic tissues, especially in reproductive organs.

Figure 5b is a graphical representation of a second experiment showing copy number of the mRNA transcription product of the rice *ERECTA* gene in various plant organs/parts. L= mature leaf blades; YL= young expanding leaves, still enclosed in sheaths of older leaves; R= mature root; YR= young root; SH= sheaths; INF= unfolded young panicle still enclosed in sheaths; 07: young panicles. Rice *ERECTA* mRNA copy numbers were determined by quantitative real-time PCR, with 18S mRNA as internal control gene for normalization of results. The values on the y-axis describe fold increases of rice *ERECTA* mRNA in various parts compared to the L sample (mature leaves) set to a value of 1 for normalization. Data confirm those shown in Figure 5a.

Figure 6 is a graphical representation showing leaf transpiration efficiency (mmol C mol $H_2O^{-1}$, Figure 6a), calculated from the direct measurements of leaf $CO_2$ assimilation rate (μmol C $m^{-2}$ $s^{-1}$, Figure 6b) and stomatal conductance (mol $H_2O$ $m^{-2}$ $s^{-1}$, Figure 6c) by gas exchange techniques, under 350 ppm $CO_2$ (ie same as ambient [$CO_2$] during seedling growth; left hand bar in each pair of bars) and 500ppm $CO_2$ (right hand bar in each pair of bars), for Ld-er1, and two Ld_ER lines: line T2(+ER), a T2 transgenic line homozygous for an ER transgene in the Ld-er1 background and line 3177, an ER ecotype near-isogenic to Ld-er1 (NASC Stock Centre information). Genotypes are shown at the bottom of the figure. Leaf temperature during measurements was controlled at 22°C, leaf to air vapour pressure deficit at around 8mb.

Figure 7 is a graphical representation showing leaf transpiration efficiency (mmol C mol $H_2O^{-1}$, Figure 7a), calculated from the direct measurements of leaf $CO_2$ assimilation rate (μmol C $m^{-2}$ $s^{-1}$, Figure 7b) and stomatal conductance (mol $H_2O$ $m^{-2}$ $s^{-1}$, Figure 7c) by gas exchange techniques, under 350 ppm $CO_2$ (ie same as ambient [$CO_2$] during seedling growth; left hand bar in each pair of bars) and 500ppm $CO_2$ (right hand bar in each pair of bars), for 4 genotypes: Col4 (ER) (left hand pair), Ld (er1) (right hand pair) and their $F_1$ progeny (middle two pairs). Genotypes are shown at the bottom of the figure.

Figure 8 is a graphical representation showing stomatal conductance and epidermal anatomy at 350ppm $CO_2$ in the genotypes described in Figures 6 and 7 and shown at the bottom of the figure. The insertion of ER transgene (line T2+ER) caused a decreased in stomatal conductance compared to the Ld-er1 line (Figure 8a), which was in part due to a decrease in stomatal density (see Figure 8c). These two effects again indicate complementation. Together Figure 8b and 8c show that the decrease in stomatal density is relatively more important than that in epidermal cell density, indicating an effect of the transgene on epidermis development.

Figure 9 is a graphical representation showing a comparison of stomatal density and epidermal cell area in a range of Col_er lines carrying mutations in the ER gene (bars 1 to 8 Fig. 9a; bar 1 to 7 in Fig. 9b, mutants er105, er106, 108, 111, 114, 116, 117, as described in Lease et al. 2001; a gift from Dr Keiko Torii) and in Col-ER wild type ecotypes (bars 9-11 or 8-10 in Figures 9a and 9b, respectively: Col0, background ecotype for these mutants; Col1 Col4 (ColER parental line for QTL analysis of Lister and Dean's RILs), two Ld_er1 lines (NW20 and CS20, bars 12&13 and 11&12 in Figs 9a and 9b respectively, two very similar lines according to NASC; NW20 is the other parental line for Lister and Dean's RILs) and finally line T2+ER, a transgenic Ld-ER line carrying the ER wild type gene in Ld-er1 background (extreme right hand bar on the figure).

Figure 10 is a graphical representation showing carbon isotopic composition (per mil, y-axis) in a range of lines (numbered 1 to 19 on the x-axis): Col-er mutants (line 1-14);

the Col0 background ecotype (line 15); Ld-er1 lines (lines 16 and 17); an Ld-ER near isogenic ecotype to Ld-er1 (line 18, line 3177 at NASC), and a transgenic T2 Ld-ER line (line numbered 19) obtained by transformation of Ld-er1 mutant with a construct carrying the wild type ER allele. The data show that the ER allele gives less negative values indicative of increased transpiration efficiency.

Figure 11 is a graphical representation showing direct measurements of transpiration efficiency in Col-er mutants transformed with ER transgene, under both high and low air humidity, such as occurs during hot temperature events causing or associated with drought. Transpiration efficiency was measured by gas exchange techniques on mature leaves of vegetative Arabidopsis rosettes, as a function of leaf-to-air vapour pressure difference (vpd) ie air humidity around the leaves. The higher the vpd, the drier the air. Solid circles describe measurements for 5 independent transgenic T2 lines homozygous for an ER transgene; these lines were generated by transforming the Col-er105 mutant (empty squares) with a construct carrying the ER allele under control of the 35S promoter. Data for null lines (ie lines that went through transgenesis but do not carry the ER transgene) are represented by solid squares. This figure demonstrates complementation, across the whole range of humidity tested, with the transpiration efficiencies in T2 ER lines being greater than those in the complemented Col-er105 mutant, and similar to those measured in the Col0-ER ecotype (empty triangles; background ecotype for Col-er105).

Figure 12 is a graphical representation of an alignment of isolated sequences with the entire coding region of the wheat ortholog of ERECTA. The position of each of the isolated sequences is shown relative to the wheat ortholog of ERECTA. Sequences are represented by either SEQ ID NO. or gene accession number.

Figure 13 is a graphical representation of an alignment of isolated sequences with the entire coding region of the maize ortholog of ERECTA. The position of each of the isolated sequences is shown relative to the maize ortholog of ERECTA. Sequences are represented by either SEQ ID NO. or gene accession number.

Figure 14 is a graphical representation of a pairwise sequence alignment of the ERECTA proteins isolated from Arabidopsis (SEQ ID NO: 2), maize (SEQ ID NO: 45), rice (SEQ ID NO: 3), Sorghum (SEQ ID NO: 5) and wheat (SEQ ID NO: 20). The alignment was performed using CLUSTALW multiple sequence alignment tool.
Residues that are conserved between all species are indicated by asterisks (*). Conservation of the groups STA NEQK NHQK NDEQ QHRK MILV MILF HY or FYW is indicated by ":". Conservation of the groups CSA ATV SAG STNK STPA SGND SNDEQK NDEQHK NEQHRK FVLIM HFY is indicated by ".". Gaps are indicated by dashes "-".

Figure 15 is a graphical representation of a phylogenetic tree indicating the relationship between each of the ERECTA proteins isolated from Arabidopsis (SEQ ID NO: 2), maize (SEQ ID NO: 45), rice (SEQ ID NO: 3), Sorghum (SEQ ID NO: 5) and wheat (SEQ ID NO: 20).

## DETAILED DESCRIPTION OF THE INVENTION

### *Loci for transpiration efficiency and their identification*

Described herein is a locus associated with the genetic variation in transpiration efficiency of a plant, wherein said locus comprises a nucleotide sequence linked genetically to an *ERECTA* locus in the genome of the plant.

As used herein, the term "locus" shall be taken to mean the location of one or more genes in the genome of a plant that affects a quantitative characteristic of the plant, in particular the transpiration efficiency of a plant. In the present context, a "quantitative characteristic" is a phenotype of the plant for which the phenotypic variation among different genotypes is continuous and cannot be separated into discrete classes, irrespective of the number of genes that determine or control the phenotype, or the magnitude of genetic effects that single gene has in determining the phenotype, or the magnitude of genetic effects of interacting genes.

By "associated with the genetic variation in transpiration efficiency of a plant" means that a locus comprises one or more genes that are expressed to determine or regulate the transpiration efficiency of a plant, irrespective of the actual rate of transpiration achieved by the plant under a specified environmental condition.

Preferably, the locus is linked to or comprises an *ERECTA* allele or *erecta* allele, or a protein-encoding portion thereof.

As used herein, the term *"ERECTA"* shall be taken to refer to a wild type allele comprising the following domains GTIGYIDPEYARTS, GAAQGLAYLHHDC, and TENLSEKXIIGYGASSTVYKC domains, wherein X means Y or H, or domains more than 94 % identical to these domains. To the inventors' knowledge no other protein comprises these domains. Preferred *ERECTA* alleles comprise a nucleotide sequence having at least about 55% overall sequence identity to the protein-encoding region of any one of the exemplified *ERECTA* alleles described herein, particularly any one of SEQ ID Nos: 1, 3, 5, 7, 9, 11, 13, or 15. Preferably, the percentage identity to any one of said SEQ ID NOs: is at least about 59-61%, or 70% or 80%, and more preferably at least about 90%, and still more preferably at least about 95% or 99%.

Preferred *ERECTA* alleles are derived from, or present in, the genome of a plant that is desiccation or drought intolerant, or poorly adapted for growth in dry or arid environments, or that suffers from reduced vigor or growth during periods of reduced rainfall or drought, or from the genome of a plant with increased growth rate or growth duration or partitioning of C to shoot and harvested parts under well-watered conditions.

More preferably, an *ERECTA* allele is derived from, or present in, the genome of a brassica plant, broad acre crop plant, perennial grass (eg. of the subfamily Pooidaea, or the Tribe Poeae), or tree. Even more preferably, an *ERECTA* allele is present in or derived from the genome of a plant selected from the group consisting of barley, wheat, rye, sorghum, rice, maize, *Phalaris aquatica, Dactylus glomerata, Lolium perenne, Festuca arundinacea,* cotton, tomato, soybean, oilseed rape, poplar, and pine.

The term *"erecta"* shall be taken to mean any allelic variant of the wild-type *ERECTA* allele that modifies transpiration efficiency of a plant

Preferred *erecta* alleles include the following *A. thaliana erecta* alleles derived from Columbia (Col) and Landsberg erecta (er) lines.

| Erecta alleles[1] | Genomic position | Lesion | Affected domain |
|---|---|---|---|
| **Ler er-1** | 2249 | T→A | PK |
| **Col er-101** | 6565 | T→A | PK |
| **Col er-102/106** | 6565 | T→A | PK |
| **Col er-103** | 846 | G→A | LRR10 |
| **Col er-105** | foreign DNA insert between +5 and +1056 | insertion | Null allele |
| **Col er-108** | 5649 | G→A | |
| **Col er-111** | 5749 | G→A | Untranslated region between LRR and transmembrane domains |
| **Col er-113** | 3274 | C→T | |
| **Col er-114** | 6807 | G→A | PK |
| **Col er-115** | 3796 | C→T | |
| **Col er-116** | 6974 | G→A | PK |
| **Col er-117** | 5203 | G→A | LRR18 |
| [1] alleles described by Lease et al. 2001, New Phytologist, 151: 133-143, except for Ler er-1, Col ear-103 and Col-er105 which were described in Torii et al., 1996, The Plant Cell 8:73 5-746 | | | |

Described herein is an *erecta* allele derived from, or present in, the genome of a plant that is desiccation or drought intolerant, or poorly adapted for growth in dry or arid environments, or that suffers from reduced vigor or growth during periods of reduced rainfall or drought, or from the genome of a plant with increased growth rate or growth duration or partitioning of C to shoot and harvested parts under well-watered conditions.

More preferably, an *erecta* allele is derived from, or present in, the genome of a brassica plant, broad acre crop plant, perennial grass (eg. of the subfamily Pooidaea, or the Tribe Poeae), or tree. Even more preferably, an *erecta* allele is present in or derived from the genome of a plant selected from the group consisting of barley, wheat, rye, sorghum, rice, maize, *Phalaris aquatica, Dactylus glomerata, Lolium perenne, Festuca arundinacea,* cotton, tomato, soybean, oilseed rape, poplar, and pine.

For the purposes of nomenclature, the nucleotide sequence of the *Arabidopsis thaliana ERECTA* protein-encoding region and the 5'-untranslated region (UTR) and 3'-UTR, is provided herein as SEQ ID NO: 1. The amino acid sequence of the polypeptide encoded by SEQ ID NO: 1 is set forth herein as SEQ ID NO: 2.

A particularly preferred *ERECTA* allele from rice (*Oryza sativa*) is derived from chromosome 6 of that plant species. For the purposes of nomenclature, the protein-encoding region of the rice *ERECTA* gene is provided herein as SEQ ID NO: 3. The amino acid sequence of the polypeptide encoded by SEQ ID NO: 3 is set forth herein as SEQ ID NO: 4.

A particularly preferred *ERECTA* gene derived from the genome of *Sorghum bicolor,* is provided herein as SEQ ID NO: 5. The amino acid sequence of the polypeptide encoded by SEQ ID NO: 5 is set forth herein as SEQ ID NO: 6.

A further exemplary *ERECTA* gene derived from *A. thaliana* is provided herein as SEQ ID NO: 7. The amino acid sequence of the polypeptide encoded by SEQ ID NO: 7 is set forth herein as SEQ ID NO: 8.

A further exemplary *ERECTA* gene derived from *A. thaliana* is provided herein as SEQ ID NO: 9. The amino acid sequence of the polypeptide encoded by SEQ ID NO: 9 is set forth herein as SEQ ID NO: 10.

Fragments of an exemplary ERECTA gene derived from the genome of wheat are provided herein as SEQ ID NOs: 11 to 18.

An exemplary *ERECTA* gene derived from the genome of wheat is provided herein as SEQ ID NO: 19. The amino acid sequence of the polypeptide encoded by SEQ ID NO: 19 is set forth herein as SEQ ID NO: 20.

Fragments of an exemplary ERECTA gene derived from the genome of maize are provided herein as SEQ ID NOs: 21 to 43.

An exemplary *ERECTA* gene derived from the genome of maize is provided herein as SEQ ID NO: 44. The amino acid sequence of the polypeptide encoded by SEQ ID NO: 44 is set forth herein as SEQ ID NO: 44.

Multiple genes may be genetically linked or map to the specified *ERECTA* locus on chromosome 2. Without being bound by any theory or mode of action, such multiple linked genes may interact, such as, for example, by epistatic interaction, to determine the transpiration efficiency phenotype.

Different alleles of any gene may be linked to the *ERECTA* locus, wherein said allele is expressed to determine the transpiration efficiency phenotype. Such alleles may be identified by detecting a particular transpiration efficiency phenotype that is linked to the expression of the particular allele. Alternatively, or in addition, the different alleles linked to a locus are identified by detecting a structural polymorphism in DNA (eg. A restriction fragment length polymorphism (RFLP), amplified fragment length polymorphism

(AFLP), single strand chain polymorphism (SSCP), and the like), that is linked to a particular transpiration efficiency phenotype.

Interacting genes and/or alleles may be

genetically linked to an *ERECTA* locus and are expressed to determine a transpiration efficiency phenotype. Such linked interacting genes and/or alleles will map to an *ERECTA* locus and be associated with the transpiration efficiency of that plant. Preferably, such interacting genes and/or alleles comprise a protein-encoding portion of a gene positioned within the *ERECTA* locus of the genome that is associated with the transpiration efficiency of that plant.

Homologs and/or orthologs of the exemplified alleles are described herein.

Those skilled in the art are aware that the terms "homolog" and "ortholog" refer to functional equivalent units. In the present context, a homolog or ortholog of a gene that maps to an *ERECTA* locus shall be taken to mean any gene from a plant species that is functionally equivalent to a gene that maps to an exemplified *ERECTA* locus, and comprises a protein-encoding region in its native plant genome that shares a degree of structural identity or similarity with a protein-encoding region of the exemplified *ERECTA* gene.

Preferably, a homologous or orthologous gene from a plant other than *A. thaliana* will be associated with the transpiration efficiency of said plant and be linked to a protein-encoding region in its native plant genome that comprises a nucleotide sequence having at least about 55% overall sequence identity to a protein-encoding region linked to the *ERECTA* locus. Even more preferably, the percentage identity will be at least about 59-61% or 70% or 80%, still more preferably at least about 90%, and even still more preferably at least about 95%.

In determining whether or not two nucleotide sequences fall within a particular percentage identity limitation recited herein, those skilled in the art will be aware that it is necessary to conduct a side-by-side comparison or multiple alignment of sequences.

in such comparisons or alignments, differences may arise in the positioning of non-identical residues, depending upon the algorithm used to perform the alignment. In the present context, reference to a percentage identity between two or more nucleotide sequences shall be taken to refer to the number of identical residues between said sequences as determined using any standard algorithm known to those skilled in the art. For example, nucleotide sequences may be aligned and their identity calculated using the BESTFIT program or other appropriate program of the Computer Genetics Group, Inc., University Research Park, Madison, Wisconsin, United States of America (Devereaux et al, Nucl. Acids Res. 12, 387-395, 1984). In determining percentage identity of nucleotide sequences using a program known in the art or described herein, it is preferable that default parameters are used.

Alternatively, or in addition, a homologous or orthologous *ERECTA* or *erecta* allele will be associated with the transpiration efficiency of a plant and be linked to a protein-encoding region in its native plant genome that comprises a nucleotide sequence that encodes a polypeptide having at least about 55% overall sequence identity to a polypeptide encoded by a protein-encoding region linked to the *ERECTA* locus. Preferably, the percentage identity at the amino acid level will be at least about 59-61% or 70% or 80%, more preferably at least about 90%, and still more preferably at least about 95%.

In determining whether or not two amino acid sequences fall within these percentage limits, those skilled in the art will be aware that it is necessary to conduct a side-by-side comparison or multiple alignment of sequences. In such comparisons or alignments, differences will arise in the positioning of non-identical residues, depending upon the algorithm used to perform the alignment. In the present context, reference to a percentage identity or similarity between two or more amino acid sequences shall be taken to refer to the number of identical and similar residues respectively, between said sequences as determined using any standard algorithm known to those skilled in the art. For example, amino acid sequence identities or similarities may be calculated using the GAP program and/or aligned using the PILEUP program of the Computer Genetics Group, Inc., University Research Park, Madison, Wisconsin, United States of America (Devereaux *et al,* 1984, *supra*). The GAP program utilizes the algorithm of Needleman and Wunsch, J. Mol. Biol. 48, 443-453, 1970, to maximize the number of identical/similar residues and to minimize the number and length of sequence gaps in the alignment. Alternatively or in addition, wherein more than two amino acid sequences are being compared, the ClustalW program of Thompson et al., Nucl. Acids Res. 22, 4673-4680, 1994, is used. In determining percentage identity of amino acid sequences using a program known in the art or described herein, it is preferable that default parameters are used.

Alternatively, or in addition, a homologous or orthologous *ERECTA* or *erecta* allele will be associated with the transpiration efficiency of a plant and be linked to a protein-encoding region in its native plant genome that hybridizes to nucleic acid that comprises a sequence complementary to a protein-encoding region linked to an *ERECTA* locus, such as, for example, from *A. thaliana,* rice, sorghum, maize, wheat or rice. Preferably, such homologs or orthologs will be identified by hybridization under at least low stringency conditions, and more preferably under at least moderate stringency or high stringency hybridization conditions.

For the purposes of defining the level of stringency, a low stringency is defined herein as being a hybridization or a wash carried out in 6xSSC buffer, 0.1% (w/v) SDS at 28°C or alternatively, as exemplified herein. Generally, the stringency is increased by reducing the concentration of salt in the hybridization or wash buffer, such as, for example, by reducing the concentration of SSC. Alternatively, or in addition, the stringency is increased, by increasing the concentration of detergent (eg. SDS). Alternatively, or in addition, the stringency is increased, by increasing the temperature of the hybridization or wash. For example, a moderate stringency can be performed using 0.2xSSC to 2xSSC buffer, 0.1% (w/v) SDS, at a temperature of about 42°C to about 65°C. Similarly, a high stringency can be performed using 0.1xSSC to 0.2xSSC buffer, 0.1% (w/v) SDS, at a temperature of at least 55°C. Conditions for performing nucleic acid hybridization reactions, and subsequent membrane washing, are well understood by one normally skilled in the art. For the purposes of further clarification only, reference to the parameters affecting hybridization between nucleic acid molecules is found in Ausubel et al., In: Current Protocols in Molecular Biology, Greene/Wiley, New York USA, 1992.

A number of mapping methods for determining useful loci and estimating their effects have been described (eg. Edwards et al., Genetics 116, 113-125, 1987; Haley and Knott, Heredity 69, 315-324, 1992; Jiang and Zeng, Genetics 140, 1111-1127, 1995; Lander and Botstein, Genetics 121, 185-199, 1989; Jansen and Stam, Genetics 136, 1447-1455, 1994; Utz and Melchinger, In: Biometrics in Plant Breeding: Applications of Molecular Markers. Proc. Ninth Meeting of the EUCARPIA Section Biometrics in Plant Breeding, 6-8 July 1994, Wageningen, The Netherlands, (J.W. van Ooijen and J. Jansen, eds), pp195-204, 1994; Zeng, Genetics 136, 1457-1468, 1994). In the present context, these methods are applied to identify the major component(s) of the total genetic variance that contribute(s) to the variation in transpiration efficiency of a plant, such as, for example, determined by the measurement of carbon isotope discrimination ($\Delta$). More particularly, the segregation of known markers is used to map and/or characterize an underlying locus associated with transpiration efficiency. The locus method involves searching for associations between the segregating molecular markers and transpiration efficiency in a segregating population of plants, to identify the linkage of the marker to the locus.

To discover a marker/locus linkage, a segregating population is required. Experimental populations, such as, for example, an F2 generation, a backcross (BC) population, recombinant inbred lines (RIL), or double haploid line (DHL), can be used as a mapping population. Bulk segregant analysis, for the rapid detection of markers at specific genomic regions using segregating populations, is described by Michelmoore et al., Proc. Natl Acad. Sci. (USA) 88, 9828-9832, 1991. In the case of F2 mapping populations, F2 plants are used to determine genotype, and F2 families to determine phenotype. Recombinant inbred lines are produced by single-seed descent. Recombinant inbred lines, such as, for example, the F9 RILs of *A. thaliana* (eg. Lister and Dean, Plant J., 4, 745-750, 1993) will be known to those skilled in the art. Near isogenic lines (NILs) are used for fine mapping, and to determine the effect of a particular locus on transpiration efficiency. An advantage of recombinant inbred lines and double haploid lines is that they are permanent populations, and as a consequence, provide for replication of the contribution of a particular locus to the transpiration efficiency phenotype.

As for statistical methods, Single Marker Analysis (Point Analysis) is used to detect a locus in the vicinity of a single genetic marker. The mean transpiration efficiencies of a population of plants segregating for a particular marker, are compared according to the marker class. The difference between two mean transpiration efficiencies provides an estimate of the phenotypic effect of substituting one allele for another allele at the locus. To determine whether or not the inferred phenotypic effect is significantly different from zero, a simple statistical test, such as t-test or F-test, is used. A significant value indicates that a locus is located in the vicinity of the marker. Single point analysis does not require a complete molecular linkage map. The further the locus is from the marker, the less likely it is to be detected statistically, as a consequence of recombination between the marker and the gene.

In the Anova, t-test or GLM approach, the association between marker genotype and transpiration efficiency phenotype comprises:

(i) classifying progeny of a segregating population of plants by marker genotype, such as for example, using RFLP, AFLP, SSCP, or microsatellite analyses, thereby establishing classes of plants;
(ii) comparing the mean transpiration efficiencies of classes of plants in the segregating population, using a t-test, GLM or ANOVA; and

(iii) determining the significance of the differences in the mean at (ii), wherein a significant difference indicates that the marker is linked to the locus for transpiration efficiency.

As will be known to those skilled in the art, the difference between the means of the classes provides an estimate of the effect of the locus in determining the transpiration efficiency of a class.

In the regression approach, the association between marker genotype and phenotype is determined by a process comprising:

(i) assigning numeric codes to marker genotypes; and

(ii) determining the regression value r for transpiration efficiency against the codes, wherein a significant value for r indicates that the marker is linked to the locus for transpiration efficiency, and wherein the regression slope gives an estimate of the effect of a particular locus on transpiration efficiency.

For QTL interval mapping, the Mapmaker algorithm developed by Lincoln et al., Constructing genetic linkage maps with MAPMAKER/EXP version 3.0: A tutorial and reference manual. Whitehead Institute for Biomedical Research, Cambridge, MA, USA, 1993, can be used. The principle behind interval mapping is to test a model for the presence of a QTL at many positions between two mapped marker loci. This model is a fit of a presumptive QTL to transpiration efficiency, wherein the suitability of the fit is tested by determining the maximum likelihood that a QTL for transpiration efficiency lies between two segregating markers. For example, in the case of a QTL located between two segregating markers, the 2-loci marker genotypes of segregating progeny will each contain mixtures of QTL genotypes. Accordingly, it is possible to search for loci parameters that best approximate the distribution in transpiration efficiency for each marker class. Models are evaluated by computing the likelihood of the observed distributions with and without fitting a QTL effect. The map position of a QTL is determined as the maximum likelihood from the distribution of likelihood values (LOD scores: ratio of likelihood that the effect occurs by linkage: likelihood that the effect occurs by chance), calculated for each locus.

Interval mapping by regression (Haley and Knott., Heredity 69, 315-324, 1992) is a simplification of the maximum likelihood method *supra* wherein basic QTL analysis or regression on coded marker genotypes is performed, except that phenotypes are regressed on the probability of a QTL genotype as determined from the linkage between transpiration efficiency and the nearest flanking markers. In most cases, regression mapping gives estimates of QTL position and effect that are almost identical to those given by the maximum likelihood method. The approximation deviates only at places where there are large gaps, or many missing genotypes.

In the composite interval mapping (CIM) method (Jansen and Stam, Genetics 136, 1447-1455, 1994; Utz and Melchinger, 1994, *supra;* Zeng, Genetics 136, 1457-1468, 1994), the analysis is performed in the usual way, except that the variance from other QTLs is accounted for by including partial regression giving more power and precision than simple interval mapping, because the effects of other QTls are not present as residual variance. CIM can remove the bias that can be caused by the QTLs that are linked to the position being tested.

Publicly available software are used to map a locus for transpiration efficiency. Such software include, for example, the following:

(i) MapMaker/QTL (ftp://genome.wi.mit.edu/pub/mapmaker3/), for analyzing F2 or backcross data using standard interval mapping;

(ii) MQTL, for composite interval mapping in multiple environments or for performing simple interval mapping using homozygous progeny (eg. double haploids, or recombinant inbred lines);

(iii) PLABQTL (Utz and Melchinger, PLABlocus Version 1.0. A computer program to map QTL, Institut für Pflanzenzüchtung, Saatgutforschung und Populationsgenetik, Universität Hohenheim, 70593 Stuttgart, Germany, 1995; http: //www.uni-hohenheim.de/~ipspwww/soft.html) for composite interval mapping and simple interval mapping of a locus in mapping populations derived from a bi-parental cross by selfing, or in double haploids;

(iv) QTL Cartographer (http://statgen.mcsu.edu/qtlcart/cartographer.html) for single-marker regression, interval mapping, or composite interval mapping, using F2 or backcross populations;

(v) MapQTL (http://www.cpro.dlo.nl/cbw/); Qgene for performing either single-marker regression or interval regression to map loci; and

(vi) SAS for detecting a locus by identifying associations between marker genotype and transpiration efficiency by a single marker analysis approach such as ANOVA, t-test, GLM or REG.

In a particularly preferred embodiment, QTL cartographer or MQTL is used to identify a locus associated with the transpiration efficiency of plants.

Those skilled in the art will also be aware that it is possible to detect multiple interacting alleles or genes for a particular trait, such as, for example, using composite interval mapping approaches. To achieve this end, the composite interval mapping may be repeated to look for additional loci. Alternatively, or in addition, two or more distinct regions of the genome can be nominated as candidate loci, and a gamete relationship matrix constructed for each candidate locus, and a 2-locus regression performed for each pair of loci, determining a best fit for the interacting effects between the two loci or aleles at those loci, including any dominance or additive effects. The algorithm described by Carlborg et al., Genetics (2000) can be used for simultaneous mapping. In the present context, such an analysis is performed with reference to the segregation of transpiration efficiency phenotypes in the segregating population.

*Use of the ERECTA locus to enhance transpiration efficiency of plants*

As will be known to those skilled in the art, a single locus, if present in the genome of a plant, can have a significant influence on the phenotype of the plant. For example, Grandillo et al., Theor. Appl. Genet. 99, 978-987, 1999, showed that for tomato a selection made from a total 28 loci determining fruit size and weight explained 20% of the total phenotypic variance in this trait.

Accordingly, described herein is a method of selecting a plant having enhanced transpiration efficiency, comprising:

(a) identifying a locus associated with genetic variation in transpiration efficiency in a plant; and
(b) selecting a plant that comprises or expresses a gene that maps to the locus.

By "enhanced transpiration efficiency" is meant that the plant loses less water per unit of dry matter produced, or alternatively, produces an enhanced amount of dry matter per unit of water transpired, or alternatively, fixes an increased amount of carbon per unit water transpired, relative to a counterpart plant. By "counterpart plant" is meant a plant having a similar or near-identical genetic background, such as, for example, a near-isogenic plant, a sibling, or parent.

In accordance with this method, a locus is identified by conventional locus mapping means, and/or by homology searching for genes that map to the *ERECTA* locus on chromosome 2 of the *A. thaliana* genome, such as, for example, by searching for *ERECTA* alleles or *erecta* alleles from a variety of plants, such as, for example, rice, wheat, sorghum, and maize, as described herein above.

Preferably, to select a plant that comprises or expresses the appropriate gene, marker-assisted selection (MAS) is used. As will be known to those skilled in the art, once a particular locus has been identified, genetic or physical markers that are linked to the locus can be readily identified and used to confirm the presence of the locus in breeding populations. For a locus that is flanked by two tightly-linked markers that recombine only at a low frequency, the presence of the flanking markers is indicative of the presence of the locus.

For marker-assisted selection, it is preferred that the marker is a genetic marker (eg. a gene or allele), or a physical marker (eg. leaf hairiness or pod shape), or a molecular marker such as, for example, a restriction fragment length polymorphism (RFLP), a restriction (RAPD), amplified fragments length polymorphism (AFLP), or a short sequence repeat (SSR) such as a microsatellite, or SNP. It is also within the scope of the invention to utilize any hybridization probe or amplification primer comprising at least about 10 nucleotides in length derived from a chromosome region that is linked in the genome of a plant to an *ERECTA* locus, as a marker to select plants. Those skilled in the art will readily be able to determine such probes or primers based upon the disclosure herein, particularly for those plant genomes which may have sufficient chromosome sequence in the region of interest in the genome (eg. *A. thaliana,* rice, cotton, barley, wheat, sorghum, maize, tomato, etc).

For flanking markers that are not tightly linked, such that there is a large recombination distance there between, the presence of the appropriate gene is assessed by identifying those plants having both flanking markers and then selecting from those plants having an enhanced transpiration efficiency. Naturally, the greater the distance between two markers, the larger the population of plants required to identify a plant having both markers, the intervening locus and a gene within said locus. Those skilled in the art will readily be able to determine the population size required to identify a plant having a particular transpiration efficiency, based upon the recombination units (cM) between two markers.

Transpiration efficiency is determined by any means known to the skilled artisan. Preferably, transpiration efficiency is determined by measuring dry matter accumulation in the plant by gravimetric means, or by measuring water loss, or the ratio of $CO_2$ assimilation rate to stomatal conductance.

The transpiration efficiency may be determined directly, by measuring the ration of carbon fixed (carbon assimilation rate) to water loss (transpiration rate).

Transpiration efficiency may be determined indirectly from the carbon isotope discrimination value ($\Delta$). Farquhar et al., Aust. J. Plant Physiol. 9, 121-137, 1982, showed that carbon isotope discrimination ($\Delta$; a measure of the extent to which the $^{13}C/^{12}C$ ratio of organic matter is less than that of $CO_2$ in the source air), is an effective indirect measure of transpiration efficiency. Discrimination, ($\Delta$), is approximately the isotope ratio of carbon in source $CO_2$ minus that of plant organic carbon. In a particular experiment, the source $CO_2$ is

common to all genotypes. The determination of transpiration efficiency in this manner is based upon the constancy of the atmospheric $^{13}C$: $^{12}C$ ratio (about 1.1:98.9) and the finding that ribulose bisphosphate carboxylase (Rubisco) enzymes discriminate against the use of $^{13}C$. Thus, in $C_3$ plants $^{13}CO_2$ is less efficiently assimilated than $^{12}CO2$, and the $^{13}CO_2$ left behind tends to diffuse back through stomata in and out of the leaf. However, when the stomata become nearly closed, the relative back-diffusion of $^{13}CO_2$ is more difficult to achieve and the relative intracellular concentrations of $^{13}CO_2$ increases, thereby increasing the proportion of this isotope that is incorporated into 3-phosphoglycerate, and subsequently into dry matter. As a consequence, carbon isotope discrimination (Δ) is greatest when the overall $CO_2$ assimilation rate during photosynthesis (A) is small, and stomatal conductance ($g_w$) to water vapor is large. This relationship is represented by the following algorithm:

$$\Delta\ (‰) = 27 - 36A/(g_w \times C_a)$$

wherein $C_a$ is the ambient $CO_2$ concentration (ie. [$^{12}CO_2$+$^{13}CO_2$]). Discrimination, Δ, is approximately the isotope composition of source $CO_2$ minus that of plant organic carbon.

For a $C_3$ plant that exhibits a value in the range of about 4.5 ‰ to about 6.7 ‰ for the term 36A/($g_w$ x $C_a$), a 1 ‰ change in carbon isotope discrimination (Δ) corresponds to a change in transpiration efficiency in the range of about 22% to about 15%, respectively.

The negative relationship between carbon isotope discrimination (Δ) and transpiration efficiency has been established for many $C_3$ plant species, including wheat (Farquhar and Richards, Aust. J. Plant Physiol. 11, 539-552, 1984; Farquhar et al., Ann. Rev. Plant Physiol. 40,388-397, 1989), *Stylosanthes* (Thumma et al., Proc. 9th Aust. Agronomy Conf., Wagga Wagga New South Wales, Australia, 1998), cotton, barley, and rice. Accordingly, a lower carbon isotope discrimination (Δ) value for a test plant relative to a counterpart plant is indicative of enhanced transpiration efficiency.

In $C_4$ species, like maize, coefficients in the equation above are different (Farquhar 1983, Australian Journal of Plant Physiology, 10:205-226; Henderson et al.,1992, Aust. J. Plant Physiol. 19: 263-285):

$$\Delta\ (‰) = 1 + 5A/(g_w \times C_a).$$

A 1‰ difference in Δ corresponds to about 38% difference in transpiration efficiency. The relationship between Δ and transpiration efficiency is positive. $^{13}C$ preferentially accumulates in bicarbonate, the substrate for PEP carboxylation, and so discrimination against $^{13}C$ is least when A is small and $g_w$ is large. However, as $CO_2$ leakineess from the budle sheath increases, $C_4$ plants behave more like $C_3$ plants.

Alternatively, or in addition, transpiration efficiency is determined by another indicator, such as, for example, leaf temperature, ash content, mineral content, or specific leaf weight (dry matter per unit leaf area). For example, specific leaf weight is positively correlated with transpiration efficiency in peanuts and other species (Virgona et al., Aust. J. Plant Physiol., 17, 207-214, 1990; Wright et al., Crop Sci 34, 92-97, 1994). Accordingly, a higher specific leaf weight or higher carbon gain rate for a test plant relative to a counterpart plant is indicative of enhanced transpiration efficiency of the test plant.

The presence of the locus can be established by hybridizing a probe or primer that is linked to an *ERECTA* locus, such as, for example, a probe or primer that hybridizes to the identified chromosome 2 region of *A. thaliana* or the identified chromosome 6 region of rice.

Preferably, the presence of the locus is established by hybridizing a probe or primer derived from any one or more of SEQ ID Nos: 1, 3, 5, 7, 9, 11 to 19 or 21 to 44 or from a homologous gene in another plant, or a complementary sequence to such a sequence, to genomic DNA from the plant, and detecting the hybridization using a detection means. In one embodiment, detection of the hybridization is performed preferably by labelling a probe with a reporter molecule capable of producing an identifiable signal, prior to hybridization, and then detecting the signal after hybridization. Preferred reporter molecules include radioactively-labelled nucleotide triphosphates and biotinylated molecules. Preferably, variants of the genes exemplified herein, including genomic equivalents, are isolated by hybridisation under moderate stringency or more preferably, under high stringency conditions, to the probe.

Alternatively, or in addition, hybridization may be detected using any format of the polymerase chain reaction (PCR), including AFLP. For PCR, two non-complementary nucleic acid primer molecules comprising at least about 20 nucleotides in length, and more preferably at least 30 nucleotides in length are hybridized to different strands of a nucleic acid template molecule, and specific nucleic acid molecule copies of the template are amplified enzymatically. Several formats of PCR are described in McPherson et al., In: PCR A Practical Approach, IRL Press, Oxford University Press, Oxford, United Kingdom, 1991.

For enhancing the transpiration efficiency of a plant wherein the locus is polymorphic, such as, for example, an allele, the method *supra* is modified to include the detection of the specific allele(s) linked to the desired enhancement. Disclosed herein is a method of selecting a plant having enhanced transpiration efficiency, comprising:

(d) identifying a locus associated with genetic variation in transpiration efficiency in a plant;
(e) identifying a polymorphic marker within said locus that is linked to enhanced transpiration efficiency; and
(f) selecting a plant that comprises or expresses the marker.

Standard means known to the skilled artisan are used to identify a marker within the locus that is linked to enhanced transpiration efficiency. A population of plants that is segregating for the polymorphic marker is generally used, wherein the transpiration efficiency phenotype of plants is then correlated or associated with the presence of a particular allelic form of the marker. As exemplified herein, near-isogenic or recombinant inbred lines of plants are screened to segregate alleles at the *ERECTA* locus and to correlate enhanced transpiration efficiency with the presence of the *ERECTA* allele as opposed to an *erecta* allele. Alternatively, mutations are introduced into an *ERECTA* allele such as, for example, by transposon mutagenesis, chemical mutagenesis or irradiation of plant material, and mutant lines of plants are established and screened to segregate alleles at the *ERECTA* locus that are correlated with the genetic variation in transpiration efficiency.

Suitable markers include any one or more of the markers described herein to be suitable for MAS.

Preferably, the selection of plants in accordance with these embodiments includes the additional step of introducing the locus or polymorphic marker to a plant, such as, for example, by standard breeding approaches or by recombinant means. This may be carried out at the same time, or before, selecting the locus or polymorphic marker.

Recombinant means generally include introducing a gene construct comprising the locus or marker into a plant cell, selecting transformed tissue and regenerating a whole plant from the transformed tissue explant. Means for introducing recombinant DNA into plant tissue or cells include, but are not limited to, transformation using $CaCl_2$ and variations thereof, in particular the method described by Hanahan (1983), direct DNA uptake into protoplasts (Krens et al, Nature 296, 72-74, 1982; Paszkowski et al., EMBO J. 3, 2717-2722, 1984), PEG-mediated uptake to protoplasts (Armstrong et al., Plant Cell Rep. 9, 335-339, 1990) microparticle bombardment, electroporation (Fromm et al., Proc. Natl. Acad. Sci. (USA), 82, 5824-5828, 1985), microinjection of DNA (Crossway et al., Mol. Gen. Genet. 202, 179-185, 1986), microparticle bombardment of tissue explants or cells (Christou et al, Plant Physiol. 87, 671-674, 1988; Sanford, Part. Sci. Technol. 5, 27-37, 1988), vacuum-infiltration of tissue with nucleic acid, or in the case of plants, T-DNA-mediated transfer from *Agrobacterium* to the plant tissue as described essentially by An et al., EMBO J. 4, 277-284, 1985; Herrera-Estrella *et al.,* Herrera-Estella et al., Nature 303, 209-213, 1983; Herrera-Estella et al., EMBO J. 2, 987-995, 1983; or Herrera-Estella et al., In: Plant Genetic Engineering, Cambridge University Press, N.Y., pp 63-93, 1985.

For microparticle bombardment of cells, a microparticle is propelled into a cell to produce a transformed cell. Any suitable ballistic cell transformation methodology and apparatus can be used in performing the present invention. Exemplary apparatus and procedures are disclosed by Stomp et al. (U.S. Patent No. 5,122,466) and Sanford and Wolf (U.S. Patent No. 4,945,050). When using ballistic transformation procedures, the gene construct may incorporate a plasmid capable of replicating in the cell to be transformed.

Examples of microparticles suitable for use in such systems include 1 to 5 micron gold spheres. The DNA construct may be deposited on the microparticle by any suitable technique, such as by precipitation.

A whole plant may be regenerated from the transformed or transfected cell, in accordance with procedures well known in the art. Plant tissue capable of subsequent clonal propagation, whether by organogenesis or embryogenesis, may be transformed with a gene construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (eg., apical meristem, axillary buds, and root meristems), and induced meristem tissue (eg., cotyledon meristem and hypocotyl meristem).

The term "organogenesis", as used herein, means a process by which shoots and roots are developed sequentially from meristematic centres.

The term "embryogenesis", as used herein, means a process by which shoots and roots develop together in a concerted fashion (not sequentially), whether from somatic cells or gametes.

The generated transformed plants may be propagated by a variety of means, such as by clonal propagation or classical breeding techniques. For example, a first generation (or T1) transformed plant may be selfed to give homozygous second generation (or T2) transformant, and the T2 plants further propagated through classical breeding techniques.

The generated transformed organisms contemplated herein may take a variety of forms. For example, they may be chimeras of transformed cells and non-transformed cells; clonal transformants (eg., all cells transformed to contain the expression cassette); grafts of transformed and untransformed tissues (eg., in plants, a transformed root stock grafted to an untransformed scion).

Alternatively, the transformed plants are produced by an *in planta* transformation method using *Agrobacterium tumefaciens,* such as, for example, the method described by Bechtold et al., CR Acad. Sci. (Paris, Sciences de la vie/ Life Sciences) 316, 1194-1199, 1993 or Clough et al., Plant J 16: 735-74, 1998, wherein *A. tumefaciens* is applied to the outside of the developing flower bud and the binary vector DNA is then introduced to the developing microspore and/or macrospore and/or the developing seed, so as to produce a transformed seed. Those skilled in the art will be aware that the selection of tissue for use in such a procedure may vary, however it is preferable generally to use plant material at the zygote formation stage for *in planta* transformation procedures.

*Identification of genes for determing the transpiration efficiency of a plant*

As exemplified herein, the inventors also identified specific genes or alleles that are linked to the *ERECTA* locus of *A. thaliana,* and rice and determine the transpiration efficiencies of those plants. More particularly, the transpiration efficiencies of near-isogenic lines, each carrying a mutation within an *ERECTA* locus, and a correlation between transpiration efficiency phenotype and *ERECTA* expression or gene copy number are determined, thereby providing the genetic contribution of genes or alleles at the *ERECTA* locus to transpiration efficiency. This analysis permits an assessment of the genetic contribution of particular alleles to transpiration efficiency, thereby determining allelic variants that are linked to a particular transpiration efficiency. Thus, the elucidation of the *ERECTA* locus for transpiration efficiency in plants facilitates the fine mapping and determination of allelic variants that modulate transpiration efficiency. The methods described herein can be applied to an assessment of the contribution of specific alleles to the transpiration efficiency phenotype for any plant species that is amenable to mutagenesis such as, for example, by transposon mutagenesis, irradiation, or chemical means known to the skilled artisan for mutating plants.

Accordingly, described herein is a method of identifying a gene that determines the transpiration efficiency of a plant comprising:

(a) identifying a locus associated with genetic variation in transpiration efficiency in a plant;
(b) identifying a gene or allele that is linked to said locus, wherein said gene or allele is a candidate gene or allele for determining the transpiration efficiency of a plant; and
(c) determining the transpiration efficiencies of a panel of plants, wherein not all members of said panel comprise or express said gene or allele, and wherein variation in transpiration efficiency between the members of said panel indicates that said gene is involved in determining transpiration efficiency.

The method may comprise:

(a) identifying a locus associated with genetic variation in transpiration efficiency in a plant;
(b) identifying multiple alleles of a gene that is linked to said locus, wherein said gene is a candidate gene involved for determining the transpiration efficiency of a plant; and
(c) determining the transpiration efficiencies of a panel of plants, wherein each member of said panel comprises, and preferably expresses, at least one of said multiple alleles, wherein variation in transpiration efficiency between the members of said panel indicates that said gene is involved in determining transpiration efficiency.

In the present context, the term "near isogenic plants" shall be taken to mean a population of plants having identity over a substantial proportion of their genomes, notwithstanding the presence of sufficiently few differences to permit the contribution of a distinct allele or gene to the transpiration efficiency of a plant to be determined by a comparison of the transpiration efficiency phenotypes of the population. As will be known to the skilled artisan, recombinant inbred lines, lines produced by introgression of a gene or transposon followed by several generations of backcrossing, or siblings, are suitable near-isogenic lines for the present purpose.

Preferably, the identified gene or allele identified by the method described in the preceding paragraph is selected from the group consisting of *ERECTA* allele, *erecta* allele, and homologs of *ERECTA,* wherein said homologs are from plants species other than *A. thaliana.*

The identified gene or allele may comprise a nucleotide sequence selected from the group consisting of:

(d) a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44;;

(e) a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45; and

(f) a sequence complementary to (a) or (b).

Preferably, the percentage identity is at least about 59-61% or 70% or 80%, more preferably at least about 90%, and even more preferably at least about 95% or 99%. In The identified gene or allele may comprise a nucleotide sequence selected from the group consisting of:

(a) a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44;

(b) a sequence encoding an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45; and

(c) a sequence complementary to (a) or (b).

*Enhancement of transpiration efficiency using isolated genes*

The identified gene or alleles, including any homologs from a plant other than *A. thaliana,* such as, for example, the wild-type *ERECTA* allele, or a homolog thereof, is useful for the production of novel plants. Such plants are produced, for example, using recombinant techniques.

Accordingly, described herein is a method of enhancing the transpiration efficiency of a plant comprising ectopically expressing in a plant an isolated *ERECTA* gene or an allelic variant thereof or the protein-encoding region thereof.

Preferably, the *ERECTA* gene or allelic variant comprises a nucleotide sequence that is homologous to a protein-encoding region of a gene that is linked to the *A. thaliana ERECTA* locus on chromosome 2.

The isolated gene may comprise a nucleotide sequence selected from the group consisting of:

(a) a sequence having at least about 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44;

(b) a sequence encoding an amino acid sequence having at least about 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45; and

(c) a sequence complementary to (a) or (b).

Preferably, the percentage identity is at least about 59-61% or 70% or 80%, more preferably at least about 90%, and even more preferably at least about 95% or 99%.

The isolated gene or allele may comprise a nucleotide sequence selected from the group consisting of:

(a) a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43 and SEQ ID NO: 44;

(b) a sequence encoding an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45; and

(c) a sequence complementary to (a) or (b).

To ectopically express the isolated gene in a plant, the protein-encoding portion of the gene is generally placed in operable connection with a promoter sequence that is operable in the plant, which may be the endogenous promoter or alternatively, a heterologous promoter, and a transcription termination sequence, which also may be the endogenous or an heterologous sequence relative to the gene of interest. The promoter and protein-encoding portion and transcription termination sequence are generally provided in the form of a gene construct, to facilitate introduction and maintenance of the gene in a plant where it is to be ectopically expressed. Numerous vectors suitable for introducing genes into plants have been described and are readily available. These may be adapted for expressing an isolated gene in a plant to enhance transpiration efficiency therein.

Reference herein to a "promoter" is to be taken in its broadest context and includes the transcriptional regulatory sequences of a classical eukaryotic genomic gene, including the TATA box which is required for accurate transcription initiation, with or without a CCAAT box sequence and additional regulatory elements (ie. upstream activating sequences, enhancers and silencers) which alter gene expression in response to developmental and/or external stimuli, or in a tissue-specific manner. In the present context, the term "promoter" is also used to describe a synthetic or fusion molecule, or derivative which confers, activates or enhances expression of said sense molecule in a cell. Preferred promoters may contain additional copies of one or more specific regulatory elements, to further enhance expression and/or to alter the spatial expression and/or temporal expression of a nucleic acid molecule to which it is operably connected. For example, copper-responsive regulatory elements may be placed adjacent to a heterologous promoter sequence driving expression of a nucleic acid molecule to confer copper inducible expression thereon.

Placing a nucleic acid molecule under the regulatory control of a promoter sequence means positioning said molecule such that expression is controlled by the promoter sequence. A promoter is usually, but not necessarily, positioned upstream or 5' of the protein-encoding portion of the gene that it regulates. Furthermore, the regulatory elements comprising a promoter are usually positioned within 2 kb of the start site of transcription of the structural protein-encoding nucleotide sequences, or a chimeric gene comprising same. In the construction of heterologous promoter/structural gene combinations it is generally preferred to position the promoter at a distance from the gene transcription start site that is approximately the same as the distance between that promoter and the gene it controls in its natural setting, ie., the gene from which the promoter is derived. As is known in the art, some variation in this distance can be accommodated without loss of promoter function. Similarly, the preferred positioning of a regulatory sequence element with respect to a heterologous gene to be placed under its control is defined by the positioning of the element in its natural setting, ie., the genes from which it is derived. Again, as is known in the art, some variation in this distance can also occur.

Promoters suitable for use in gene constructs of the present invention include those promoters derived from the genes of viruses, yeasts, moulds, bacteria, insects, birds, mammals and plants which are capable of functioning in plant cells, including monocotyledonous or dicotyledonous plants, or tissues or organs derived from such cells. The promoter may regulate gene expression constitutively, or differentially with respect to the tissue in which expression occurs or, with respect to the developmental stage at which expression occurs, or in response to external stimuli such as physiological stresses, pathogens, or metal ions, amongst others.

Examples of promoters useful in performing this embodiment include the CaMV 35S promoter, rice actin promoter, rice actin promoter linked to rice actin intron (PAR-IAR) (McElroy et al, Mol and Gen Genetics, 231(1), 150-160, 1991), *NOS* promoter, octopine synthase (OCS) promoter, *Arabidopsis thaliana SSU* gene promoter, napin seed-specific promoter, PcSVMV, promoters capable of inducing expression under hydric stress, as described by, for example, Kasuga et al, Nature Biotechnology, 17, 287-291, 1999), SCSV promoter, SCBV promoter, 35s promoter (Kay et al, Science 236, 4805, 1987) and the like. In addition to the specific promoters identified herein, cellular promoters for so-called housekeeping genes, including the actin promoters, or promoters of histone-encoding genes, are useful.

The term "terminator" refers to a DNA sequence at the end of a transcriptional unit which signals termination of transcription. Terminators are 3'-non-translated DNA sequences containing a polyadenylation signal, that facilitate the addition of a polyadenylate sequence to the 3'-end of a primary transcript. Terminators active in cells derived from viruses, yeasts, moulds, bacteria, insects, birds, mammals and plants are known and described in the literature. They are isolatable from bacteria, fungi, viruses, animals and/or plants.

Examples of terminators particularly suitable for use in the gene constructs of the present invention include the nopaline synthase (NOS) gene terminator of *Agrobacterium tumefaciens,* the terminator of the Cauliflower mosaic virus (CaMV) 35S gene, the zein gene terminator from *Zea mays,* the Rubisco small subunit (SSU) gene terminator sequences and subclover stunt virus (SCSV) gene sequence terminators, amongst others.

Those skilled in the art will be aware of additional promoter sequences and terminator sequences that may be suitable for use in performing the invention. Such sequences may readily be used without any undue experimentation.

Preferably, the gene construct further comprises an origin of replication sequence for its replication in a specific cell type, for example a bacterial cell, when said gene construct is required to be maintained as an episomal genetic element (eg. plasmid or cosmid molecule) in said cell. Preferred origins of replication include, but are not limited to, the *f1*-ori and *col*E1 origins of replication.

Preferably, the gene construct further comprises a selectable marker gene or genes that are functional in a cell into which said gene construct is introduced.

As used herein, the term "selectable marker gene" includes any gene which confers a phenotype on a cell in which it is expressed to facilitate the identification and/or selection of cells which are transfected or transformed with a gene construct described herein or a derivative thereof.

Suitable selectable marker genes contemplated herein include the ampicillin resistance ($Amp^r$), tetracyclin-resistance gene ($Tc^r$), bacterial kanamycin resistance gene ($Kan^r$), phosphinothricin resistance gene, neomycin phosphotransferase gene (*npt*II), hygromycin resistance gene, gentamycin resistance gene (gent), (β-glucuronidase (GUS) gene, chloramphenicol acetyltransferase (CAT) gene, and luciferase gene, Green Fluorescent Protein gene (EGFP and variants), amongst others.

Also described herein is the use of an isolated gene comprising a nucleotide sequence that is homologous to a protein-encoding region of a gene of *A. thaliana* that is positioned between about 46cM to about 50.74cM on chromosome 2 in the preparation of a gene construct for enhancing the transpiration efficiency of a plant.

Also described herein is a plant having enhanced transpiration efficiency, wherein said plant is produced by a method described herein.

Clearly the ERECTA genes, allelic variants and protein coding regions described herein are useful in determining other proteins that are involved in the transpiration process in plants. For example, an ERECTA gene, allelic variant thereof or protein coding region thereof may be used in a forward 'n'-hybrid assay to determine if said peptide is able to bind to a protein or peptide of interest. Forward 'n' hybrid methods are well known in the art, and are described for example, by Vidal and Legrain Nuc/. Acid Res 27(4), 919-929 (1999) and references therein, and include yeast two-hybrid, bacterial two-hybrid, mammalian two-hybrid, PolIII (two) hybrid, the Tribrid system, the ubiquitin based split protein sensor system and the SOS recruitment system.

In adapting a standard forward two-hybrid assay to the present invention, an ERECTA protein is expressed as a fusion protein with a DNA binding domain from, for example, the yeast GAL4 protein. Methods of constructing expression constructs for the expression of such fusion proteins are well known in the art, and are described, for example, in Sambrook et al (In: Molecular Cloning: A laboratory Manual, Cold Spring Harbour, New York, Second Edition, 1989). A second fusion protein is also expressed in the yeast all, said fusion protein comprising, for example, a protein thought to interact with an ERECTA protein, for example the GAL4 activation domain. These two constructs are then expressed in a yeast cell in which, a reporter molecule (e.g., $tet^r$, $Amp^r$, $Rif^r$, $bsdf^r$, $zeof^r$, $Kan^r$, *gfp, cobA, LacZ, TRP1*, *LYS2, HIS3, HIS5, LEU2, URA3, ADE2, MET13, MET15)* under the control of a minimal promoter placed in operable connection with a GAL 4 binding site. If the proteins do not interact, a reporter molecule is not expressed. However, if said proteins do interact, said reporter molecule is expressed. Accordingly a protein, polypeptide, peptide that is able to specifically bind a target protein is identified.

A forward 'n'-hybrid method may be modified to facilitate high throughput screening of a library of peptides, polypeptides and/or proteins in order to determine those that interact with an ERECTA protein. Methods of screening libraries of proteins are well known in the art and are described, for example, in Scopes (In: Protein Purification: Principles and Practice, Third Edition, Springer Verlag, 1994). Proteins identified by this method are potentially involved in the transpiration process in plants.

The present invention is further described with reference to the following non-limiting examples.

EXAMPLE 1

$^{12}C/^{13}C$ discrimination as a marker for screening genetic variation in transpiration efficiency.

Experimental conditions and sampling procedures were established to allow the control of many factors, other than genetic, that influence transpiration efficiency at the level of individual leaves and plants. These factors fall into several categories: (a) characteristics of the seedling's micro-environment: temperature, light, humidity, boundary layer around the leaves, root growth conditions; (b) developmental and morphological effects that modify gas exchange and C metabolism and therefore carbon isotopic signature (eg age, stage, posture); and (c) seed effects.

We developed high resolution mass-spectrometer techniques for measuring C isotope ratios in whole tissues or carbon compounds such as soluble sugars -ie a measure of integrated transpiration efficiency over the plant's life or over a day, respectively, and also for measuring instantaneous transpiration efficiency during gas exchange.

This means:

- 0.1 per mil analytical precision in the measurement of the isotopic composition of leaf carbon. Discrimination, (Δ), is approximately the isotope ratio of carbon in source $CO_2$ minus that of plant organic carbon. In a particular experiment, the source $CO_2$ is common to all genotypes.
- 0.1 per mil biological precision, that is variation between replicated seedlings, grown in soil, either in growth chambers or in glasshouses with $CO_2$, humidity and temperature control (corresponding to approximately 1.5% variation in transpiration efficiency).

• The ability to grow and screen large batches of seedlings in glasshouses or growth chambers (up to 1500), under standardised leaf and root growth conditions, to a rosette size of several' cm within 2-3 weeks allowing individual measurements, on the same plant, of isotope ratios and also of the underlying properties (eg in situ measurement of leaf temperature by infra-red thermometry as a measure of stomatal conductance; chlorophyll fluorescence; leaf expansion).

EXAMPLE2

Natural genetic variation in transpiration efficiency in *Arabidopsis thaliana*

*A. thaliana* ecotypes were screened for leaf Δ under glasshouse conditions. There was a large spread of values (corresponding to approximately 30 % genetic variation in transpiration efficiency). However, large environmental effects were noted. A few contrasted ecotypes were selected at the two extremes of the range of Δ values and compared under various conditions of irradiance (150 to 500 $\mu E\ m^{-2}s^{-1}$), light spectrum (Red/Far-Red ratios) and air humidity (60 to 90%) while roots were always well watered. The magnitude of genetic differences in transpiration efficiency was very much influenced by environmental conditions. This was in part due to variations among ecotypes in the dependence of photosynthesis on light and vapour pressure deficit. Genetic differences were maximal under a combination of high light and low humidity, in growth chambers.

The ecotypes *Columbia (Col)* and *Landsberg erecta* (Ld-*er*) have extreme carbon isotope discrimination values, with *Col* always having smaller Δ values than *Ld-er* ie less negative $\delta^{13}C$ isotopic compositions, and thus a greater transpiration efficiency.

EXAMPLE 3

Identification of a locus associated with transpiration efficiency in *A. thaliana*

Quantitative Trait Loci (QTL) analysis of the Lister and Dean's (1993) Recombinant Inbred Lines (later referred to as RILs) was performed to identify and map a locus associated with carbon isotope discrimination Δ). The RILs were from a cross between Col-4 and Ler-0. Our analysis showed the importance of genes around the *ER* locus on chr2, and a role for genes other than *ERECTA* in conferring transpiration efficiency on *A. thaliana.*

More particularly, 300 RI mapping lines between Col and Ler ecotypes, available at the Arabidopsis Stock Centre, were generated from a cross between the *Arabidopsis* ecotypes Columbia (Col4) and Landsberg erecta (Ler-0 carrying *er1*) (Lister and Dean, 1993), using Columbia as the male parent. A subset of 100 of these lines, chosen as the most densely and reliably mapped were used in the present analysis.

The seeds were multiplied in a glasshouse in an attempt to minimize confounding seed effects in our comparisons. Large numbers of seeds were obtained for most lines except for a few, including Col4 parent, which had to be re-ordered following low seed viability of the original sample sent by the Stock Centre. The seeds harvested in these propagation runs were used throughout all our experiments to date.

Loci were analysed using two programs, QTL cartographer and MQTL. These programs compute statistics of a trait at each marker position, using a range of methods [linear regression (LR), stepwise regression (SR), and likelihood approaches (Single interval mapping (SIM) which treats values at individual markers as independent values, and composite interval mapping (CIM) which allows for interactions between markers and associated locus)]. By nature each of these methods has some biases and embedded assumptions, hence the importance of analysing data with more than one program. Only results that were consistent between the two programs, and robust to additions or deletions to the set of background markers used for composite interval mapping are reported below.

Initial QTL analysis was done in parallel to seed multiplication on a subset of 40 lines for which enough seeds were sent. Once all seeds had been multiplied this was repeated on the full set of 100 lines. These two analyses indicated the existence of a locus for carbon isotope discrimination (Δ), that maps to the region including the *ERECTA* locus on chromosome 2, at approximately 46-51 cM (Table 1, run 1&2).

Given the complexity and integrative nature of Δ as a physiological trait, such a small number of loci associated with the trait was not expected. Subsequent experiments were therefore designed to test these results and assess their stability across the range of environmental conditions known for their effects on gene expression related to Δ (see above). QTL analysis was repeated on several completely independent data sets obtained under highly controlled conditions in glasshouses or growth chambers, where either air humidity, photoperiod or irradiance (amount, diurnal pattern, day to day variation) was varied. Depending on the experiment, all 100 recombinants inbred lines were included or only the subset of lines with cross-overs on chromosome 2. These experiments confirmed that genetic variation in Δ could be mostly ascribed to a portion of chromosome 2 (Table 1) between about 46-50.7 cM.

When RILs were sorted graphically according to carbon isotope discrimination and their genotype at the *ER* marker (50.64 cM) and its vicinity (Ld-*er1* genotype or Col-*ER* genotype), lines which were Ld-*er* at the *ERECTA* marker ranked mostly at the high end of carbon isotope discrimination values. In contrast, lines having a Col-*ERECTA* marker genotype ranked mostly at the low end of carbon isotope discrimination values (data available on request). In the middle of the range of carbon isotope discrimination values, there was some overlap between the two sets of lines. Some lines were always at an extreme (in all 18 experiments performed), while the ranking of other lines was more unstable. These data indicate a locus for transpiration efficiency, as determined by the carbon isotope discrimination value, in the vicinity of the *ERECTA* locus on chromosome 2 (Table 1). This locus most likely involves the *ER* gene. Depending on the positions of cross-overs between *L*d-er and *Col,* recombination between *ERECTA* and one or more of the other genes influences the transpiration efficiency phenotype of the progeny.

EXAMPLE 4

Transformation protocol for maize

Gun transformation

A suitable method for maize transformation is based on the use of a particle gun identical to that described by J. Finer (1992, Plant Cell Report, 11:323-328). The target cells are fast dividing undifferentiated cells having maintained a capacity to regenerate in whole plants. This type of cells composes the embryogenic callus (called type II) of maize. These calluses are obtained from immature embryos of genotype HiII according to the method and on medium described by Armstrong (Maize Handbook ; 1994 M. Freeling, V. Walbot Eds ; pp.665-671).

These fragments of the calluses having a surface from 10 to 20 mm2 are arranged, 4 hour before bombardment, by putting 16 fragments by dish in the center of a Petri dish containing an culture medium identical to the medium of initiation of calluses, supplemented with 0.2 M of mannitol + 0,2 M of sorbitol. Plasmids containing the ERECTA sequences to be introduced, are purified on QiagenR column following the instructions of the manufacturer.

They are then precipitated on particles of tungsten (M10) following the protocol described by Klein et al, Nature, 327, 70-73, (1987). Particles so coated are sent towards the target cells by means of the gun according to the protocol described by Finer et al, Plant Cell Report, 11:323-328, 1992. The bombarded dishes of calluses are then sealed by means of ScellofraisR then cultivated in the dark at 27°C.

The first transplanting takes place 24 hours later, then every other week during 3 months on medium identical to the medium of initiation supplemented with a selective agent. After 3 months or sometimes earlier, one can obtain calluses the growth of which is not inhibited by the selective agent, usually and mainly consisting of cells resulting from the division of a cell having integrated into its genetic patrimony one or several copies of the gene of selection. The frequency of obtaining of such calluses is about 0,8 callus by bombarded dish.

These calluses are identified, individualized, amplified then cultivated so as to regenerate seedlings, by modifying the hormonal and osmotic equilibrium of the cells according to the method described by Vain and al. (1989, Plant Cell tissue and organ Culture 18:143-151). These plants are then acclimatized in greenhouse where they can be crossed for obtaining hybrids or self-fertilized.

In a preferential way, one can use a similar protocol, the principle of which is described in Methods of Molecular Biology: Plant gene transfer and expression protocols (1995, vol. 49, PP113-123), and in which the immature embryos of genotype HiII are directly bombarded with golden particles coated with plasmides ERECTA to introduce, prepared according to the protocol described by Barcelo and Lazzeri (1995, Methods of Molecular Biology, 49:113-123).

Steps of transformation, selection of the events, maturation and regeneration are similar to those described in the previous protocol.

Agrobacterium transformation

Another technique of useful transformation within the framework of the invention uses Agrobacterium tumefaciens, according to the protocol described by Ishida and al (1996, Nature Biotechnology 14 : 754-750), in particular starting from immature embryos taken 10 days after fertilization.
All the used media are referenced in the quoted reference. The transformation begins with a phase of co-culture where the immature embryos of the maize plants are put in contact during at least 5 minutes with Agrobacterium tumefaciens LBA 4404 containing the superbinary vectors.

The superbinary plasmid is the result of an homologous recombination between an intermediate vector carrying the T-DNA, and containing the gene of interest and/or the marker gene of selection, and the vector pSB1 of Japan Tobacco (EP 672 752) containing: the virB and virG genes of the plasmide pTiBo542 present in the supervirulent strain A281 of Agrobacterium tumefaciens (ATCC 37349) and an homologous region found in the intermediate vector, allowing homologous recombination.

Embryos are then placed on LSAs medium for 3 days in the dark and at 25°C. A first selection is made on the transformed calluses: embryogenic calluses are transferred on LSD5 medium containing phosphinotricine (5 mg / l) and céfotaxime (250 mg / l) (elimination or limitation of contamination by *Agrobacterium tumefaciens*).

This step is performed during 2 weeks in the dark and at 25°C. The second step of selection is realized by transfer of the embryos which developed on LSD5 medium, on LSD10 medium (phosphinotricine, 10 mg /l) in the presence of céfotaxime, during 3 weeks at the same conditions as previously. The third stage of selection consists in excising the calluses of type I (fragments from 1 to 2 mm) and in transferring them for 3 weeks in the darkness and at 25°C on LSD 10 medium in the presence of céfotaxime. The regeneration of seedlings is made by excising the calluses of type I which proliferated and by transferring them on LSZ medium in the presence of phosphinotricine (5 mg / l) and of céfotaxime for 2 weeks at 22°C and under continuous light.

Seedlings having regenerated are transferred on RM medium + G2 containing Augmentin (100mg /l) for 2 weeks at 22°C and under continuous illumination for the development step. The obtained plants are then transferred to the phytotron with the aim of acclimatizing.

EXAMPLE 5

Detecting expression of ERECTA protein

Extraction of ERECTA from leaves and seeds of maize.

Leaves are harvested and immediately frozen in liquid nitrogen. Grinding is made in a mortar cleaned in ethanol 100 % and cooled on ice. A foliar disc of 18 mm diameter is extracted in 200 μL of extraction buffer: Tris-HCl pH 8.0, glycerol 20%, MgCl2 10 mM, EDTA 1 mM, DTT 1 mM, PVP insoluble 2% (p/v), Fontainebleau sand et protease inhibitors: leupeptin 2mg/L, chymostatin 2mg/L, PMSF 1mM and E64 1mg/L. The ground material is then centrifuged in 4°C during 15 minutes at 20000g to eliminate fragments.

Grains are first reduced to powder in a bead-crusher (Retsch). Proteins are extracted by suspending 100μL of powder in 400 μL of the previously described buffer on ice. This mixture is vortexed and centrifuged at 4°C during 15 minutes et 20000g to eliminate fragments.

ERECTA protein levels are then measured using techniques known to those skilled in the art, and described, for example, in Scopes (In: Protein purification: principles and practice, Third Edition, Springer Verlag, 1994).

EXAMPLE 6

Determination of a role for the *ERECTA* gene in regulating transpiration efficiency

We compared Col and Ler ecotypes with near-isogenic mutant lines for the *erecta* gene, to examine a possible role of the *ERECTA* gene in determining carbon isotope discrimination (Δ).

Plants expressing the wild type *ERECTA* gene (SEQ ID NO: 1), or an *erecta* mutant allele in the Columbia background (eg. Col-er1, Col-er2, Col-*er101* to *-er105;* or Col-*er106* to *-er123*) and in Landsberg background (*Ld-er1*) have been publicly described. Three of these mutants were available for comparison to the isogenic or near-isogenic lines (Table 2).

*Col4* (ER) and Ld-*er1*, the parental lines for Lister and Dean's RILs were systematically included in the comparison. Where possible, other Col "ecotypes" were also included, (eg. Co10, Col1, Col3-7), to assess their similarity with respect to carbon isotope discrimination, especially compared to the RIL parental ecotype Col4.

The results of these comparisons are described in Table 3. Data indicate the differences in carbon isotope discrimination values between *er* and *ER* lines for 15 different experimental runs corresponding to growth under low to high light (100 to 800 μE $m^{-2}$ $s^{-1}$), low to high humidity (40 to 85%), short to long days (8, 10, 24hrs), normal to high temperatures (22/20°C to 28/20 °C).

As expected, the spread of carbon isotope discrimination values among lines varied with environmental conditions. Lines carrying er mutations have a greater carbon isotope discrimination value overall than those having the *ER* wild type gene (see Table 3, column 1), indicative of a lower water use-efficiency. There is usually little difference in C isotopic discrimination between the various Col lines, (see the similar averages obtained for columns 2, 3, and 4 in Table 3, wherein *er105* is compared to 3 different Col ecotypes, Co10, Col4 and 3176 or Col1). When present, the *er105* mutant always has the greatest carbon isotope discrimination value of all lines, including *er1* and *er2* (columns 2-4 compared to columns 5-6 in Table 3, or column 8 compared to column 9 in Table 3). The value measured in the *er105* mutant is always significantly greater than in the *ER* isogenic line (column 4 in Table 3). The value measured in *er1* (Landsberg parental line NW20) is usually also greater than that in the *ER* lines 3177 (near isogenic, column 6 of Table 3), and to a lesser extent Col4 (Columbia parental line, column 7 of Table 3). These observations give direct evidence that the *ERECTA* gene plays a significant role in determining genetic differences in carbon isotopic discrimination in *Arabidopsis.*

This conclusion is independently confirmed by leaf gas exchange measurements that allow the direct measure of transpiration efficiency (ratio of net $CO_2$ fixation to water loss; column 4 in Table 4; Figures 1a-1c, 2a-2c). Measurements on mature leaves reveal that *ER* lines are characterised by a greater ratio of $CO_2$ assimilation to water loss than lines carrying *er* mutations. This is most obvious when comparing the pair *Col1*/ *er105* with a 21% greater transpiration efficiency (ratio A/E) in *Col1* than *er105,* or the pair *Col1*/*er2* with a 16% greater transpiration efficiency in *Col1.* Consistent with the measurements of carbon isotope discrimination, the effect *er*/*ER* is relatively smaller in the *Ld* background (9% greater ratio A/E in *Ld-ER* (3177) than in the *Ld-er1* (NSW20) line).

Also consistent with the carbon discrimination measurements, is the 20% difference in transpiration efficiency between the two RILs parental lines (4.06 and 3.38 mmolC/molH2O in *Col4-ER* and *Ld-er1,* respectively).

The fact that of all 3 *erecta* mutants examined, *er105* has the most extreme carbon discrimination and transpiration efficiency phenotypes suggests that the *er105* mutation affects a more crucial part of the *ERECTA* gene than *er2* or *er1.* This is consistent with the published data on the *er105* mutant. This mutation corresponds to the insertion of a large "foreign insert" in the *ERECTA* gene. The insertion inhibits transcription of the gene and causes the strongest *erecta* phenotype of all *erecta* mutants isolated in Col (with respect to inflorescence clustering and silique width and shape). Alternatively, or in addition, data indicate that *erecta* mutations have a stronger effect on carbon isotope discrimination values in a Columbia genetic background than in a Landsberg background (comparison of phenotypic effects of *er105* and *er1*), implying that other genes, polymorphic between Landsberg and Columbia ecotypes, interact with *ERECTA* in determining transpiration efficiency. This could also account for the greater difference in transpiration efficiency between *er*/*ER* lines in Col background than in a Ld background (see above, Table 4). Alternatively, or in addition, data indicate that the *erecta* mutation is not the only mutation present in the *er105* mutant. For example, the mutagenized Col seeds may have carried the *gl1* mutation, induced by the fast neutron irradiation, that also contributes to the phenotype observed.

A comparison of transcript profiles in *er*/*ER* isogenic lines (in both Col and Ld background) allows determination of the involvement of additional genes to *ERECTA* and the effect of environment on their expression.

EXAMPLE 7

QTL detection centred on the *ERECTA* marker and *ERECTA* gene locus on chromosome 2 of *Arabidopsis thaliana*

1. Methods

Numerous runs using the Lister and Dean (1993) Recombinant Inbred Lines between Col-4 and Ler-0 were grown in a temperature controlled glass house (20/20°C) or within growth cabinets (21°C and light levels ranging from 100 to 500 $\mu E\ m^{-2}\ s^{-1}$ irradiance, and 50-70% relative humidity). Runs included a variety of all 100 RILs as well as subsets of these 100 along with parental Col-4 and Ler-0 (NW20) parental lines. Individual RILs were replicated within runs. Seeds were either cold-treated on moist filter paper for 2-4 days, cold-treated and planted directly onto soil; or plated onto agar, cold-treated for 2-4 days, grown on agar for about 11-15 days before being transferred to soil. Plants were well-watered, and grown for 4-5 weeks before harvest. Samples (whole or part of rosette) were collected and dried in an 80°C oven before being ground and analysed for C isotopic composition. The value used for QTL analysis for an individual line was the average of the replicated plants of that line within one run.

2. Marker Selection

The standard set of 64 markers for the Lister and Dean recombinant lines were downloaded from the NASC website. Additional markers were added to this data set when significance was first determined to get finer scale mapping in the regions of interest. A total of 121 markers were used across the 5 chromosomes.

3. Analysis

Runs were analysed using Simple Interval Mapping (SIM)(Lander & Botstein 1989) and Composite Interval Mapping (CIM) (Zeng 1993 & 1994). Two programs were used to analyses the data, QTL Cartographer version 1.14 (Basten et al. 1999) and MQTL version 0.98 (Tinker and Mathers 1995). The two programs differ in how they deal with background markers for Composite Interval Mapping (CIM). Within MQTL the background markers are chosen at random and put into the map input file. Within QTL Cartographer the background markers are not chosen at random but rather are chosen from the Stepwise Regression analysis selecting the "best" background markers. The setting or choosing of these markers also has an influence on the level of statistical significance. Tinker argues that it is not possible to find an appropriate threshold for statistical error control when background markers are selected based on the data. Hence we used the two programs and have concentrated on QTLs that were present in both sets of analysis.

a) QTL cartographer

Qstat was used to determine whether the data had a normal distribution (if not then measures were taken to fix the distribution). Linear Regression (LR) and Stepwise Regression (SR) were performed using the default settings (Stepwise regression used forward with backward elimination) 5% significance. Simple Interval and CIM were performed using the Zmap.qtl function. The data were analysed across all chromosomes with a walking speed of 2 cM. For model 6 (CIM) the number of background parameters was left at the default of 5 along with the window size which was left on the default of 10 cM. One thousand permutations were performed within CIM (Churchill and Doerge 1994). Eqtl was then run to determine the significant QTLs.

b) MQTL

The same set of markers used in QTL Cartographer was used in MQTL. Background markers were chosen at random for CIM. The number of markers chosen was approximately half that of the number of RILs used in the set. The default setting of a walking speed of 5 cM was selected, 3000 permutations were performed to determine significance levels with type 1 error set at 5% .

QTLs that were present in both programs and from varied background marker sets from within MQTL were considered genuine. This, coupled with repeated QTL analysis across independent experiments, lead to a significant repeatable locus surrounding the ERECTA gene on Chromosome 2 (Table 5).

Data in Table 5 indicate that there is a major QTL with a LOD score significant at the probability level of 5% and, for most runs, of even 1%, on *Arabidopsis thaliana* chromosome 2. In all cases, that interval sits above the *ER* marker on chromosome 2. Depending on the experimental run, this QTL explains 18 to 64% (see column $R^2$) of the total genetic variance in transpiration efficiency.

Data in Figure 3 indicate a positive additive effect of the identified QTL based upon the mean value of the carbon isotope composition in plants carrying the Col-4 *ERECTA* allele.

EXAMPLE 8

Complementation Test: transformation of *A. thaliana* lines carrying *erecta* mutations with the wild type *ERECTA* gene under the control of the 35S promoter.

1. Methods

Two Columbia *erecta* lines were transformed using a binary vector generously given by Dr Keiko Torii. That plasmid was constructed using the vector plasmid pPZP222 (see details on this vector in Hajdukiewics et al. Plant Mol Biol 25, 989-994, 1994). The pPZP vectors carry chimeric genes in a CaMV 35S expression cassette that confer resistance to kanamycin or gentamycin in plants. The plant selectable marker (gentamycin resistance gene for the pPZP222 vector) is cloned next to the LB. Cloning sites for the gene of interest (ER in our case) is between the plant marker and the RB sequences. This ensures that that gene is transferred to plant first, followed by the *gent* gene. Resistance to gentamycin will therefore be obtained only if the ER gene is also present.

The binary vector was transferred to disarmed strain AGL1 of Agrobacterium tumefaciens by standard tri-parental matings (Ditta et al, 1980, PNAS 77,7347-7351) using the pRK2013 helper strain of *E coli.*

Arabidopsis plants were transformed using the standard floral dip method for transformation by disarmed strains of A tumefaciens (Clough and Bent, 1998, The Plant Journal 16, 735-743).

Two Columbia *erecta* lines were transformed, for which we had numerous data showing consistently more negative isotopic values in those lines (ie lower transpiration efficiency) than in near-isogenic Col ER wild type plants. These two lines were as follows:

1. er 105, a knock-out mutant due to the insertion of a large piece of DNA in the ERECTA gene and
2. line Col-er2 (3401 NASC identifier), same as er106 (Lease *et al. 2001*).

Seedlings were screened on MS plates on 100 ug/ml gentamycin sulfate. Putative transformants were transferred to soil and their progeny screened again for gentamycin resistance, for confirmation and identification of homozygous lines and T3 seed collection.

Many independent transformant lines were obtained and among those were several ER homozygous lines, which were selected for subsequent analysis (see Table 6).

A stable transgenic homozygous Landsberg ER line also obtained by transforming the Ld-er1 ecotype (NW20) with the same construct as described above was given to us by Dr Keiko Torii (line T3-7K in Table 6 or "T2+ER" in Figures 6-9).

2. Results:

Initial analysis of several ER transformants in the Col-er105, Col-er106/er2, and Ld-er1 background (as shown in Table 6 above):

Effective transformation was ascertained and ER expression levels were quantified in several independent T2 transformants using real-time quantitative PCR (ABI PRISM 7700, Sequence Detection System User Bulletin #2. 1997). Basically that technique allowed us to quantify the copy number of the ER gene in lines of interest, after normalisation to the copy number of a control gene, in the same plants (same RNA pool). 18S ribosomal RNA gene was used as a control gene after checking its expression was not affected by changes in ER expression.

Results are shown in Figures 4a, 4b and 4c, wherein the y-axis in each figure describes the *erecta* mRNA copy number (normalised to that of 18S mRNA) in wild type ER lines, er mutants, and ER transgenics in both Columbia and Landsberg backgrounds.

All ER transgenic lines, except line 145 (Figure 4a) showed increased mRNA copy number: from 4 to 170 fold increase compared with the null controls. Interestingly, all lines, even those with hugely increased mRNA levels look "normal", healthy and of similar size.

Initial phenotypic analysis shows complementation of the "transpiration efficiency phenotype". In other words, *ER* transgenic lines show less negative carbon isotopic composition values than null er control and null lines as shown in Table 7. Those values converge towards values measured for wild type ER ecotypes. Hence in a Columbia background, ER transgenics display values of -30.6 to -31.2 per mil on average compared to values of -31.7 to -32.2 per mil in the null transgenics (Table 7), and -30.9 per mil in the Col0 ER wild type (background ecotype for mutant er-105). The less negative carbon isotopic compositions in ER transgenics is indicative of greater transpiration efficiency in these plants, as expected.

The data presented in Table 7 are confirmed by direct measurement of leaf transpiration efficiency (ratio A/E of $CO_2$ assimilation rate per unit leaf area to transpiration rate) using gas exchange techniques. Stomatal density, leaf photosynthetic capacity and growth rate are also determined to analyze the underlying causes of the reversion of the transpiration efficiency phenotype (leaf development and anatomy, biochemical properties of leaves, stomatal characteristics).

EXAMPLE 9

Tissue specificity in the expression of the *ERECTA* gene in wild type rice *Oryza sativa* (cv Nipponbare):

An ortholog of the *A. thaliana ERECTA* allele (SEQ ID NO: 1) in rice was identified in *silico* by homology searching of the NCBI protein database using the BLAST programme under standard conditions. The input sequence was SEQ ID NO: 2. The nucleotide sequence of the rice ortholog is presented in SEQ ID NO: 3, with the encoded protein comprising the amino acid sequence set forth in SEQ ID NO: 4.

The mRNA copy number of the rice *ERECTA* gene was determined for various plant organs/parts, as indicated in Figures 5a and 5b. *ERECTA* mRNA copy numbers were determined by quantitative real-time PCR, using 18S mRNA as an internal control gene for normalization of data. The pattern *of ERECTA* expression in rice was similar to the pattern of gene expression in *A. thaliana,* with highest expression observed in young meristematic tissues, young leaves and even more, the inflorescences. No or very low expression is found in roots, as for *A. thaliana.*

These similarities in tissue specificity between rice and Arabidopsis indicates that the rice orthologue provided herein as SEQ ID NO: 3 is a true orthologue of the *A. thaliana ERECTA* allele set forth in SEQ ID NO: 1, with similar function

EXAMPLE 10

Demonstration of a functional role for the rice *ERECTA* gene in modulating transpiration efficiency

To determine a functional role for the rice *ERECTA* gene (SEQ ID NO: 3), lines of rice plants carrying transposon insertions that affect expression of that gene are analyzed. Nine such mutants were identified in the publicly available collection of transposon TOS 17 insertional mutants at the Japanese NIAS Institute. The TOS 17 retrotransposon is described in detail by Hirochika, Current Opinion in Plant Biology, 4, 118-122, 2001 and by Hirochika *Plant Mol Biol 35*, 231-240, 1997, . The nine mutant lines were identified through the website URL http://tos.nias.affrc.go.jp/~miyao/pub/tos17/, and they have the accession numbers NG0578 (mutant A), ND3052 (mutant B), ND4028 (mutant C), NC0661 (mutant D), NE1049 (mutant E), NF8517 (mutant F), NE8025 (mutant G), NE3033 (mutant H) and NF8002 (mutant I).

Nine transposon insertional mutants were ordered from NIAS, that carry the TOS17 stable retrotransposon insert in various parts of the *ERECTA* gene in the Nipponbare background, the genotype used for rice genome sequencing: NG0578, ND3052, ND4028, NC0661, NE1049, NF8517, NE8025, NE3033 and NF8002.

The transposon insertions in these nine lines affect the membrane spanning region of the protein (mutants I, D, E) or the Leucine Rich Repeat (LRR) domains (mutant H and G) in LRR 7 and LRR 18, respectively. In mutant B, TOS 17 alters the coding sequence just upstream of sequences encoding the protein kinase domain I. In mutants C and F, the TOS17 insertion alters the sequence encoding domain VIa of the ERECTA protein. In mutant A, the TOS7 insertion is in a sequence encoding a region between domains IX and X. The sequence information on these mutants is publicly available from the NIAS website.

Using mutant seed for lines A-I received from NIAS, plants were grown for amplification of seed and analysis. Except in two mutants where several plants died, plants look healthy, with good growth indicating that, as in Arabidopsis, there is great potential to alter the *ERECTA* gene towards altered transpiration efficiency without adversely affecting growth and/or yield.

Based upon sequence information for each mutant A-I, primers were designed to amplify the mutant *erecta* alleles from seedling material derived from 20 seeds. Amplification is performed under standard conditions, to identify for each mutant, plants that are homozygous, heterozygous or null at the *ERECTA* locus. Homozygous TOS17 mutants B and E, and heterozygous lines and null lines in all lines A-l were identified.

In parallel to gaining information on whether or not the mutant lines were homozygous or heterozygous or null mutants, specific plant parts are removed for analysis of the consequences of the mutations on the *ERECTA* gene expression (levels and tissue specificity of expression), using quantitative real-time PCR as described herein. Additionally, the transpiration efficiency phenotype of each mutant line is determined by measuring C&O isotopic composition and ash contents of plant samples.

Initial results on $^{13}C$ isotopic composition of mature blades of rice seedlings reveals significant variation between mutant lines (-32.8 to -34.2 per mil) and, in at least 4 mutants, significant deviations from the wild type values, towards more negative values, suggesting that the *erecta* mutations do affect transpiration efficiency in rice, as in Arabidopsis.

Similar methods as above are applied to anaylzing the progeny of the mutant plants, to facilitate analysis of the effects of the *erecta* mutations under a range of conditions, including flooding (as is the most common practice for Nipponbare), water stress such as from soil drying (upland rice growth conditions) or low air humidity (heat spells). Differences in plant morphology, anatomy and apical dominance are noted under each environmental condition. Parameters that are characterised include tillering patterns, the anatomy of leaves and meristems, development and growth rates.

Comparisons between mutants A-I are further used to characterize the role of the different protein domains in conferring different phenotypes observed for each line under different environmental and/or agricultural growth conditions. It is interesting that, among the 4 mutants that exhibit much lower C isotopic composition than the wild type, three are those mutants where the TOS17 insert affects the membrane spanning region.

## EXAMPLE 11

### Effect of silencing *ERECTA* gene expression on transpiration efficiency

To confirm the role of the *ERECTA* gene in conferring the transpiration efficiency phenotype on a plant, expression of the wild-type *ERECTA* allele is reduced or inhibited using standard procedures in plant molecular biology, such as, for example, antisense inhibition of *ERECTA* expression, or the expression of inhibitory interfering RNA (RNAi) that targets *ERECTA* expression at the RNA level. All such procedures will be readily carried out by the skilled artisan using the disclosed nucleotide sequences of the *ERECTA* genes provided herein or sequences complementary thereto.

For transformation of rice and *Arabidopsis,* transgenes are prepared in disarmed, non-tumorigenic binary vectors carrying T-DNA left and right borders and a selectable marker operable in *E Coli.*

Binary vectors used for DNA transfer include vectors selected from the group consisting of:

1. pPZP222 (Hajdukiewicz et al, 1994, Plant Mol Biol 25, 989-994);
2. PBI 121 (Clonetech) (Ueda et al 1999, Protoplasma 206, 201-206);
3. pOCAl8 (Olszewski et al 1988, Nucl. Acid Res, 16 10765-10782);
4. pGreen and pSoup or variants thereof (Hellens et al., 2000, Plant Mol Biol 42, 819-832) and
5. binary vectors developed on the pCAMBIA vectors backbone described at the webiste of CAMBIA.

The starting material for all these vectors was the backbone developed by Hajdukiewicz et al., 1994. The pPZP series of vectors comprise (i) a wide-host-range origin of replication from the *Pseudomonas* plasmid pVSl, which is stable in the absence of selection; (ii) the pBR322 origin of replication (pMB9-type) to allow high-yielding DNA preparations in *E. coli;* (iii) T-DNA left (LB) and right (RB) borders, including overdrive; and (iv) a CaMV35S promoter expression cassette. While the pPZP series of vectors also served as the backbones for the pCAMBIA series, they have been very extensively modified for particular applications.

Vectors containing in their T-DNA various combinations of the following components are particularly preferred:

1. hptII resistance gene cassette for conferring resistance to hygromycin on transformed plant material, wherein expression of hptII is operably under control of Ubi 1 or 3 5 S promoter;
2. a reporter gene cassette comprising nucleic acid encoding the EGFP (Enhanced Green Fluorescence Protein) and/or beta-glucuronidase (GUS and GUSPlus) reporters;
3. Gal4/VP16 transactivator cassette; and
4. one or more plant gene expression cassettes comprising either full-length or partial cDNAs of *ERECTA* genes in the sense or antisense orientation, or capable of expressing RNAi comprising sequences derived from the *ERECTA* gene, including any genomic fragments of plant DNA.

The binary vectors are transferred to disarmed strain AGL1 of *Agrobacterium tumefaciens* by standard tri-parental matings (Ditta et al, 1980, Proc. Natl Acad. Sci. 77,7347-7351) using the pRK2013 helper strain of *E coli. A thaliana* plants are transformed using the standard floral dip method for transformation by disarmed strains of *A tumefaciens* (Clough and Bent, 1998, The Plant Journal 16, 735-743). Rice is transformed by generating embryogenic calli from excised embryos and subjecting the embryogenic calli to *Agrobacterium tumefaciens* mediated transformation according to published procedures (eg Wang et al 1997, J Gen and Breed, 51 325-334, 1997). Transformed plants are analyzed to confirm that those lines expressing antisense or RNAi constructs have reduced expression of functional ERECTA protein and more closely resemble the *erecta* phenotype than do wild-type plants or plants ectopically expressing a wild-type *ERECTA* gene in the sense orientation.

EXAMPLE 12

Identification of a sorghum ortholog of *A. thaliana ERECTA*

An ortholog of the *A. thaliana ERECTA* allele (SEQ ID NO: 1) in sorghum was identified *in silico* by homology searching of the NCBI protein database using the BLAST programme under standard conditions. The input sequence was SEQ ID NO: 2. The nucleotide sequence of the sorghum ortholog is presented in SEQ ID NO: 5, with the encoded protein comprising the amino acid sequence set forth in SEQ ID NO: 6.

EXAMPLE 13

Identification of *A.thaliana ERECTA* homologs

Two homologs of the *A. thaliana ERECTA* allele (SEQ ID NO: 1) were identified in *silico* by homology searching of the NCBI protein database using the BLAST programme under standard conditions. The input sequence was SEQ ID NO: 2. The nucleotide sequences of the *A. thaliana ERECTA* homologs are presented in SEQ ID NOs: 7 and 9, with the encoded proteins comprising the amino acid sequences set forth in SEQ ID NOs: 8 and 10, respectively.

T-DNAinsertional mutants for these two homologous genes, both on chr5, have been identified in the Salk Institute mutant collection (web address: signal.salk.edu/cg:-bin/tdnaexpress). Several of these mutants were ordered: Salk_007643 and Salk_026292 for gene At5g07180; Salk_045045 and Salk_081669for gene At5g62230. Primer pairs were designed in order to determine insert copy number and homozygozity/heterozygozity in the seedlings grown from the seeds that were received. Homozygous lines with 1 insert were identified and are under characterisation in order to compare the expression patterns (tissue localisation and mRNA levels) of the two genes and of the ERECTA gene across a range of environmental conditions and determine whether the three genes are functionally related.

EXAMPLE 14

Identification of wheat orthologs of *A. thaliana ERECTA*

Partial cDNA sequence of orthologs of the *A. thaliana ERECTA* allele (SEQ ID NO: 1) in wheat were initially identified *in silico* by homology searching of the NCBI protein database using the BLAST programme under standard conditions. It was necessary, however, to conduct additional searches of private databases in order to link the partial sequences identified in the NCBI database. Correction of partial sequences located in the NCBI database was also necessary in order to generate a contig corresponding to the wheat ERECTA ortholog.

The input sequence is the *A. thaliana* (SEQ ID NO: 2) or rice (SEQ ID NO: 4) amino acid sequences or a nucleotide sequence encoding same. The nucleotide sequences of the wheat ortholog are presented in SEQ ID NOs: 11-19, with the encoded proteins comprising the amino acid sequences set forth in SEQ ID NO: 20.

The sequence set forth in SEQ ID NOs: 11 to 18 are partial cDNA sequences. The corresponding sequence of the wheat *ERECTA* ortholog (SEQ ID NO: 19) is isolated by standard nucleic acid hybridization screening of a wheat cDNA library.

To confirm the role of the wheat *ERECTA* orthologs in transpiration efficiency, expression data sets are used for *in silico* studies of *ERECTA* gene expression in a range of tissues of wheat plants grown under a range of environmental conditions, thereby providing indications of tissue specificities in expression patterns and preliminary data on the types of environments where the *ERECTA* ortholog is most likely to play a physiological role in relation to water use in this species. In these studies, nucleic acid comprising the sequence set forth in SEQ ID NO: 11 to 19, or a sequence complementary thereto, are used to produce hybridization probes and/or amplification primers.

Additionally, an *ERECTA* gene (SEQ ID NO: 11 to 19) in the sense or antisense orientation is introduced into wheat, thereby producing transformed expression lines. Gene constructs are specifically to silence *ERECTA* gene expression using RNAi technology, or alternatively, to ectopically express the entire open reading frame of the gene.

Based upon similar function, the open reading frame of the *A. thaliana ERECTA* gene (i.e., SEQ ID NO: 1) is also introduced into wheat plant material in the sense orientation, thereby ectopically expressing *A. thaliana* ERECTA in wheat.

Gene constructs are introduced into wheat following any one of a number of standard procedures, such as, for example, using *A. tumefaciens* mediated transformation as described in published AU 738153 or EP 856,060-A1 or CA 2,230,216 to Monsanto Company, or using published biolistic transformation methods as described by Pellegrineschi et al., Genome 45(2), 421-30, 2002. Accordingly, genetic transformation is readily used to generate wheat lines with altered expression of an *ERECTA* gene. About 30 to 40 different transformants are produced, depending upon the efficiency of RNAi in reducing expression *of ERECTA* in wheat.

Primary transformants (T0) are characterized to determine the number and loci at which transgenes are inserted. T1 and T2 segregating progenies are then generated from selected T0 transformants, and analyzed to determine segregation ratio and to confirm the number of loci having inserted transgenes. Those T1 and/or T2 lines having single transgene insertions are selected and used to generate and multiply seed for physiological studies.

Water use efficiency in the T1 and/or T2 lines is determined through (a) gravimetric measurements of water transpired and biomass increases; (b) $^{13}C$ isotopic discrimination in plant tissues, (i.e., by determining $\Delta$; and (c) ash content of plant tissue.

Meristem and leaf development are also analyzed, especially with respect to the differentiation and anatomy of the epidermis, the stomatal complexes and the mesophyll tissue and by examining leaf gas exchange properties. This is done using microscopy, in situ imaging techniques and concurrent on-line measurements of C isotopic discrimination ($\Delta$) and of $CO_2$ and water fluxes in and out of leaves. Information on gene regulation and the network of genes in which the *ERECTA* ortholog operates in its effects on transpiration efficiency, is determined by transcriptome analysis of a restricted set of the transgenic lines with altered *ERECTA* expression.

As described herein for *A. thaliana* and rice, correlations between physiological measurements and gene expression level or copy number confirm the role of the ortholog in conferring the transpiration efficiency phenotype in wheat.

## EXAMPLE 15

### Identification of a maize ortholog of *A. thaliana ERECTA*

Partial cDNA sequence of ortholog of the *A. thaliana ERECTA* allele (SEQ ID NO: 1) in maize were initially identified *in silico* by homology searching of the NCBI protein database using the BLAST programme under standard conditions. It was necessary, however, to conduct additional searches of private databases in order to link the partial sequences identified in the NCBI database. Correction of partial sequences located in the NCBI database was also necessary in order to generate a contig corresponding to the maize ERECTA ortholog.

The input sequence was SEQ ID NO: 2. The nucleotide sequence of a maize ortholog is presented in SEQ ID NOs: 21 to 44, with the encoded protein comprising the amino acid sequence set forth in SEQ ID NO: 45.

The sequence set forth in SEQ ID NOs: 21 to 43 are partial cDNA sequences. The corresponding sequence of the maize ortholog (SEQ ID NO: 44) is isolated by standard nucleic acid hybridization screening of a wheat cDNA library.

To confirm the role of the maize *ERECTA* ortholog in transpiration efficiency, expression data sets are used for *in silico* studies *of ERECTA* gene expression in a range of tissues of maize plants grown under a range of environmental conditions, thereby providing indications of tissue specificities in expression patterns and preliminary data on the types of environments where the *ERECTA* ortholog is most likely to play a physiological role in relation to water use in this species. In these studies, nucleic acid comprising the sequence set forth in SEQ ID NO: 15, or a sequence complementary thereto, is used to produce hybridization probes and/or amplification primers.

Additionally, collections of transposon-tagged maize mutants are searched to select those having insertions that affect expression of the *ERECTA* gene and the expression level and/or copy number of the *ERECTA* ortholog is correlated to transpiration efficiency under the range of environmental growth conditions, essentially as described herein for *A. thaliana* and rice.

Additionally, an *ERECTA* gene in the sense or antisense orientation is introduced into maize, thereby producing transformed expression lines. Gene constructs are specifically to silence *ERECTA* gene expression using RNA technology, or alternatively, to ectopically express the entire open reading frame of the gene.

Based upon similar function, the open reading frame of the *A. thaliana ERECTA* gene (i.e., SEQ ID NO: 1) is also introduced into maize plant material in the sense orientation, thereby ectopically expressing *A. thaliana* ERECTA in maize.

Gene constructs are introduced into maize following any one of a number of standard procedures, such as, for example, any of the methods described by Gordon-Kamm et al., Plant Cell 2(7), 603-618, 1990; US Patent No. 5,177,010 to University of Toledo; US Patent No. 5,981,840 to Pioneer Hi-Bred; or published US application No. 20020002711 A1 (Goldman and Graves);. Accordingly, genetic transformation is used to generate maize lines with altered expression of an *ERECTA* gene.

About 30 to 40 different transformants are produced, depending upon the efficiency of RNAi in reducing expression *of ERECTA.*

Primary transformants (T0) are characterized to determine the number and loci at which transgenes are inserted. T1 and T2 segregating progenies are then generated from selected T0 transformants, and analyzed to determine segregation ratio and to confirm of number of loci having inserted transgenes. Those T1 and/or T2 lines having single transgene insertions are selected and used to generate and multiply seed for physiological studies.

Water use efficiency in the T1 and/or T2 lines is determined through (a) gravimetric measurements of water transpired and biomass increases; (b) $^{13}C$ isotopic discrimination in plant tissues, (i.e., by determining $\Delta$); and (c) ash content of plant tissue.

Meristem and leaf development are also analyzed, especially with respect to the differentiation and anatomy of the epidermis, the stomatal complexes and the mesophyll tissue and by examining leaf gas exchange properties. This is done using microscopy, in situ imaging techniques and concurrent on-line measurements of $\Delta$ and of $CO_2$ and water fluxes in and out of leaves. Information on gene regulation and the network of genes in which the *ERECTA* ortholog operates in its effects on transpiration efficiency, is determined by transcriptome analysis of a restricted set of the transgenic lines with altered *ERECTA* expression.

As described herein for *A. thaliana* and rice, correlations between physiological measurements and gene expression level or copy number confirm the role of the ortholog in conferring the transpiration efficiency phenotype in maize.

## EXAMPLE 16

### Mechanism of enhanced transpiration efficiency and inheritance *of ERECTA* in Arabidopsis (Landsberg and Columbia backgrounds)

The present inventors performed direct measurements of transpiration efficiency (ratio of $CO_2$ assimilation rate to transpiration rate) in both Landsberg and Columbia backgrounds. To confirm the role of *ERECTA* under a wider range of agronomically relevant conditions, the transpiration efficiencies of transformed plants carrying an *ERECTA* allele in response to varying environmental conditions (i.e., soil water and ion content, atmospheric humidity and $CO_2$ levels) were determined and compared to the response of wild type plants (e.g., ER). Results of these experiments are presented in Figures 6-11, and Tables 8 and 9.

Data in Figures 6 show that the enhanced transpiration efficiency obtained by inserting a transgene carrying the wild type ER allele in the Ld-er1 mutant (line T2+ER) is mostly due to a decreased stomatal conductance. The phenotype of the transgenic line (T2+ER in graphs) is similar to that of a Ld-ER ecotype near isogenic to Ld-er1 obtained from the Stock Centre (line 3177 on graphs). The increased transpiration efficiency in transgenic ER, compared to levels observed in wild type ER line is observed under both current ambient $CO_2$ levels and increased $CO_2$ levels that are within the limits predicted to occur worldwide over the next two decades.

Data in Figure 8 show that the reduced stomatal conductance in the ER T2 transgenic line compared to the Ld-er1 line is, at least for a large part, caused by a reduced stomatal density (decrease in the number of stomata per unit area by more than half, down to similar levels as those observed in wild type Ld-ER). This decrease in stomatal density is relatively higher than that in the density of epidermal cells whose surface area is increased by only about 10%. It therefore follows that the ER transgene has affected stomatal development, specifically, and caused a decreased in stomatal index. These data show complementation with respect to the processes driving variation in transpiration efficiency.

Reciprocal crosses were also performed between the two parental lines NW20 (Ld-er1) and Col4 (Col-ER). The notation F2 (Col*Ld) refers to the F1 plants where Col was the recipient of Ld pollen, while the notation F1 (Ld*Col) indicates the converse (Ld ovary receiving Col pollen). Initial analysis of these two types of F1 plants has been made for: gas exchange and photosynthetic properties, transpiration efficiency (Figure 7) and C isotopic composition (Table 9), rosette shape and developmental rate, anatomy of leaf epidermis (Figure 8), flowering date, inflorescence and pod shape. Consistent with our analysis of complementation experiments, the data show that the ERECTA gene affects all these phenotypes and not only inflorescence and pod shape.

The data also show a complex inheritance of the *ERECTA* gene, such that the gene is dominant, with no reciprocal effect on pod shape (longer pods, longer stems and pedicels in all $F_1$ plants, similar to the Col -ER parent). However, for other traits, results indicate maternal effects: hence the transpiration efficiency values (see Figure 7a) and rosettes carbon isotope composition in $F_1$ plants (Table 9) are intermediate between the parental values, but different between the two sets of F1 plants: values for $F_1$ plants (Col*Ld) are closer to the Col values, while those for F1 plants (Ld*Col) are closer to values for the Ld parent.

Data in Figure 8 indicate that stomatal conductance (transpiration per unit leaf area, Figure 8a) displays values close to the Ld-er1 parent in all F1 plants, despite the stomatal densities being close to the Col-ER parent (Figure 8c). This shows that the ER gene affects not only epidermis development but also stomatal aperture (dynamics of stomata) and that while the ER effect on stomatal density appears to be dominant, effect on stomatal aperture is not.

Data in Figure 9 show the effect of various er mutations (in Col background, mutants obtained from the Stock Centre or Dr Torii) on the number of stomata per unit leaf area. The stomatal densities for all but two of those mutants are greater than those the ColER wild type leaves, and confirm the effect of the ERECTA gene on that parameter.

Data in Figure 10 show that enhanced transpiration efficiency in the ER transgenic line compared with null Ld-er1 (no insertion of transgene) is confirmed by the less negative C isotopic composition values measured in leaf material (compare values for lines NW20 and CS20 (Ld er1; lines 16 and 17 on x-axis) and a transgenic T2 ld-ER line, homozygous for he ER transgene (line 19 in the Figure). The C isotopic values measured in the ER transgenic line are similar to those in the near isogenic Ld-ER ecotype (line 18 in Figure 10). This demonstrates complementation on this phenotypic trait, and validates once again the use of C isotopic composition as a quantitative indicator (substitute) of transpiration efficiency.

Data in Figure 10 also the C isotopic compositions of a range of Col-er mutants, including those analysed in Figure 9 for stomatal densities. Most mutants show more negative C isotopic values than the COL-ER ecotype. This is consistent with the increased stoamtal densities described in Figure 9 and with all other comparisons of C isotopic compositions or direct measurements of transpiration efficiencies in er/ER lines and again indicative of the positive effect of the ER allele on transpiration efficiency.

A few mutants in Figure 10 stand out, eg Col-er105, or line 3140 (a line from NASC carrying the er1 and gl1-1 mutations). As genetic information is available for these mutants (nature and position of mutations) these mutants provide very useful functional information on the protein domain(s) of the ERECTA protein that are essential for conferring the transpiration efficiency phenotype and underlying processes.

The present inventors also perform direct measurements of transpiration efficiency (ratio of $CO_2$ assimilation rate to transpiration rate) in several T2 transformants generated in a Columbia background (i.e. transformation of mutant er-105 and er-2/106 above). Results from these measurements are shown in Figure 11. These data show that the phenotype can be complemented in a Columbia background, as determined by measuring transpiration efficiency, transpiration and $CO_2$ assimilation rates. Complementation is observed under conditions of both high humidity and low humidity, hence the demonstration that the ERECTA gene plays a role in the control of transpiration efficiency under both well watered and drought conditions, and that overexpression of that gene has the potential of increasing growth and resistance to drought and drought related stresses.

More particularly, the data in Figure 11 demonstrate the role of the *ERECTA* gene on transpiration efficiency across a range of humidities, including low humidities such as prevail in warm and dry areas:

- the er-105 mutant which carries a knock-out mutation of ERECTA (quasi no ER transcript) (open black squares) in Col0 background has lower transpiration efficiency than the wild type near isogenic Col0 (open triangles).
- this mutant was transformed with an ER transgene under the 35S promoter and several ER homozygous T2 lines were produced (solid circles). Those lines (5 independent transformants are included in the graph) have much increased transpiration efficiencies (+40 to 70%) compared to the null lines (solid squares) and similar to those measured for the wild type ER-Col0 line, across the whole range of leaf to air vapour pressure deficit tested In our experiments.

Additionally, null lines that carry no transgene insertion but went through transformation and selection on antibiotics display similar values as the starting er-105 mutant demonstrating that these manipulations themselves have no detectable confounding effect on transpiration efficiency.

**TABLE 1**

| QTL Analysis of Carbon Isotope Discrimination in Lister and Dean's Recombinant Inbred Lines | | | | | | |
|---|---|---|---|---|---|---|
| | | | | | | |
| **RUN No.** | **chr2 locus** | **QTL** | **chr4 locus** | **CONCLUSION** | | |
| **Experimental conditions** | **(cM)** | **analysis method** | **(cM)** | **QTLs number** | **predicted map position** | |
| **Run 1** (40 lines) Glasshouse- | | | | | | |
| 12h day length | **58.5** | SIM&CIM | | 2 | chr2: | 58.5-61.02 |
| irradiance150-350 $\mu E\ m^{-2}\ s^{-1}$ | 46.77 | SIM&CIM | | | chr2: | 46.77-50.75 |
| Seedlings transferred from agar plates | **61.02** | SIM | 108.5 | | | |
| **Run1 data** but with using different markers | | | | | | |
| | **56.94 to 58.00** | CIM&SIM | | 1 | | |
| | 46.77 to 50.75 | SIM | | | | |
| | 63.02 | | | | | |
| | | | | | | |
| | | | | | | |
| **Run 1** with different number of lines | | | | | | |
| | **58.5 to 61.02** | | | 2 | | |
| | **56-61** | | | | | |
| **Run2** Glasshouse September from seeds sown on soil | | | | | | |
| batch 1 | 50.75 | CIM (QTL cart) | | 2 | chr2: | 56.94-61.02 |
| | 61.02 | MQTL | | | chr2: | 50.75 |
| batch 2 | ?50.75 | MQTL | | NS | | |
| batch3-5 | | | | | | |
| all batches | 58.5 | MQTLcart | | NS | | |

(continued)

| | | | | | | |
|---|---|---|---|---|---|---|
| **Run2** | 56.94-58.5 | MQTL | | | | |
| **Run 3**<br>37 lines: parents and lines with crossing-overs on chromosome 2<br>5 growth conditions differing in humidity, irradiance, mode of establishment (seeds sown on soil or seedlings transplanted from agar) | | | | | | |
| batch B | 61.02-61.06 | | 108 NS | | | |
| batch C | 56.94-58.00 | | | | | |
| batch D | 63.02 | QTLcar | | | | |
| | 63.02 | MQTL | | | | |
| all batches (conditions) | 58.5 | | | lor 2 | chr2: | 56.94-58.5 |
| | 61.02 | | | 3 | chr2: | 61.02-63.02 |
| | | | | | | |
| | | | | | | |
| **Run 4**<br>same lines as Run 3 growth chambers 10h daylight | | | | | | |
| | 50.74 | | | | chr2: | 50.74 |
| **Run 5**<br>repeat of run 1 BUT ALL lines | | | | | | |
| | 50.74 | | | 1 | chr2: | 50.74 |
| **Run 7**<br>same lines as in run 1 but in growth chamber and higher light | | | | | | |
| 10h daylength | 46.77-50.75 | CIM&SIM | | 1 | chr 2: | 46.77-5065 |

(continued)

| | | | | | |
|---|---|---|---|---|---|
| **Run 7**<br>470-510 μE $m^{-2}$ $s^{-1}$ irradiance | | | | | |

**TABLE 2**

| Background | Mutation | Stock Centre name | Isogenic *ER* line and Stock Centre Name |
|---|---|---|---|
| **Landsberg** | *er1* | CS20 or NW20 [a] | 3177 or CS163 |
| | | | |
| **Columbia** | *er2*[b]/*er106* | 3401 | Col1 or 3176 |
| | | | |
| **Columbia** | *er105*[c] | | Col3 with gl1 marker<br>or Col0 |
| | | | |

a, NW20 is an *Ler* parent for Lister and Dean's recombinant lines, carrying the *er1* mutation. Lines 3177 or CS163 are the closest isogenic ER lines.
b, *er2* is *an er* allele identified by Rédéi in Col background. Coll or 3176 are the closest Col near-isogenic lines. The er2 is same mutation as mutation er-106 later reported by Torii and collaborators (Lease et al. 2001)
c, *er105* was isolated from a fast-neutron-irradiated Col seed population (Torii *et al.,* 1996).
d, Col4, the *Col* parent for the Lister and Dean's parent was systamically included in all comparisons.

**TABLE 3**

Comparison of er/ER lines in both Col and Ld background for carbon isotope discrimination values (per mil) in leaf material under a range of environmental conditions

| Run No. | Differences in mean carbon isotope discrimination values (per mil) | | | | | | | | |
|---|---|---|---|---|---|---|---|---|---|
| | (1) ***er-ER (all lines)*** | (2) **er105-Col0** | (3) **er105-Col4** | (4) **er105-3176** | (5) **er2-3176** | (6) **er1-3177** | (7) **er1-Col4 (parental lines for RILs)** | (8) **er105-Coli** | (9) **er1-Coli** |
| 1 | 0.13 | 0.16 | | | | | | 0.16 | |
| 2 | 0.89 | 1.18 | | | | | | 1.18 | |
| 3 | 0.26 | 0.11 | | | | | | 0.11 | |
| 4 | 1.12 | 1.60 | | | | | | 1.60 | |
| 5 | 1.03 | | 1.83 | 1.67 | 0.92 | 0.64 | 0.82 | 1.75 | 0.73 |
| 6 | 0.70 | 1.13 | 1.01 | 0.71 | 0.27 | 0.74 | 0.73 | 0.95 | 0.73 |
| 7 | 0.70 | 1.32 | 1.12 | 1.23 | 0.75 | 0.35 | 0.05 | 1.22 | 0.15 |
| 8 | 0.59 | 1.16 | 1.11 | 1.19 | 0.54 | 0.28 | 0.06 | 1.16 | 0.17 |
| 9 | 0.30 | 1.09 | 0.77 | 0.77 | 0.02 | 0.00 | 0.56 | 0.88 | 0.28 |
| 10 | 0.56 | 1.05 | 0.94 | 0.87 | 0.38 | 0.39 | 0.33 | 0.95 | 0.36 |

(continued)

| Run # | (1) *difference er-ER (all lines)* | (2) er105-Col0 | (3) er105-Col4 | (4) er105-3176 | (5) er2-3176 | (6) erl-3177 | (7) erl-Col4 | (8) er105-Coli | (9) erl-Coli |
|---|---|---|---|---|---|---|---|---|---|
| 11 | 0.48 | | 0.40 | | | | 0.52 | 0.40 | 0.52 |
| 12 | 0.36 | 0.82 | 1.31 | 1.08 | 0.33 | 0.05 | 0.07 | 1.07 | 0.01 |
| 13 | 0.38 | | 0.90 | 0.82 | 0.07 | 0.60 | 0.52 | 0.86 | 0.56 |
| 14 | 0.65 | 1.42 | 0.60 | | | 0.58 | 0.06 | 1.01 | 0.32 |
| 15 | 0.82 | | | | | | 0.82 | | 0.82 |
| | | | | | | | | | |
| **For all runs:** | | | | | | | | | |
| **Mean** | **0.60** | **1.01** | **1.00** | **1.04** | **0.41** | **0.40** | **0.41** | **0.95** | **0.42** |
| **S.E.** | 0.07 | 0.14 | 0.11 | 0.11 | 0.10 | 0.08 | 0.09 | 0.12 | 0.08 |
| **For Common runs:** | | | | | | | | | |
| **Mean:** | **0.58** | **1.10** | **1.12** | **1.04** | **0.41** | **0.38** | **0.39** | **1.11** | **0.37** |
| **S.E.** | 0.08 | 0.05 | 0.11 | 0.11 | 0.10 | 0.09 | 0.10 | 0.10 | 0.09 |

**TABLE 4**

**Run 9- December 2001: Leaf gas exchange measurements in er/ER Arabidopsis lines**

| | | | | **(1)** | **(2)** | **(3)** | **(4)** | **(5)** | **(6)** | **(7)** | **(8)** |
|---|---|---|---|---|---|---|---|---|---|---|---|
| | | **Genotype** | | **E** | **A** | **Gw** | **A/E** | **pa** | **pi** | **pi/pa** | **1-pi/pa** |
| | | | | **(mmol $H_{2O}$/$m^2$/s)** | **(μmolC/ $m^2$/s)** | **(molm$^2$/s)** | **(mmolC/ mol$H_2$O)** | **(μbar)** | **(μbar)** | | |
| | | | | | | | | | | | |
| **Row** (1) | **Ld-ER** | **3177** | Mean | 3.38 | 12.33 | 0.273 | 3.67 | 360 | 282 | 0.782 | 0.218 |
| | | | S.E. | 0.48 | 1.64 | 0.039 | 0.14 | 10 | 11 | 0.010 | 0.010 |
| | | | | | | | | | | | |
| **Row** (2) | **Ld-er** | **NW20** | Mean | 2.59 | 8.73 | 0.218 | 3.38 | 348 | 280 | 0.804 | 0.196 |
| | | | S.E. | 0.07 | 0.31 | 0.005 | 0.04 | 5 | 4 | 0.002 | 0.002 |
| | | | | | | | | | | | |
| **Row** (3) | **Col-ER** | **933** | Mean | 3.41 | 13.55 | 0.291 | 4.06 | 350 | 270 | 0.772 | 0.228 |
| | | | S.E. | 0.40 | 1.16 | 0.040 | 0.22 | 4 | 7 | 0.020 | 0.020 |
| | | | | | | | | | | | |
| **Row** (4) | **Col-ER** | **3176 (col1)** | Mean | 2.23 | 10.13 | 0.180 | 4.55 | 346 | 254 | 0.734 | 0.266 |
| | | | S.E. | 0.50 | 1.47 | 0.048 | 0.24 | 5 | 9 | 0.021 | 0.021 |
| **Row** (5) | **Col-er** | **er105** | Mean | 2.27 | 8.55 | 0.198 | 3.76 | 356 | 283 | 0.795 | 0.205 |
| | | | S.E. | 0.03 | 0.17 | 0.005 | 0.07 | 11 | 10 | 0.006 | 0.006 |
| **Row** (6) | **Col-er** | **er2** | Mean | 3.06 | 11.90 | 0.256 | 3.92 | 357 | 279 | 0.780 | 0.220 |
| | | | S.E. | 0.22 | 0.56 | 0.027 | 0.12 | 1 | 6 | 0.014 | 0.014 |
| **CONCLUSION:** | | | | | | | | | | | |
| **ComparisonLd-ER/Ld-er** | | | | er line has lower A/E with lower g and lower A. The difference in A/E is driven by A | | | | | | | |
| **Comparison 933/NW20** | | | | NSW20 (er1) has lower A/E with lower g and lower A | | | | | | | |
| **Comparison Col1/Ld-er1** | | | | The difference in A/E is driven by A | | | | | | | |
| **Comparison Col1/Col-er105** | | | | er105 has MUCH lower A/E with Higher g and lower A<br>i.e. the difference in A/E is driven by A and g | | | | | | | |

(continued)

**TABLE 4**
**Run 9- December 2001: Leaf gas exchange measurements in er/ER Arabidopsis lines**

| | | | | (1) | (2) | (3) | (4) | (5) | (6) | (7) | (8) |
|---|---|---|---|---|---|---|---|---|---|---|---|
| | | **Genotype** | | **E** | **A** | **Gw** | **A/E** | **pa** | **pi** | **pi/pa** | **1-pi/pa** |
| | | | | **(mmol $H_{2O}/m^2/s$)** | **(μmolC/ $m^2/s$)** | **(molm$^2$/s)** | **(mmolC/ mol$H_2O$)** | **(μbar)** | **(μbar)** | | |
| **Comparison Col1/Col-er2** | | | | er2 has lower A/E with MUCH higher g and HIGHER A<br>i.e. the difference in A/E is driven by g and is opposed or not driven by A | | | | | | | |
| NOTE: $p_a$ and $p_l$ are the ambient and intercellular partial pressures of $CO_2$, respectively. | | | | | | | | | | | |

**TABLE 5**

| **experimental run** | **LOD** | **P( 0.05)** | **P(0.01)** | **QTL position cM gap** | **LOD-LOD$_{p0.05}$** | **$R^2$** | **Total $R^2$** |
|---|---|---|---|---|---|---|---|
| 1 | 5.2861 | 4.7469 | 5.9923 | 39.32-50.65 | 0.5392 | 0.19 | 0.89 |
| 4 | 3.519 | 3.3561 | 4.2134 | 50.63-50.65 | 0.1629 | 0.29 | 0.57 |
| 7 | 9.6489 | 3.9627 | 4.9083 | 50.63-50.65 | 5.6862 | 0.53 | 0.77 |
| 9 | 6.1748 | 4.2328 | 5.0278 | 46.77-50.65 | 1.942 | 0.44 | 0.81 |
| 10 | Zero Qtl's | 3.6051 | 4.3889 | | | | |
| 16 | 11.5132 | 3.188 | 3.8896 | 48.96-50.65 | 8.3252 | 0.64 | 0.65 |
| 2(batch1) | 5.5459 | 3.3264 | 4.2907 | 50.63-51.02 | 2.2195 | 0.26 | 0.40 |
| LOD = $\log_{10}$ of the likelihood ratio | | | | | | | |

TABLE 6

| Summary table of the lines used for initial functional characterisation and analysis ofER effects: | | |
|---|---|---|
| Background ecotype | Stable T2 homozygous ER transformants | Null er control |
| Col-er105 | T8; T29; T19; T61 | T18 |
| Col-er106/er2/3401 | T165; T169; T279; T290 | T143 |
| Ld-er1 | T3-7K | NW20 |

TABLE 7

| Carbon isotope composition (per mil) of 3 mature leaves, ground together harvested 4/6/03 ie 32 days after sowing from still vegetative rosettes | | | |
|---|---|---|---|
| | **T2 ER homozygous transformants** | **Null transgenics (er)** | **background er mutant** |
| **Line:** | **T46** -31.4 **T29** -31.2 | **T18** -32.2 | **Col-er105** |
| Line: | **T145** -30.4 **T165** -31 **T279** -30.5 **T290** -30.8 | **T154** -31.5 **T143** -32 **T247** -31.7 | **Col er106/er2 /3401** |
| *Average:* | **-30.6** | **-31.7** | **-31.7** |
| *se:* | **0.15** | **0.12** | |
| **line** | **T3-7K** -30.4 | | **Ld-er1**<br>**NW20**<br>-31.3 |

TABLE 8

| Erecta alleleLine | Line name | C isotopic composition (per mil) | | | |
|---|---|---|---|---|---|
| | | Run 14 | | Run 18 | |
| | | Average | St Err | Average | St Err |
| **Col_er mutants** | 102K | | | -29.4 | .011 |
| | 103K | | | -29.0 | .10 |
| | 105C | -31.4 | 0.12 | -30.3 | .09 |
| | 105KH | -30.2 | | -29.3 | 0.11 |
| | 105KS | -29.8 | 0.07 | -29.5 | .07 |
| | 3401 | -30 | 0.21 | -29.4 | .05 |
| | 106C | -30.2 | 0.04 | -29.4 | .12 |
| | 108K | -30.2 | 0.07 | -29.5 | .06 |
| | 111KH | -30.4 | 0 | -29.5 | .11 |
| | 111KS | -30.2 | 0.11 | -29.7 | .12 |
| | 114K | -30.2 | 0.11 | -29.5 | .04 |
| | 116K | -29.7 | 0.21 | -29.0 | .13 |
| | 117K | -29.7 | 0.18 | -29.3 | .08 |
| | 3140 | | | -32.2 | .06 |
| **Col0_ER** | 1093 | -29.6 | 0 | -29.0 | .10 |
| **Ld_er1** | NW20 | -30.0 | 0.25 | -28.9 | .15 |
| | CS20 | -29.9 | 0.08 | | |
| **Ld_ER** | 3177 | **-29.4** | | **-28.2** | .09 |
| Transgenic Ld_er1 +wild type ER | 3-7K | -29.5 | 0.07 | -28.4 | .10 |

TABLE 9

| | C isotope composition (per mil) |
|---|---|
| Col_ER (line 933) | -28.1 |
| F1 (Col*Ld) | -28.5 |
| F1 (Ld*COL) | -29.3 |
| Ld-er1 (line NW20) | -29.9 |

## Claims

1. A method of selecting a plant having enhanced transpiration efficiency, comprising detecting a genetic marker for transpiration efficiency which marker comprises an ERECTA locus in the genome of the plant and selecting a plant that comprises or expresses the genetic marker, wherein the genetic marker comprises a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto.

2. The method according to claim 1 wherein the genetic marker comprises a nucleotide sequence selected from the group consisting of:

   (a) a sequence haying at least 55% identity to a sequence selected from the group consisting of SEQ ID NO: 1, SEQ ID NO:3, SEQ ID NO:5, SEQ ID NO: 7, SEQ ID NO: 9, SEQ ID NO: 11, SEQ ID NO:12, SEQ ID NO: 13, SEQ ID NO:14, SEQ ID NO:15, SEQ ID NO: 16, SEQ ID NO:17, SEQ ID NO:18, SEQ ID NO:19, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO: 30, SEQ ID NO: 31, SEQ ID NO:32, SEQ ID NO:33, SEQ ID NO: 34, SEQ ID NO: 35, SEQ ID NO:36, SEQ ID NO: 37, SEQ ID NO:38; SEQ ID NO:39, SEQ ID NO: 40, SEQ ID NO: 41, SEQ ID NO:42, SEQ ID NO: 43, and SEQ ID NO:44;
   (b) a sequence encoding an amino acid sequence having at least 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45; and
   (c) a sequence complementary to (a) or (b).

3. The method according to claim 1 or claim 2 wherein the plant is selected from the group consisting of *Arabidopsis thaliana,* rice, sorghum, wheat and maize.

**4.** The method according to any one of claims 1 to 3 comprising linking the transpiration efficiency phenotype of the plant to the expression of the marker in the plant.

**5.** The method according to any one of claims 1 to 3 comprising linking a structural polymorphism in DNA to a transpiration efficiency phenotype in the plant.

**6.** The method according to claim 5 wherein the polymorphism is determined by a process comprising detecting a restriction fragment length polymorphism (RFLP), amplified fragment length polymorphism (AFLP), single strand chain polymorphism (SSCP) or microsatellite analysis.

**7.** The method according to any one of claims 1 to 6 comprising hybridizing a probe or primer of at least about 20 nucleotides in length from any one of SEQ ID Nos: 1, 3, 5, 7, 9, 11 to 19 or 21 to 44 or a complementary sequence thereto to genomic DNA from the plant, and detecting the hybridization using a detection means.

**8.** The method according to any one of claims 1 to 7 wherein the selected plant has enhanced transpiration efficiency compared to a near-isogenic plant that does not comprise or express the genetic marker.

**9.** As method according to claim 1, wherein detecting the genetic marker comprises:

(a) screening mutant or near-isogenic or recombinant inbred lines of plants to segregate alleles at an ERECTA locus; and
(b) identifying a polymorphic maker linked to said ERECTA locus.

**10.** A method of modulating the transpiration efficiency of a plant comprising introducing an isolated ERECTA gene or an allelic variant thereof or the protein-encoding region thereof to a plant and selecting a plant having a different transpiration efficiency compared to a near-isogenic plant that does not comprise the introduced ERECTA gene or allelic variant or protein-encoding region, wherein the ERECTA gene or allelic variant or protein-encoding region comprises a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto, wherein the ERECTA gene or allelic variant or protein-encoding region is introduced to the plant by a process comprising transforming plant material with a gene construct comprising the gene or allelic variant or protein-encoding region thereof.

**11.** The method according to claim 10 wherein the ERECTA gene or allelic variant or protein-encoding region comprises a nucleotide sequence selected from the group consisting of:

(c) a sequence having at least 55% identity to a sequence selected from the group consisting of SEQ ID NO: 1, SEQ ID NO:3, SEQ ID NO:5, SEQ ID NO: 7, SEQ ID NO: 9, SEQ ID NO: 11, SEQ ID NO:12, SEQ ID NO: 13, SEQ ID NO:14, SEQ ID NO:15, SEQ ID NO: 16, SEQ ID NO:17, SEQ ID NO:18, SEQ ID NO:19, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO: 30, SEQ ID NO: 31, SEQ ID NO:32, SEQ ID NO:33, SEQ ID NO: 34, SEQ ID NO: 35, SEQ ID NO:36, SEQ ID NO: 37, SEQ ID NO:38; SEQ ID NO:39, SEQ ID NO: 40, SEQ ID NO: 41, SEQ ID NO:42, SEQ ID NO: 43, and SEQ ID NO:44; and
(d) a sequence encoding an amino acid sequence having at least 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

**12.** The method according to claim 10 or 11 wherein the plant is selected from the group consisting of *Arabidopsis thaliana,* rice, sorghum, wheat and maize.

**13.** The method according to any one of claims 10 to 12 further comprising expressing the introduced gene or allelic variant or protein encoding region in the plant.

**14.** The method according to any one of claims 10 to 13 wherein transpiration efficiency is enhanced in the plant.

**15.** The method of claim 14 wherein the transpiration efficiency is enhanced as a consequence of the ectopic expression of an ERECTA allele or the protein-encoding region thereof in the plant.

**16.** The method according to any one of claims 10 to 13 wherein transpiration efficiency is reduced in the plant.

**17.** The method of claim 16 wherein the transpiration efficiency is reduced as a consequence of reduced expression of an ERECTA allele in the plant.

**18.** Use of an isolated ERECTA gene or allelic variant or protein-encoding region thereof, comprising a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto, in the preparation of a genetic construct for modulating the transpiration efficiency of a plant.

**19.** Use according to claim 18 wherein the ERECTA gene or allelic variant or protein-encoding region comprises a nucleotide sequence selected from the group consisting of:

(a) a sequence having at least 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38; SEQ ID NO: 39, SEQ ID NO: 40, SEQ ID NO: 41, SEQ ID NO: 42, SEQ ID NO: 43, and SEQ ID NO: 44; and
(b) a sequence encoding an amino acid sequence having at least 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

**20.** A method of increasing the resistance of a plant to an environmental stress comprising enhancing the level of expression of an ERECTA gene or allelic variant thereof or protein encoding region thereof in said plant, wherein the environmental stress is selected from increased rainfall, decreased rainfall or drought, and wherein the ERECTA gene or allelic variant thereof or protein encoding region thereof comprises a nucleotide sequence having at least 55% overall sequence identify to at least 20 nucleotides in length of any one of SEQ ID Nos: 1, 3, 5, 7, 9, 11 to 19 or 21 to 44 or a complementary sequence thereto.

**21.** A method of increasing seed or grain weight in a plant comprising enhancing the level of expression in said plant of an ERECTA gene or allelic variant thereof or protein encoding region thereof comprising a nucleotide sequence having at least 55% overall sequence identity to at least 20 nucleotides in length of any one of SEQ ID Nos: 1, 3, 5, 7, 9, 11 to 19 or 21 to 44 or a complementary sequence thereto.

**22.** The method of claim 20 or claim 21, wherein the level of expression is enhanced by introducing an ERECTA gene or allelic variant thereof or the protein encoding region thereof to a plant.

**23.** The method of claim 22, wherein the ERECTA gene or allelic variant or protein-encoding region comprises a nucleotide sequence selected from the group consisting of:

(a) a sequence having at least 55% identity to a sequence selected from the group consisting of SEQ ID NO: 1, SEQ ID NO: 3, SEQ ID NO: 5, SEQ ID NO: 7, SEQ ID NO: 9, SEQ ID NO: 11, SEQ ID NO:12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO:36, SEQ ID NO: 37, SEQ ID NO: 38; SEQ ID NO: 39, SEQ ID NO: 40, SEQ ID NO: 41, SEQ ID NO: 42, SEQ ID NO: 43, and SEQ ID NO:44; and
(b) a sequence encoding an amino acid sequence having at least 55% identity to an amino acid sequence selected from the group consisting of SEQ ID NO: 2, SEQ ID NO: 4, SEQ ID NO: 6, SEQ ID NO: 8, SEQ ID NO: 10, SEQ ID NO: 12, SEQ ID NO: 20 and SEQ ID NO: 45.

## Patentansprüche

**1.** Verfahren zum Auswählen einer Pflanze mit erhöhter Transpirationseffizienz, bei dem man einen genetischen Marker für die Transpirationseffizienz erfasst, welcher Marke einen ERECTA-Locus in dem Genom der Pflanze umfasst, und eine Pflanze auswählt, die den genetischen Marker umfasst oder exprimiert, wobei der genetische Marker eine Nukleotidsequenz mit wenigstens 55% Gesamtsequenzidentität mit wenigstens 20 Nukleotiden in Länge von einer beliebigen der SEQ ID Nr.: 1, 3, 5, 7, 9, 11 bis 19 oder 21 bis 44 oder einer komplementären Sequenz dazu umfasst.

**2.** Verfahren nach Anspruch 1, wobei der genetische Marker eine Nukleotidsequenz umfasst, die aus der folgenden Gruppe ausgewählt ist:

(a) eine Sequenz mit wenigstens 55% Identität mit einer Sequenz, die ausgewählt ist aus der Gruppe, bestehend aus SEQ ID Nr.: 1, SEQ ID Nr.: 3, SEQ ID Nr.: 5, SEQ ID Nr.: 7, SEQ ID Nr.: 9, SEQ ID Nr.: 11, SEQ ID Nr.: 12, SEQ ID Nr.: 13, SEQ ID Nr.: 14, SEQ ID Nr.: 15, SEQ ID Nr.: 16, SEQ ID Nr.: 17, SEQ ID Nr.: 18, SEQ ID Nr.: 19, SEQ ID Nr.: 21, SEQ ID Nr.: 22, SEQ ID Nr.: 23, SEQ ID Nr.: 24, SEQ ID Nr.: 25, SEQ ID Nr.: 26, SEQ ID Nr.: 27, SEQ ID Nr.: 28, SEQ ID Nr.: 29, SEQ ID Nr.: 30, SEQ ID Nr.: 31, SEQ ID Nr.: 32, SEQ ID Nr.: 33, SEQ ID Nr.: 34, SEQ ID Nr.: 35, SEQ ID Nr.: 36, SEQ ID Nr.: 37, SEQ ID Nr.: 38, SEQ ID Nr.: 39, SEQ ID Nr.: 40, SEQ ID Nr.: 41, SEQ ID Nr.: 42, SEQ ID Nr.: 43 und SEQ ID Nr.: 44,
(b) eine Sequenz, kodierend eine Aminosäuresequenz mit wenigstens 55% Identität mit einer Aminosäuresequenz, die ausgewählt ist aus der Gruppe, bestehend aus SEQ ID Nr.: 2, SEQ ID Nr.: 4, SEQ ID Nr.: 6, SEQ ID Nr.: 8, SEQ ID Nr.: 10, SEQ ID Nr.: 12, SET ID Nr.: 20, SEQ ID Nr.: 45, und
(c) eine Sequenz, die zu (a) oder (b) komplementär ist.

**3.** Verfahren nach Anspruch 1 oder Anspruch 2, wobei die Pflanze ausgewählt ist aus der Gruppe, bestehend aus *Arabidopsis thaliana,* Reis, Hirse, Weizen und Mais.

**4.** Verfahren nach einem der Ansprüche 1 bis 3, umfassend die Verknüpfung des Transpirationseffizienzphänotyps der Pflanze mit der Expression des Markers in der Pflanze.

**5.** Verfahren nach einem der Ansprüche 1 bis 3, umfassend das Verknüpfen eines strukturellen Polymorphismus der DNA mit einem Transpirationseffiizienzphänotypen in der Pflanze.

**6.** Verfahren nach Anspruch 5, wobei der Polymorphismus bestimmt wird durch ein Verfahren, umfassend die Erfassung eines Restriktionsfiragmentlängen-Polymorphismus (RFLP), eines Längen-Polymorphismus amplifizierten Fragmente (AFLP), eines Einzelstrang-Ketten-Polymorphismus (SSPC) oder die Mikrosatelliten-Analyse.

**7.** Verfahren nach einem der Ansprüche 1 bis 6, bei dem man eine Sonde oder einen Primer von wenigstens etwa 20 Nukleotiden in Länge aus einer beliebigen von SEQ ID Nr.: 1, 3, 5, 7, 9, 11 bis 19 oder 21 bis 44 oder von einer komplementären Sequenz dazu mit genomischer DNA aus der Pflanze hybridisiert und die Hybridisierung unter Verwendung von Detektionsmitteln erfasst.

**8.** Verfahren nach einem der Ansprüche 1 bis 7, wobei die ausgewählte Pflanze im Vergleich zu einer nahezu isogenen Pflanze, die den genetischen Marker nicht umfasst oder exprimiert, eine verbesserte Transpirationseffiizienz aufweist.

**9.** Verfahren nach Anspruch 1, wobei das Erfassen des genetischen Markers umfasst:

(a) das Suchen nach Mutanten oder nahezu isogenen oder rekombinanten Inzuchtlinien von Pflanzen, um Allele an einem ERECTA-Locus zu segregieren, und
(b) das Identifizieren eines mit dem ERECTA-Locus verknüpften polymorphen Markers.

**10.** Verfahren zum Modulieren der Transpirationseffizienz einer Pflanze, bei dem man ein isoliertes ERECTA-Gen oder eine allele Variante davon oder den proteinkodierenden Bereich davon in eine Pflanze einbringt und eine Pflanze auswählt, die im Vergleich zu einer nahezu isogenen Pflanze, die das eingebrachte ERECTA-Gen oder die allele Variante oder den proteinkodierenden Bereich nicht umfasst, eine unterschiedliche Transpirationseffizienz aufweist, wobei das ERECTA-Gen oder die allele Variante oder der proteinkodierende Bereich eine Nukleotidsequenz aufweist mit wenigstens 55% Gesamtsequenzidentität zu wenigstens 20 Nukleotiden in Länge von einer beliebigen der SEC ID Nr.: 1, 3, 5, 7, 9, 11 bis 19 oder 21 bis 44 oder einer komplementären Sequenz dazu, wobei das ERECTA-Gen oder die allele Variante oder der proteinkodierende Bereich in die Pflanze durch ein Verfahren eingebracht wird, welches das Transformieren von Pflanzenmaterial mit einem Genkonstrukt, welches das Gen oder die allele Variante oder den proteinkodierenden Bereich davon enthält, umfasst.

**11.** Verfahren nach Anspruch 10, wobei das ERECTA-Gen oder die allele Variante oder der proteinkodierende Bereich eine Nukleotidsequenz umfasst, die aus der Gruppe ausgewählt ist, die besteht aus:

(c) eine Sequenz, die wenigstens 55% Identität mit einer Sequenz aufweist, die ausgewählt ist aus der Gruppe, bestehend aus SEQ ID Nr.: 1, SEQ ID Nr.: 3, SEQ ID Nr.: 5, SEQ ID Nr.: 7, SEQ ID Nr.: 9, SEQ ID Nr.: 11, SEQ ID Nr.: 12, SEQ ID Nr.: 13, SEQ ID Nr.: 14, SEQ ID Nr.: 15, SEQ ID Nr.: 16, SEQ ID Nr.: 17, SEQ ID Nr.: 18, SEQ ID Nr.: 19, SEQ ID Nr.: 21, SEQ ID Nr.: 22, SEQ ID Nr.: 23, SEQ ID Nr.: 24, SEQ ID Nr.: 25, SEQ ID Nr.: 26, SEQ ID Nr.: 27, SEQ ID Nr.: 28, SEQ ID Nr.: 29, SEQ ID Nr.: 30, SEQ ID Nr.: 31, SEQ ID Nr.: 32, SEQ ID Nr.: 33, SEQ ID Nr.: 34, SEQ ID Nr.: 35, SEQ ID Nr.: 36, SEQ ID Nr.: 37, SEQ ID Nr.: 38, SEQ ID Nr.: 39, SEQ ID Nr.: 40, SEQ ID Nr.: 41, SEQ ID Nr.: 42, SEQ ID Nr.: 43 und SEQ ID Nr.: 44, und
(d) eine Sequenz, die eine Aminosäuresequenz kodiert, welche wenigstens 55% Identität mit einer Aminosäuresequenz aufweist, die aus der Gruppe ausgewählt ist, bestehend aus SEQ ID Nr.: 2, SEQ ID Nr.: 4, SEQ ID Nr.: 6, SEQ ID Nr.: 8, SEQ ID Nr.: 10, SEQ ID Nr.: 12, SEQ ID Nr.: 20 und SEQ ID Nr.: 45.

**12.** Verfahren nach einem der Ansprüche 10 oder 11 wobei die Pflanze ausgewählt ist aus der Gruppe, bestehend aus *Arabidopsis thaliana,* Reis, Hirse, Weizen und Mais.

**13.** Verfahren nach einem der Ansprüche 10 bis 12, bei dem man außerdem das eingebrachte Gen oder die allele Variante oder den proteinkodierenden Bereich in der Pflanze exprimiert.

**14.** Verfahren nach einem der Ansprüche 10 bis 13, wobei die Transpirationseffizienz in der Pflanze verbessert wird.

**15.** Verfahren nach Anspruch 14, wobei die Transpirationseffizienz als eine Konsequenz der ektopischen Expression eines ERECTA-Allels oder des proteinkodierenden Bereichs davon in der Pflanze verbessert wird.

**16.** Verfahren nach einem der Ansprüche 10 bis 13, wobei die Transpirationseffizienz in der Pflanze reduziert wird.

**17.** Verfahren nach Anspruch 16, wobei die Transpirationseffizienz als eine Konsequenz der reduzierten Expression eines ERECTA-Allels in der Pflanze reduziert wird.

**18.** Verwendung eines isolierten ERECTA-Gens oder einer allelen Variante oder eines proteinkodierenden Bereichs davon, umfassend eine Nukleotidsequenz mit wenigstens 55% Gesamtsequenzidentität mit wenigstens 20 Nukleotiden in Länge von einer beliebigen der SEQ ID Nr.: 1, 3, 5, 7, 9, 11 bis 19 oder 21 bis 44 oder einer komplementären Sequenz davon, bei der Herstellung eines genetischen Konstrukts für die Modulierung der Transpirationseffizienz in einer Pflanze.

**19.** Verwendung nach Anspruch 18, wobei das ERECTA-Gen oder die allele Variante oder der proteinkodierende Bereich eine Nukleotidsequenz umfasst, die ausgewählt ist aus der Gruppe, bestehend aus:

(a) eine Sequenz mit wenigstens 55% Identität mit einer Sequenz, die ausgewählt ist aus der Gruppe, bestehend aus SEQ ID Nr.: 1, SEQ ID Nr.: 3, SEQ ID Nr.: 5, SEQ ID Nr.: 7, SEQ ID Nr.: 9, SEQ ID Nr.: 11, SEQ ID Nr.: 12, SEQ ID Nr.: 13, SEQ ID Nr.: 14, SEQ ID Nr.: 15, SEQ ID Nr.: 16, SEQ ID Nr.: 17, SEQ ID Nr.: 18, SEQ ID Nr.: 19, SEQ ID Nr.: 21, SEQ ID Nr.: 22, SEQ ID Nr.: 23, SEQ ID Nr.: 24, SEQ ID Nr.: 25, SEQ ID Nr.: 26, SEQ ID Nr.: 27, SEQ ID Nr.: 28, SEQ ID Nr.: 29, SEQ ID Nr.: 30, SEQ ID Nr.: 31, SEQ ID Nr.: 32, SEQ ID Nr.: 33, SEQ ID Nr.: 34, SEQ ID Nr.: 35, SEQ ID Nr.: 36, SEQ ID Nr.: 37, SEQ ID Nr.: 38, SEQ ID Nr.: 39, SEQ !D Nr.: 40, SEQ ID Nr.: 41, SEQ ID Nr.: 42, SEQ ID Nr.: 43 und SEQ ID Nr.: 44, und
(b) eine Sequenz, die eine Aminosäuresequenz kodiert, welche wenigstens 55% Identität mit einer Aminosäuresequenz aufweist, die ausgewählt ist aus der Gruppe, bestehend aus SEQ ID Nr.: 2, SEQ ID Nr.: 4, SEQ ID Nr.: 6, SEQ ID Nr.: 8, SEQ ID Nr.: 10, SEQ ID Nr.: 12, SEQ ID Nr.: 20 und SEQ ID Nr.: 45.

**20.** Verfahren zum Erhöhen der Resistenz einer Pflanze gegenüber einem Umweltstress, bei dem man das Niveau der Expression eines ERECTA-Gens oder einer allelen Variante davon oder eines proteinkodierenden Bereichs davon in der Pflanze erhöht, wobei der Umweltstress ausgewählt ist unter erhöhtem Niederschlag, verringertem Niederschlag oder Trockenheit, und wobei das ERECTA-Gen oder die allele Variante davon oder der proteinkodierende Bereich davon eine Nukleotidsequenz umfasst, die wenigstens 55% Gesamtsequenzidentiät mit wenigstens 20 Nukleotiden in Länge von einer beliebigen der SEQ ID Nr.: 1, 3, 5, 7, 9, 11 bis 19 oder 21 bis 44 oder einer komplementären Sequenz dazu aufweist.

**21.** Verfahren zum Erhöhen des Samen- oder Korngewichts in einer Pflanze, bei dem man in dieser Pflanze das Niveau der Expression von einem ERECTA-Gen oder einer allelen Variante davon oder eines proteinkodierenden Bereichs davon, umfassend eine Nukleotidsequenz mit wenigstens 55% Gesamtsequenzidentität mit wenigstens 20 Nukleotiden in Länge von einer beliebigen von SEQ ID Nr.: 1, 3, 5, 7, 9, 11 bis 19 oder 21 bis 44 oder einer komplementären Sequenz dazu, verstärkt.

**22.** Verfahren nach Anspruch 20 oder Anspruch 21, wobei das Niveau der Expression verstärkt wird durch das Einbringen eines ERECTA-Gens oder einer allelen Variante davon oder des prateinkodierenden Bereichs davon in eine Pflanze.

**23.** Verfahren nach Anspruch 22, wobei das ERECTA-Gen oder eine allele Variante oder ein proteinkodierender Bereich eine Nukleotidsequenz umfasst, die ausgewählt ist aus der Gruppe, bestehend aus:

(a) eine Sequenz mit wenigstens 55% Identität mit einer Sequenz, die ausgewählt ist aus der Gruppe, bestehend aus SEQ ID Nr.: 1, SEQ ID Nr.: 3, SEQ ID Nr.: 5, SEQ ID Nr.: 7, SEQ ID Nr.: 9, SEQ ID Nr.: 11, SEQ ID Nr.: 12, SEQ ID Nr.: 13, (SEQ ID Nr.: 14, SEQ ID Nr.: 15, SEQ ID Nr.: 16, SEQ ID Nr.: 17, SEQ ID Nr.: 18, SEQ ID Nr.: 19, SEQ ID Nr.: 21, SEQ ID Nr.: 22, SEQ ID Nr.: 23, SEQ ID Nr.: 24, SEQ ID Nr.: 25, SEQ ID Nr.: 26, SEQ ID Nr.: 27, SEQ ID Nr.: 28, SEQ ID Nr.: 29, SEQ ID Nr.: 30, SEQ ID Nr.: 31, SEQ ID Nr.: 32, SEQ ID Nr.: 33, SEQ ID Nr.: 34, SEQ ID Nr.: 35, SEQ ID Nr.: 36, SEQ ID Nr.: 37, SEQ ID Nr.: 38, SEQ ID Nr.: 39, SEQ ID Nr.: 40, SEQ ID Nr.: 41, SEQ ID Nr.: 42, SEQ ID Nr.: 43 und SEQ ID Nr.: 44, und
(b) eine Sequenz, die eine Aminosäuresequenz kodiert, welche wenigstens 55% Identität mit einer Aminosäuresequenz aufweist, die aus der Gruppe ausgewählt ist, bestehend aus SEQ ID Nr.: 2, SEQ ID Nr.: 4, SEQ ID Nr.: 6, SEQ ID Nr.: 8, SEQ ID Nr.: 10, SEQ ID Nr.: 12, SEQ ID Nr.: 20 und SEQ ID Nr.: 45.

## Revendications

**1.** Procédé pour sélectionner une plante présentant une efficacité de transpiration améliorée, consistant à détecter un marqueur génétique d'une efficacité de transpiration, lequel marqueur comprend un locus ERECTA dans le génome de la plante, et sélectionner une plante qui comprend ou exprime le marqueur génétique, où le marqueur génétique comprend une séquence de nucléotides ayant au moins 55 % d'identité de séquence globale avec au moins 20 nucléotides en longueur de l'une quelconque des SEQ ID NO: 1, 3, 5, 7, 9, 11 à 19 ou 21 à 44, ou une séquence complémentaire à celles-ci.

**2.** Procédé selon la revendication 1, dans lequel le marqueur génétique comprend une séquence de nucléotides sélectionnée dans le groupe consistant en :

(a) une séquence ayant au moins 55 % d'identité avec une séquence sélectionnée dans le groupe consistant en SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO:21, SEQ ID NO: 22, SEQ ID NO: 23, SEQ ID NO: 24, SEQ ID NO:25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO:38, SEQ ID NO: 39, SEQ ID NO:40, SEQ ID NO:41, SEQ ID NO: 42, SEQ ID NO: 43, et SEQ ID NO: 44 ;
(b) une séquence codant pour une séquence d'acides aminés ayant au moins 55 % d'identité avec une séquence d'acides aminés sélectionnée dans le groupe consistant en SEQ ID NO: 2, SEQ ID NO: 4, SEQ ID NO: 6, SEQ ID NO: 8, SEQ ID NO: 10, SEQ ID NO: 12, SEQ ID NO: 20 et SEQ ID NO: 45 ; et
(c) une séquence complémentaire à (a) ou (b).

**3.** Procédé selon la revendication 1 ou la revendication 2, dans lequel la plante est sélectionnée dans le groupe consistant en *Arabidopsis thaliana,* le riz, le sorgho, le blé et le maïs.

**4.** Procédé selon l'une quelconque des revendications 1 à 3, comprenant d'associer le phénotype d'efficacité de transpiration de la plante à l'expression du marqueur chez la plante.

**5.** Procédé selon l'une quelconque des revendications 1 à 3, comprenant d'associer un polymorphisme structurel dans l'ADN à un phénotype d'efficacité de transpiration chez la plante.

**6.** Procédé selon la revendication 5, dans lequel le polymorphisme est déterminé par un procédé comprenant de détecter un polymorphisme de longueur des fragments de restriction (RFLP), un polymorphisme de longueur des fragments amplifiés (AFLP), un polymorphisme de conformation des simples brins (SSCP) ou une analyse des microsatellites.

**7.** Procédé selon l'une quelconque des revendications 1 à 6, comprenant d'hybrider une sonde ou une amorce ayant une longueur d'au moins environ 20 nucléotides parmi l'une quelconque des SEQ ID NO: 1, 3, 5, 7, 9, 11 à 19 ou 21 à 44, ou une séquence complémentaire à celles-ci, à un ADN génomique de la plante, et à détecter l'hybridation en utilisant un moyen de détection.

**8.** Procédé selon l'une quelconque des revendications 1 à 7, dans lequel la plante sélectionnée présente une efficacité de transpiration améliorée par comparaison à une plante presque isogénique qui ne comprend ou n'exprime pas le marqueur génétique.

**9.** Procédé selon la revendication 1, dans lequel la détection du marqueur génétique comprend :

(a) de cribler des lignées consanguines, presque isogéniques, ou recombinantes de plantes mutantes afin d'isoler des allèles au niveau d'un locus ERECTA ; et
(b) d'identifier un marqueur polymorphe associé audit locus ERECTA.

**10.** Procédé pour moduler l'efficacité de transpiration d'une plante, comprenant d'introduire un gène ERECTA isolé ou un variant allélique de celui-ci ou la région codant pour une protéine de celui-ci dans une plante, et sélectionner une plante ayant une efficacité de transpiration différente par comparaison à une plante presque isogénique qui ne comprend pas le gène ERECTA introduit, ou le variant allélique ou la région codant pour une protéine, où le gène ERECTA ou variant allélique ou région codant pour une protéine comprend une séquence de nucléotides ayant au moins 55 % d'identité de séquence globale avec au moins 20 nucléotides en longueur de l'une quelconque des SEQ ID NO: 1, 3, 5, 7, 9, 11 à 19 ou 21 à 44, ou une séquence complémentaire à celles-ci, où le gène ERECTA ou variant allélique ou région codant pour une protéine est introduit(e) dans la plante par un procédé comprenant de transformer le matériel végétal avec un produit de recombinaison génétique comprenant le gène ou variant allélique ou région codant pour une protéine de celui-ci.

**11.** Procédé selon la revendication 10, dans lequel le gène ERECTA ou variant allélique ou région codant pour une protéine comprend une séquence de nucléotides sélectionnée dans le groupe consistant en :

(a) une séquence ayant au moins 55 % d'identité avec une séquence sélectionnée dans le groupe consistant en SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, et SEQ ID NO: 44; et
(b) une séquence codant pour une séquence d'acides aminés ayant au moins 55 % d'identité avec une séquence d'acides aminés sélectionnée dans le groupe consistant en SEQ ID NO: 2, SEQ ID NO: 4, SEQ ID NO: 6, SEQ ID NO: 8, SEQ ID NO: 10, SEQ ID NO: 12, SEQ ID NO: 20 et SEQ ID NO: 45.

**12.** Procédé selon la revendication 10 ou 11, dans lequel la plante est sélectionnée dans le groupe consistant en *Arabidopsis thaliana,* le riz, le sorgho, le blé et le maïs.

**13.** Procédé selon l'une quelconque des revendications 10 à 12, comprenant en outre d'exprimer le gène introduit ou le variant allélique ou la région codant pour une protéine chez la plante.

**14.** Procédé selon l'une quelconque des revendications 10 à 13, dans lequel l'efficacité de transpiration est améliorée chez la plante.

**15.** Procédé selon la revendication 14, dans lequel l'efficacité de transpiration est améliorée en conséquence de l'expression ectopique d'un allèle ERECTA ou de la région codant pour une protéine de celui-ci chez la plante.

**16.** Procédé selon l'une quelconque des revendications 10 à 13, dans lequel l'efficacité de transpiration est réduite chez la plante.

**17.** Procédé selon la revendication 16, dans lequel l'efficacité de transpiration est réduite en conséquence d'une expression réduite d'un allèle ERECTA chez la plante.

**18.** Utilisation d'un gène ERECTA isolé ou d'un variant allélique ou d'une région codant pour une protéine de celui-ci, comprenant une séquence de nucléotides ayant au moins 55 % d'identité de séquence globale avec au moins 20 nucléotides en longueur de l'une quelconque des SEQ ID NO: 1, 3, 5, 7, 9, 11 à 19 ou 21 à 44, ou une séquence complémentaire celles-ci, dans la préparation d'un produit de recombinaison génétique permettant de moduler l'efficacité de transpiration d'une plante.

**19.** Utilisation selon la revendication 18, dans laquelle le gène ERECTA ou variant allélique ou région codant pour une protéine comprend une séquence de nucléotides sélectionnée dans le groupe consistant en :

(a) une séquence ayant au moins 55 % d'identité avec une séquence sélectionnée dans le groupe consistant en SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID Nô: 15, SEQ ID N0: 1 f, SEQ ID NO: 17, SEQ ID NO: 18, SEQ ID NO: 19, SEQ ID NO: 2I, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO:41, SEQ ID NO: 42, SEQ ID NO: 43, et SEQ ID NO: 44 ; et
(b) une séquence codant pour une séquence d'acides aminés ayant au moins 55 % d'identité avec une séquence d'acides aminés sélectionnée dans le groupe consistant en SEQ ID NO: 2, SEQ ID NO: 4, SEQ ID NO: 6, SEQ ID NO: 8, SEQ ID NO: 10, SEQ ID NO: 12, SEQ ID NO: 20 et SEQ ID NO: 45.

**20.** Procédé pour augmenter la résistance d'une plante à un stress environnemental, consistant à améliorer le taux d'expression d'un gène ERECTA ou d'un variant allélique de celui-ci ou d'une région codant pour une protéine de celui-ci chez ladite plante, où le stress environnemental est sélectionné parmi des précipitations augmentées, des précipitations réduites ou la sécheresse, et où le gène ERECTA ou variant allélique de celui-ci ou région codant pour une protéine de celui-ci comprend une séquence de nucléotides ayant au moins 55 % d'identité de séquence globale avec au moins 20 nucléotides en longueur de l'une quelconque des SEQ ID NO: 1, 3, 5, 7, 9, 11 à 19 ou 21 à 44, ou une séquence complémentaire à celles-ci.

**21.** Procédé pour augmenter le poids d'une graine ou d'un grain chez une plante, comprenant d'améliorer le taux d'expression chez ladite plante d'un gène ERECTA ou d'un variant allélique de celui-ci ou d'une région codant pour une protéine de celui-ci, comprenant une séquence de nucléotides ayant au moins 55 % d'identité de séquence globale avec au moins 20 nucléotides en longueur de l'une quelconque des SEQ ID NO: 1, 3, 5, 7, 9, 11 à 19 ou 21 à 44, ou une séquence complémentaire à celles-ci.

**22.** Procédé selon la revendication 20 ou la revendication 21, dans lequel le taux d'expression est amélioré en introduisant un gène ERECTA ou un variant allélique de celui-ci ou la région codant pour une protéine de celui-ci dans une plante.

**23.** Procédé selon la revendication 22, dans lequel le gène ERECTA ou variant allélique ou région codant pour une protéine comprend une séquence de nucléotides sélectionnée dans le groupe consistant en :

(a) une séquence ayant au moins 55 % d'identité avec une séquence sélectionnée dans le groupe consistant en SEQ ID NO: 1, SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 21, SEQ ID NO:22, SEQ ID NO: 23, SEQ ID NO:24, SEQ ID NO: 25, SEQ ID NO: 26, SEQ ID NO: 27, SEQ ID NO: 28, SEQ ID NO: 29, SEQ ID NO: 30 SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO:40, SEQ ID NO: 41, SEQ ID NO: 42, SEQ ID NO: 43, et SEQ ID NO:44, et
(b) une séquence codant pour une séquence d'acides aminés ayant au moins 55 % d'identité avec une séquence d'acides aminés sélectionnée dans le groupe consistant en SEQ ID NO: 2, SEQ ID NO: 4, SEQ ID NO: 6, SEQ ID NO: 8, SEQ ID NO: 10, SEQ ID NO: 12, SEQ ID NO: 20 et SEQ ID NO: 45.

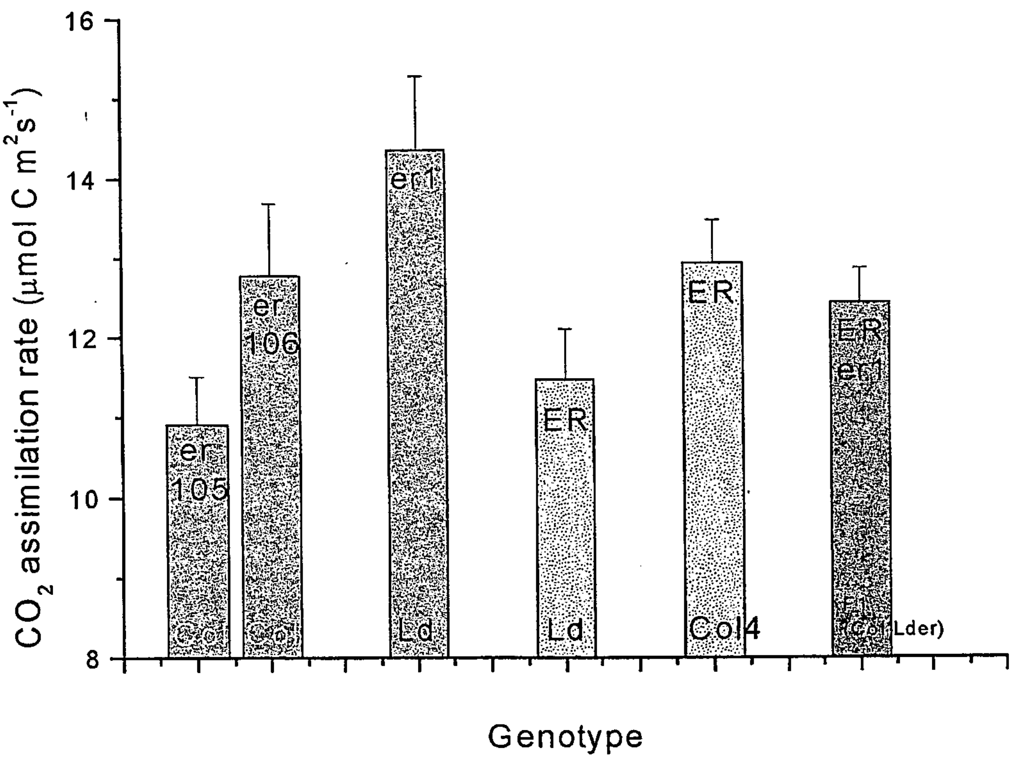
16
14
12
10
8
$CO_2$ assimilation rate (μmol C $m^2s^{-1}$)
er 105
Col
er 106
Col
er1
Ld
ER
Ld
ER
Col4
ER er1
F1 (Col Lder)
Genotype

Figure 1a

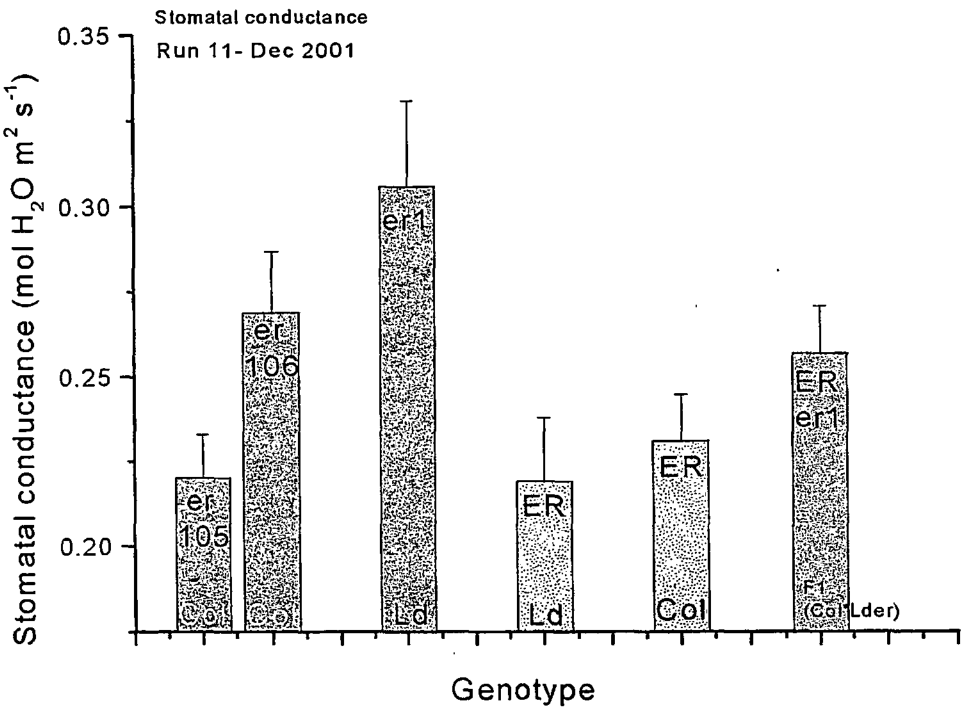
Stomatal conductance
Run 11- Dec 2001
Stomatal conductance (mol $H_2O$ $m^2$ $s^{-1}$)
0.35
0.30
0.25
0.20
er 105 Col
er 106 Col
er1 Ld
ER Ld
ER Col
ER er1 F1 (Col Lder)
Genotype

Figure 1b

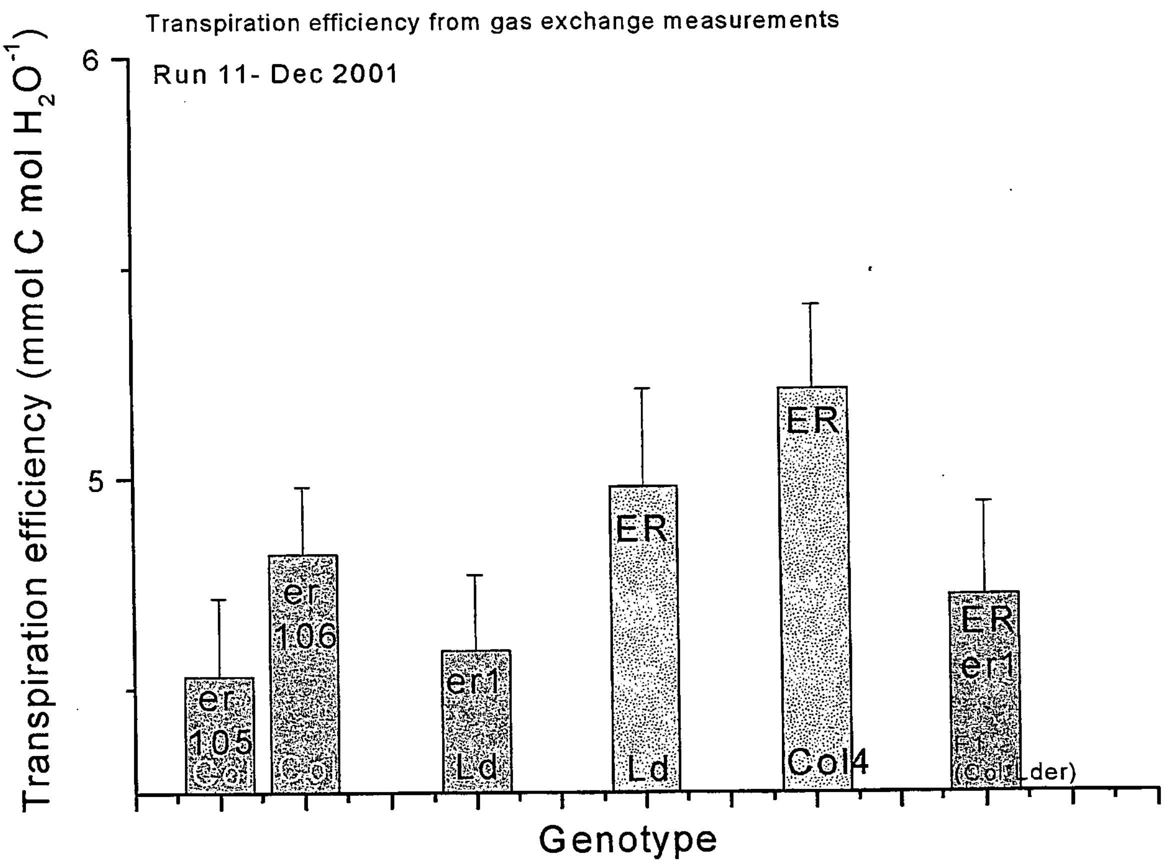
Transpiration efficiency from gas exchange measurements
Run 11- Dec 2001
Transpiration efficiency (mmol C mol H2O-1)
6
5
er 105 Col
er 106 Col
er1 Ld
ER Ld
ER Col4
ER er1 F1 (Col Lder)
Genotype

Figure 1c

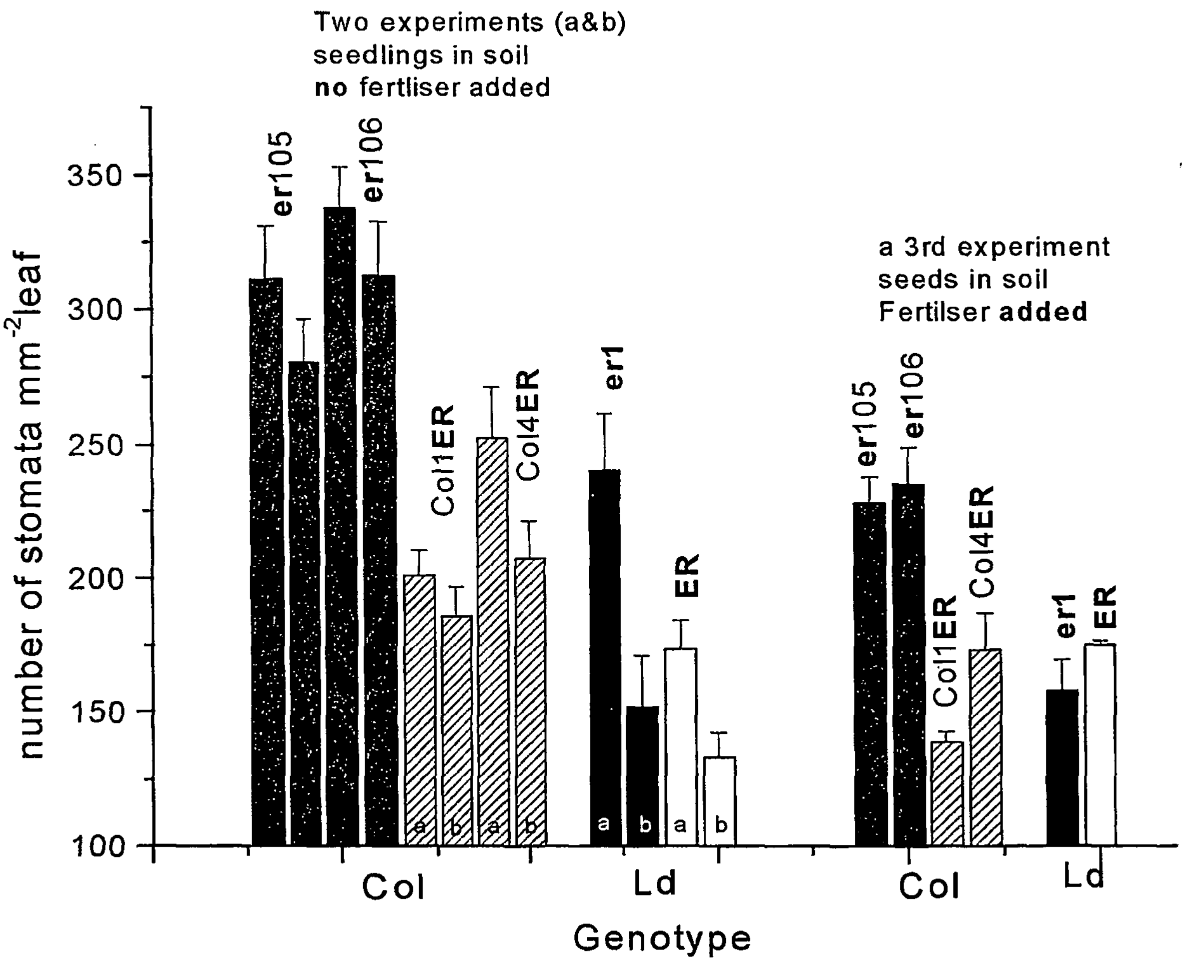
Two experiments (a&b)
seedlings in soil
no fertliser added
a 3rd experiment
seeds in soil
Fertilser added
number of stomata mm-2leaf
350
300
250
200
150
100
er105
er106
Col1ER
Col4ER
er1
ER
er105
er106
Col1ER
Col4ER
er1
ER
a b a b a b a b
Col
Ld
Col
Ld
Genotype

Figure 2a

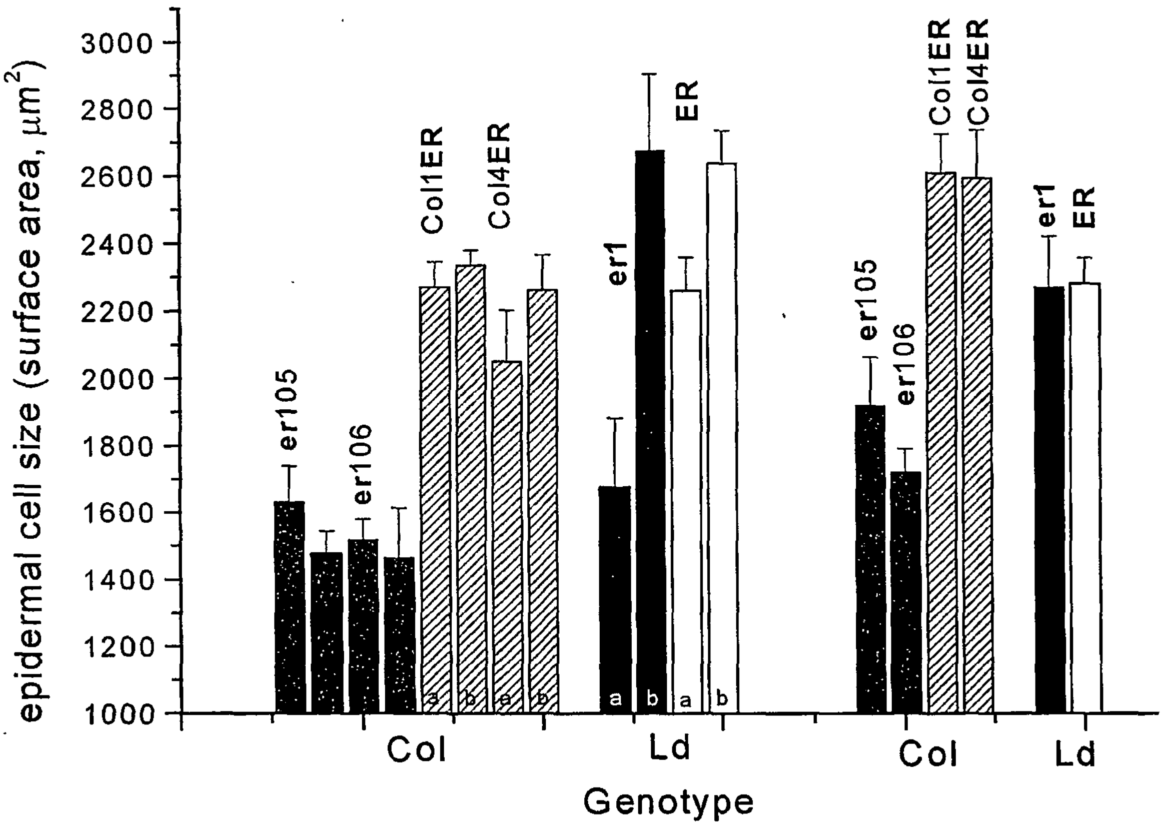
epidermal cell size (surface area, $\mu m^2$)
3000
2800
2600
2400
2200
2000
1800
1600
1400
1200
1000
er105
er106
Col1ER
Col4ER
er1
ER
er105
er106
Col1ER
Col4ER
er1
ER
a
b
a
b
a
b
a
b
Col
Ld
Col
Ld
Genotype

Figure 2b

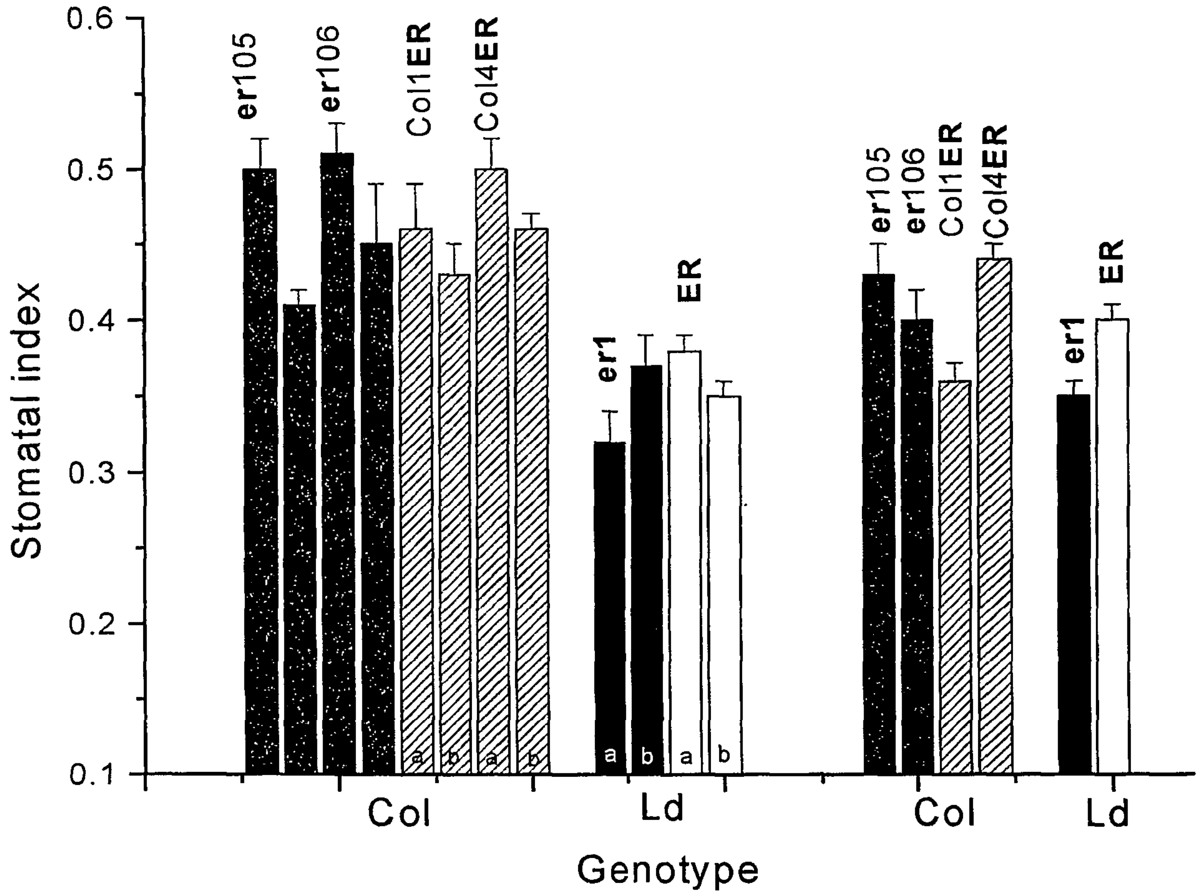
0.6
0.5
0.4
0.3
0.2
0.1
Stomatal index
er105
er106
Col1ER
Col4ER
a
b
a
b
er1
ER
a
b
a
b
Col
Ld
Genotype
er105
er106
Col1ER
Col4ER
Col
er1
ER
Ld

Figure 2c

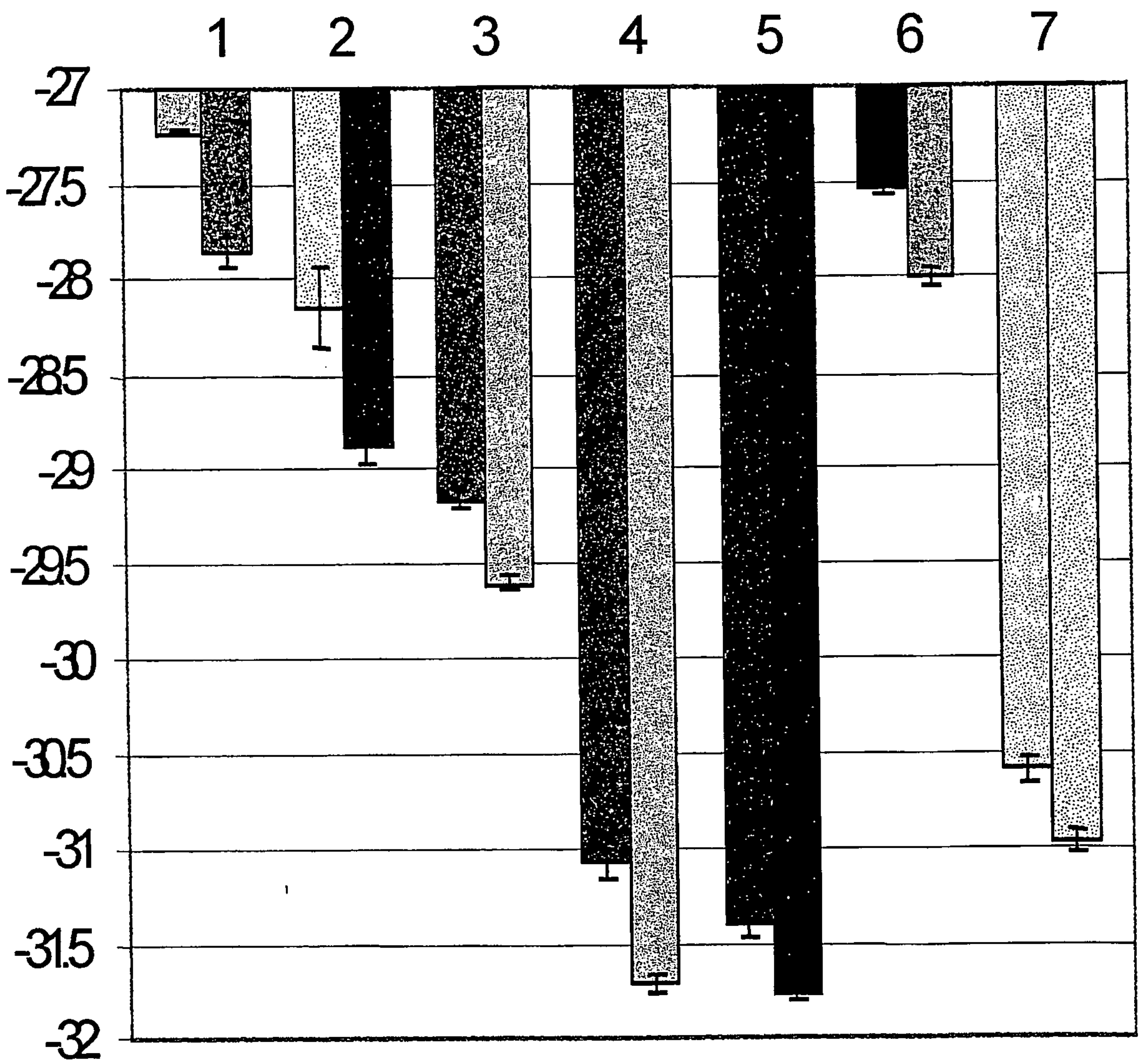


Figure 3

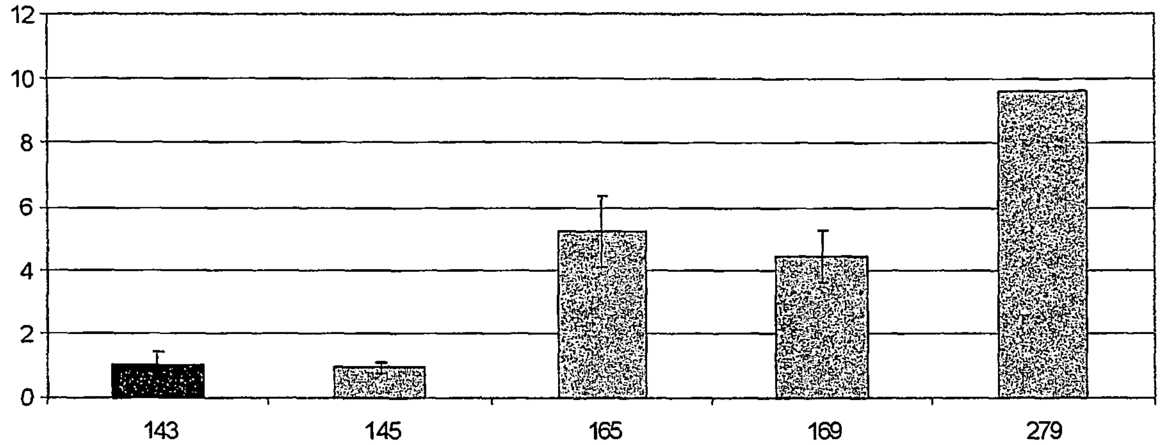


Figure 4a

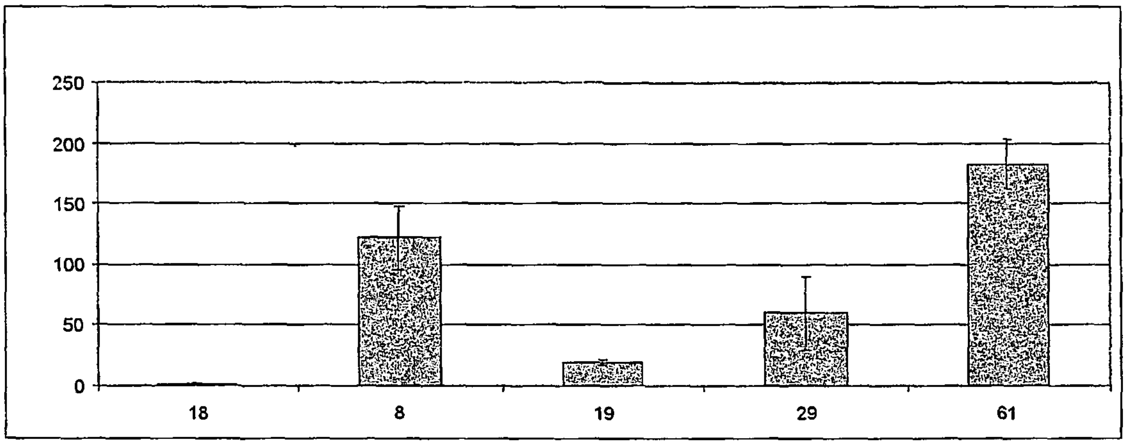


Figure 4b

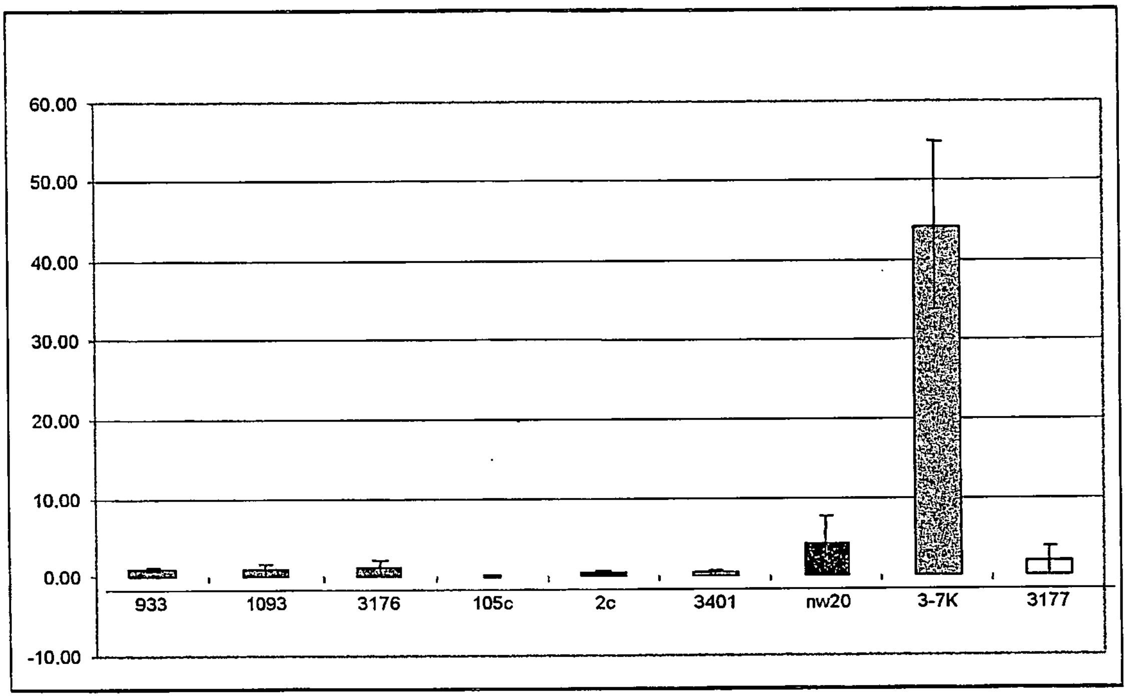
60.00
50.00
40.00
30.00
20.00
10.00
0.00
-10.00
933
1093
3176
105c
2c
3401
nw20
3-7K
3177

Figure 4c

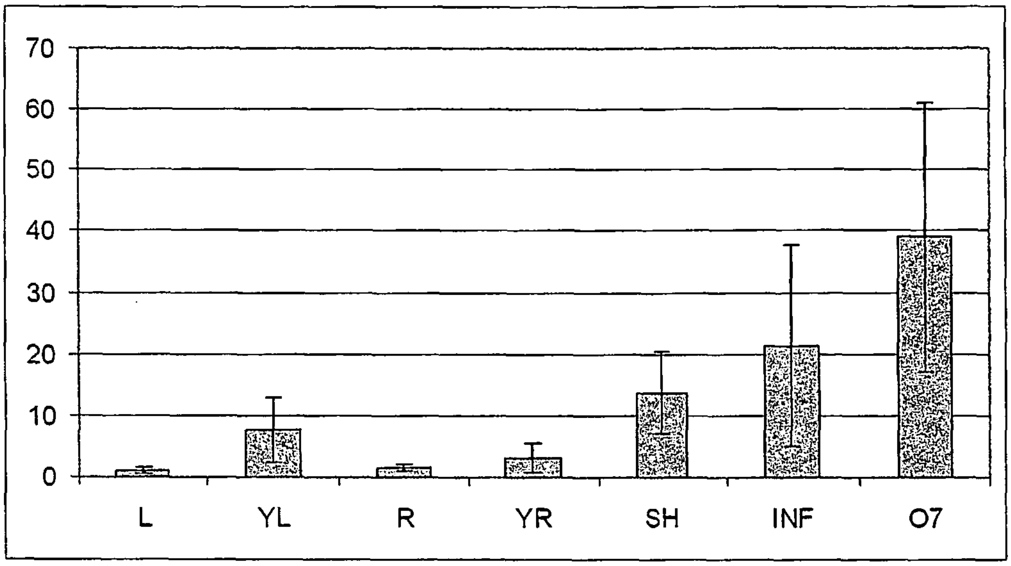


Figure 5a

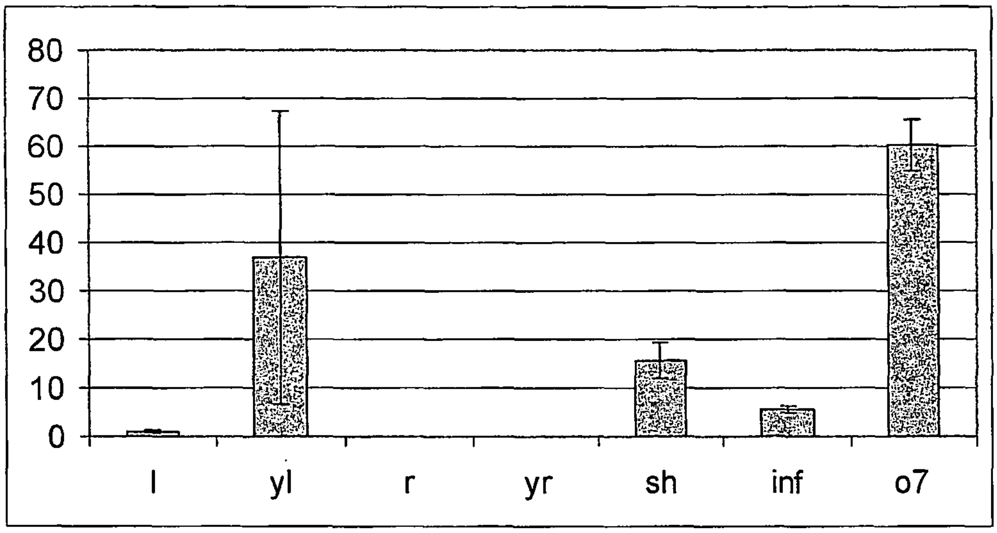


Figure 5b

Figure 6a: Transpiration efficiency at 350 and 500ppm $CO_2$

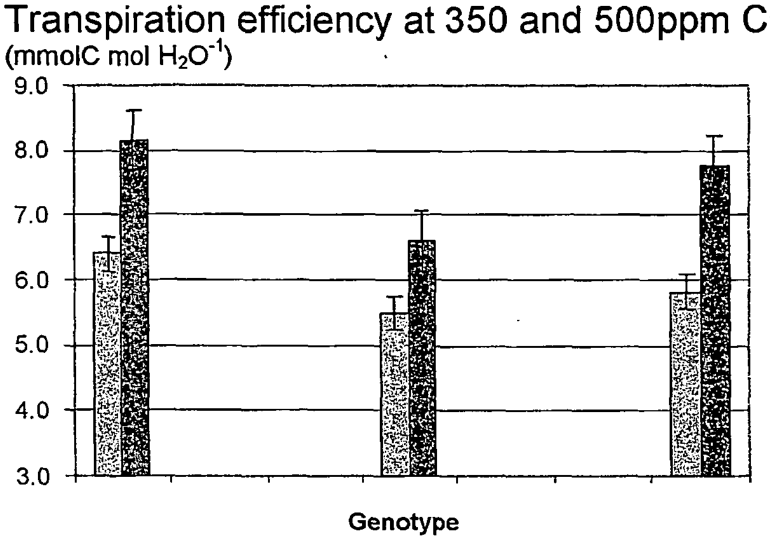


Figure 6b: $CO_2$ assimilation rate

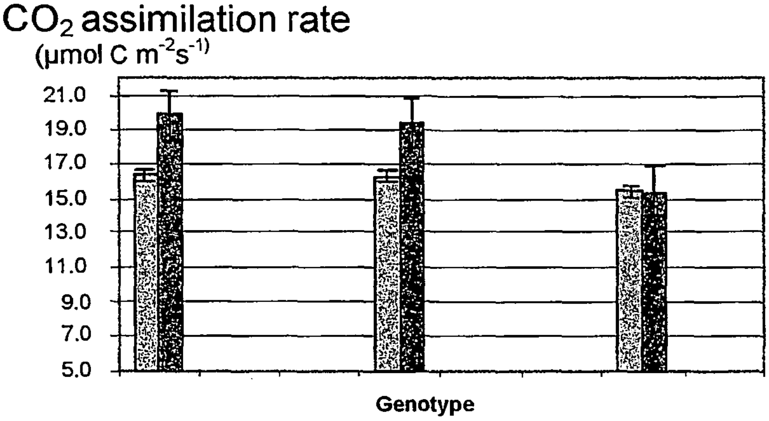


Figure 6c: Stomatal conductance

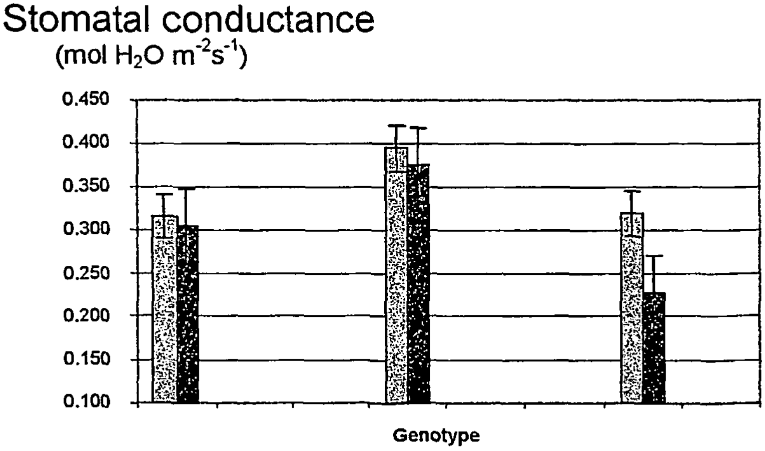


T2(+ER) Ld_ER

Ld -er1

Ld_ER (line 3177)

Figure 7a: **Transpiration efficiency (mmol C mol $H_2O^{-1}$) at 350 and 500ppm $CO_2$**

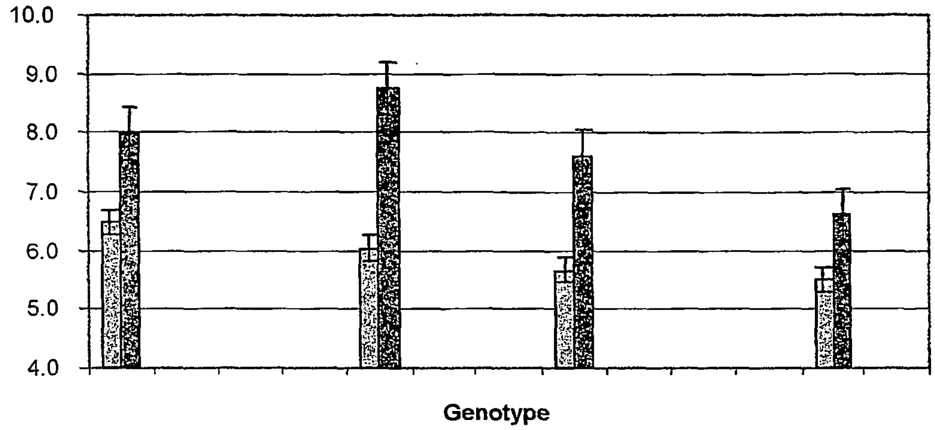


Figure 7b: **$CO_2$ assimilation rate** (µmol C $m^{-2}s^{-1}$)

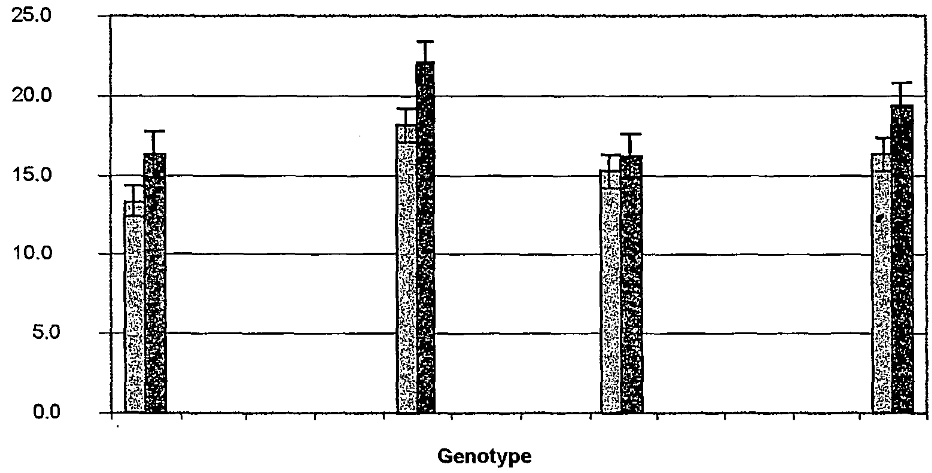


Figure 7c: **Stomatal conductance** (mol $H_2O$ $m^{-2}$ $s^{-1}$)

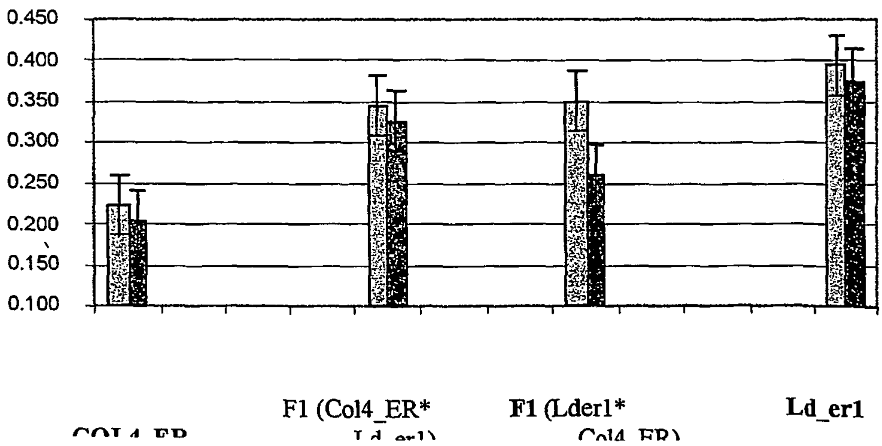

Figure 8a Stomatal conductance (mol $H_2O$ $m^{-2}$ $s^{-1}$)

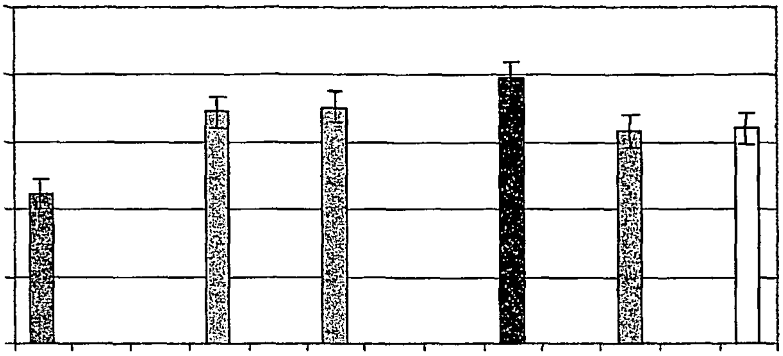

Figure 8b Epidermal cell area ($\mu m^2$)

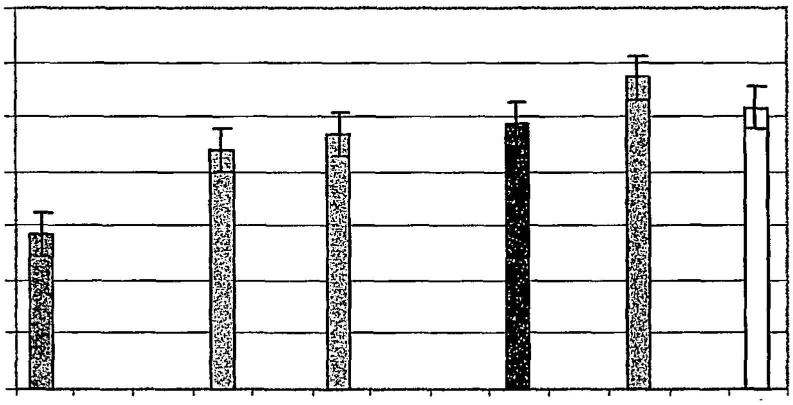

Figure 8c Number of stomata per $mm^2$

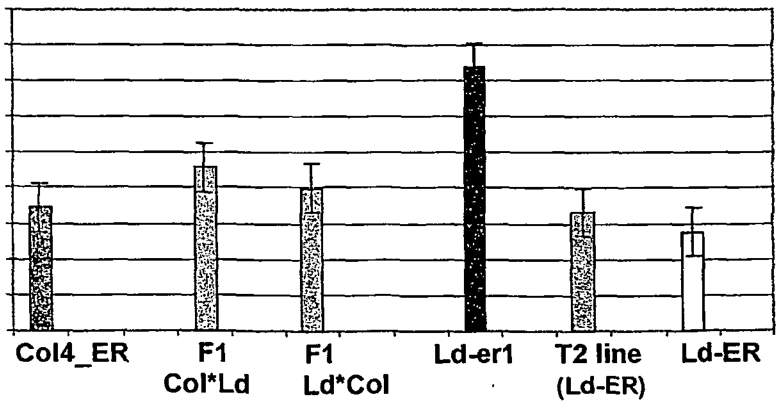

Figure 9a Number of stomata per $mm^2$

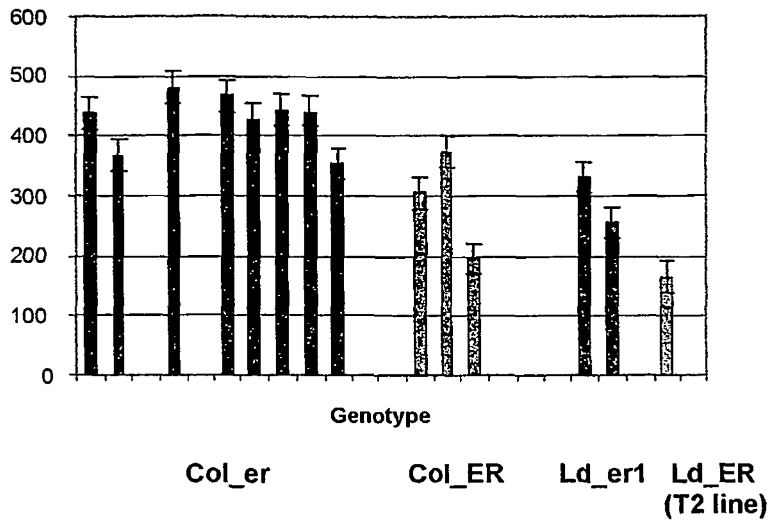


Figure 9b Epidermal cell area ($\mu m^2$)

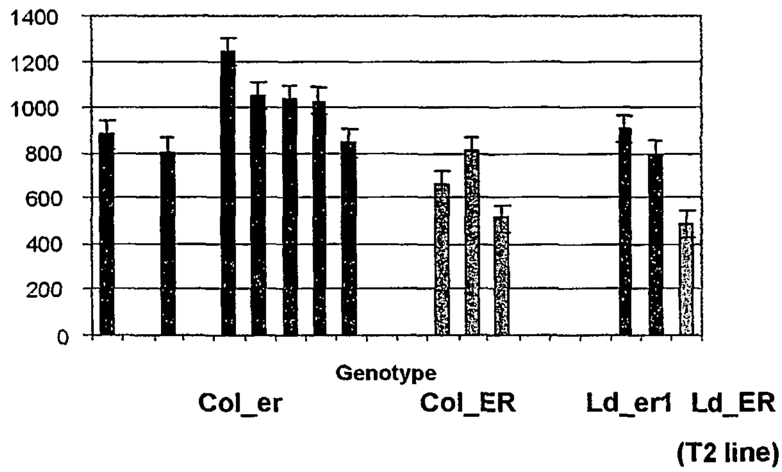

Carbon isotope composition (per mil) for a range of er mutants and ER ecotypes or transgenic lines

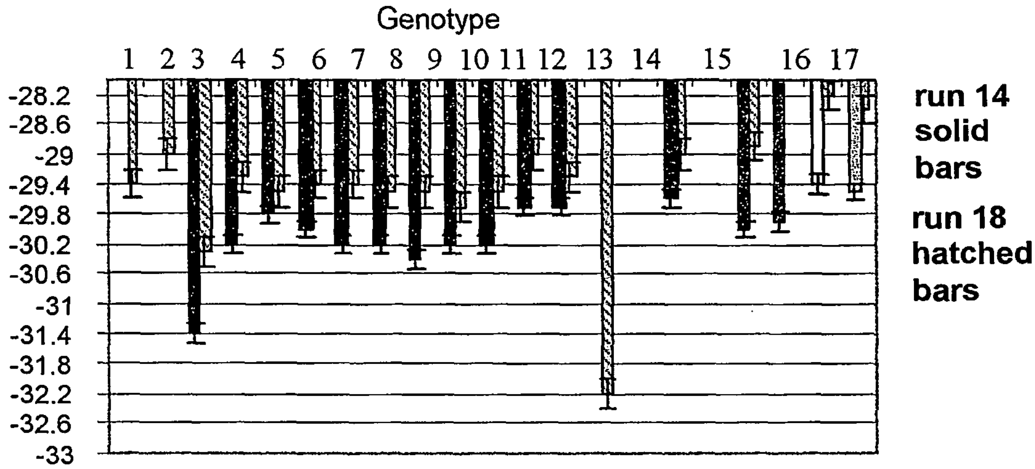


Figure 10

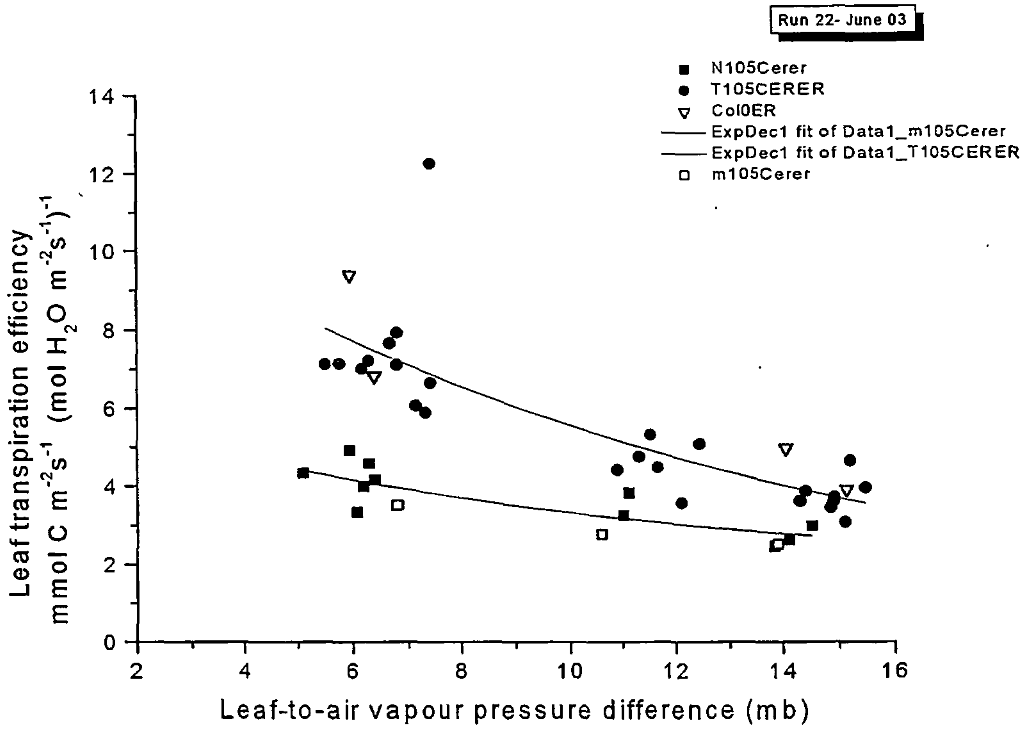
Run 22- June 03
N105Cerer
T105CERER
Col0ER
ExpDec1 fit of Data1_m105Cerer
ExpDec1 fit of Data1_T105CERER
m105Cerer
Leaf transpiration efficiency
mmol C m-2s-1 (mol H2O m-2s-1)-1
Leaf-to-air vapour pressure difference (mb)
0
2
4
6
8
10
12
14
16

Figure 11

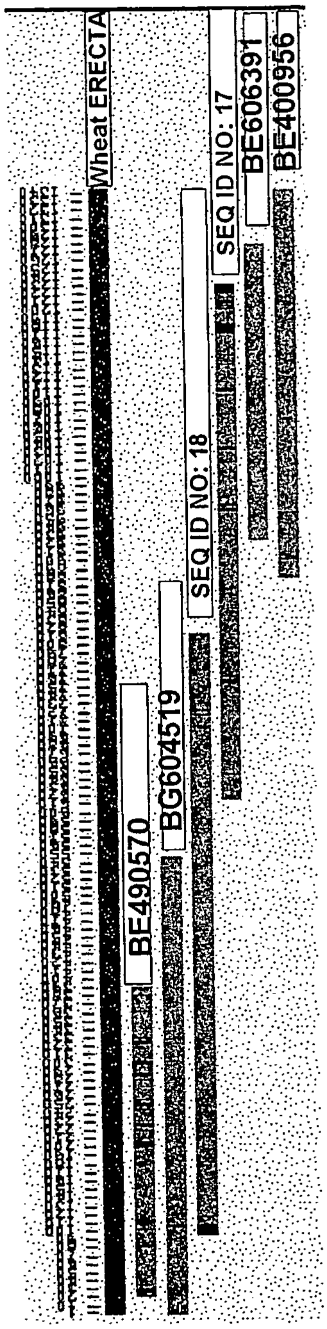
Wheat ERECTA
BE490570
BG604519
SEQ ID NO: 18
SEQ ID NO: 17
BE606391
BE400956

Figure 12

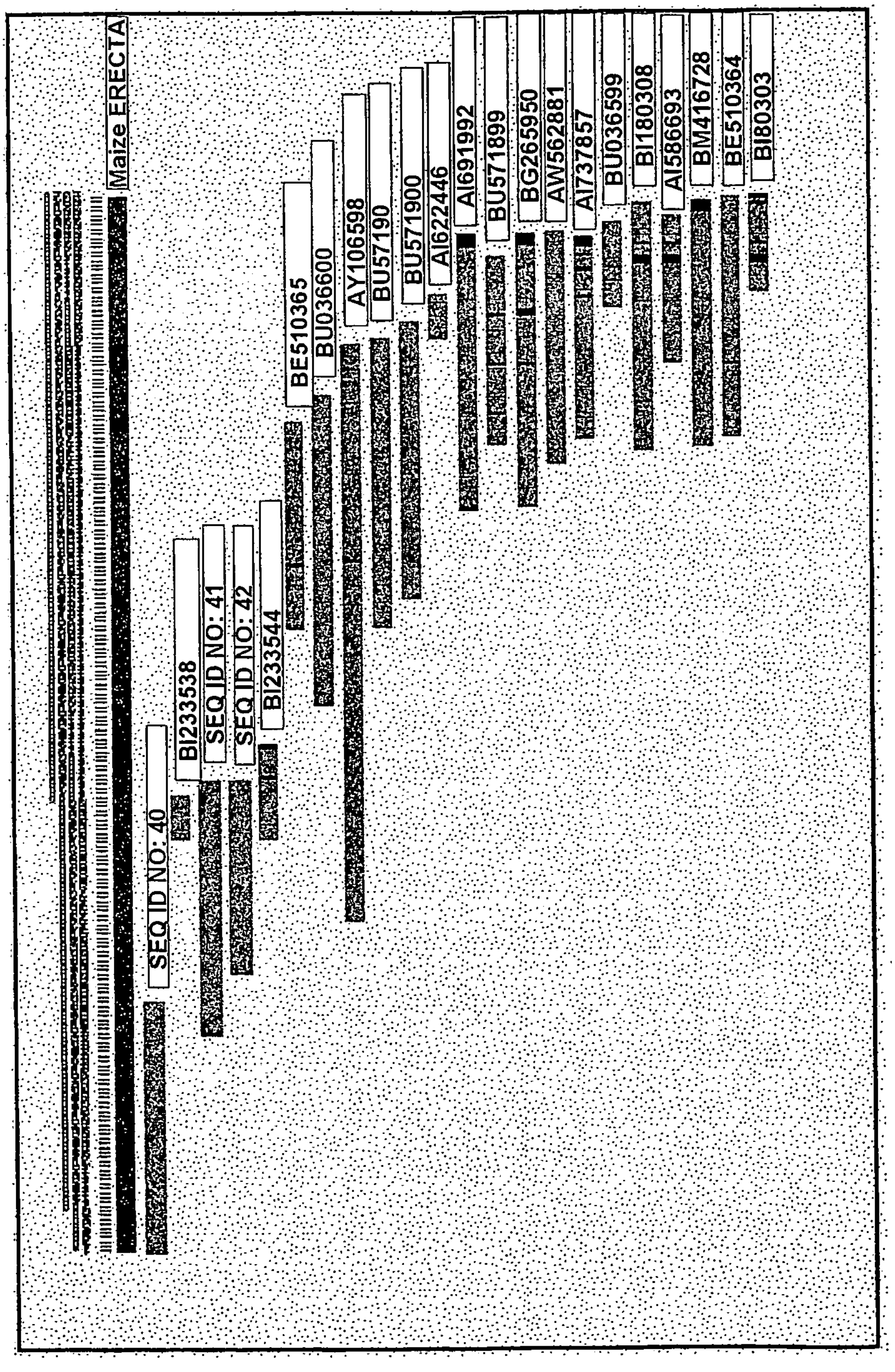
Maize ERECTA
SEQ ID NO: 40
BI233538
SEQ ID NO: 41
SEQ ID NO: 42
BI233544
BE510365
BU036600
AY106598
BU57190
BU571900
AI622446
AI691992
BU571899
BG265950
AW562881
AI737857
BU036599
BI180308
AI586693
BM416728
BE510364
BI80303

Figure 12

```
Erecta_sorghum        ----MTTTAARALVALLLVAVAVA---------DDGATLVEIKKSFRNVGNVLYDWAG--
Erecta_maize          ------------------------------------------------------------
Erecta_Arabidopsis    MALFRDIVLLGFLFCLSLVATVTS---------EEGATLLEIKKSFKDVNNVLYDWTTSP
Erecta_rice           MAAARAPWLWWVVVVVGVAVAEAASGGGGGGDGEGKALMGVKAGFGNAANALVDWDG--
Erecta_wheat          ------------------------------------------------------------

Erecta_sorghum        -DDYCSWRGVLCDNVTFAVAALNLSGLNLEGEISPAVGSLKSLVSIDLKSNGLSGQIPDE
Erecta_maize          ------------------------------------------------------------
Erecta_Arabidopsis    SSDYCVWRGVSCENVTFNVVALNLSDLNLDGEISPAIGDLKSLLSIDLRGNRLSGQIPDE
Erecta_rice           GADHCAWRGVTCDNASFAVLALNLSNLNLGGEISPAIGELKNLQFVDLKGNKLTGQIPDE
Erecta_wheat          ------------------------------------------------------------

Erecta_sorghum        IGDCSSLRTLDFSFNNLDGDIPFSISKLKHLENLILKNNQLIGAIPSTLSQLPNLKILDL
Erecta_maize          ------------------------------------------------------------
Erecta_Arabidopsis    IGDCSSLQNLDLSFNELSGDIPFSISKLKQLEQLILKNNQLIGPIPSTLSQIPNLKILDL
Erecta_rice           IGDCISLKYLDLSGNLLYGDIPFSISKLKQLEELILKNNQLTGPIPSTLSQIPNLKTLDL
Erecta_wheat          ------------------------------------------------------------

Erecta_sorghum        AQNKLTGEIPRLIYWNEVLQYL------------------------DVKNNSLTGVIPDT
Erecta_maize          ------------------------------------------------------------
Erecta_Arabidopsis    AQNKLSGEIPRLIYWNEVLQYLGLRGNNLVGNISPDLCQLTGLWYFDVRNNSLTGSIPET
Erecta_rice           AQNQLTGDIPRLIYWNEVLQYLGLRGNSLTGTLSPDMCQLTGLWYFDVRGNNLTGTIPES
Erecta_wheat          ------------------------------------------------------------

Erecta_sorghum        IGNCTSFQVLDLSYNRFTGPIPFNIGFLQVATLSLQGNKFTGPIPSVIGLMQALAVLDLS
Erecta_maize          ------------------------------------------------------------
Erecta_Arabidopsis    IGNCTAFQVLDLSYNQLTGEIPFDIGFLQVATLSLQGNQLSGKIPSVIGLMQALAVLDLS
Erecta_rice           IGNCTSFEILDISYNQISGEIPYNIGFLQVATLSLQGNRLTGKIPDVIGLMQALAVLDLS
Erecta_wheat          ------------------------------------------------------------

Erecta_sorghum        YNQLSGPIPSILGNLTYTEKLYIQGNKLTGSIPPELGNMSTLHYLELNDNQLTGSIPPEL
Erecta_maize          ----------------------MQVNRLTGSIPPELGNMSTLHYLELNDNQLTGSIPPEL
Erecta_Arabidopsis    GNLLSGSIPPILGNLTFTEKLYLHSNKLTGSIPPELGNMSKLHYLELNDNHLTGHIPPEL
Erecta_rice           ENELVGPIPSILGNLSYTGKLYLHGNKLTGVIPPELGNMSKLSYLQLNDNELVGTIPAEL
Erecta_wheat          ------------------------------------------------------------

Erecta_sorghum        GRLTGLFDLNLANNHLEGPIPDNLSSCVNLNSFNAYGNKLNGTIPRSLRKLESMTYLNLS
Erecta_maize          GRLTGLFDLNLANNHLEGPIPDNLSSCVNLNSFNAYGNKLNGTIPRSLRKLESMTYLNLS
Erecta_Arabidopsis    GKLTDLFDLNVANNDLEGPIPDHLSSCTNLNSLNVHGNKFSGTIPRAFQKLESMTYLNLS
Erecta_rice           GKLEELFELNLANNNLQGPIPANISSCTALNKFNVYGNKLNGSIPAGFQKLESLTYLNLS
Erecta_wheat          --------------------------------FNVYGNRLNGSIPAGFQNLESLTNLNLS
                                                      :*.:**::.*:** .:::***:* ****

Erecta_sorghum        SNFISGSIPIELSRINNLDTLDLSCNMMTGPIPSSIGSLEHLLRLNLSKNGLVGFIPAEF
Erecta_maize          SNFISGSIPIELSRINNLDTLDLSCNMMTGPIPSSIGNLEHLLRLNLSKNDLVGFIPAEF
Erecta_Arabidopsis    SNNIKGPIPVELSRIGNLDTLDLSNNKINGIIPSSLGDLEHLLKMNLSRNHITGVVPGDF
Erecta_rice           SNNFKGNIPSELGHIINLDTLDLSYNEFSGPVPATIGDLEHLLELNLSKNHLDGPVPAEF
Erecta_wheat          SNNFKGHIPSELGHIINLDTLDLSYNELSGPVPATIGDLEHLLQLNLSKNHLSGSVPAEF
                      ** :.* ** **.:* ******** * :.* :*:::*.*****.:***:* : * :*.:*

Erecta_sorghum        GNLRSVMEIDLSYNHLGGLIPQELEMLQNLMLL---------------------------
Erecta_maize          GNLRSVMEIDLSYNHLGGLIPQELGMLQNLMLLKLENNNITGDV-SSLMNCFSLN---IL
Erecta_Arabidopsis    GNLRSIMEIDLSNNDISGPIPEELNQLQNIILLRLENNNLTGNV-GSLANCLSLT---VL
Erecta_rice           GNLRSVQVIDMSNNNLSGSLPEELGQLQNLDSLILNNNNLVGEIPAQLANCFSLNNLAFQ
Erecta_wheat          GNLRSIQVIDLSNNAMSGYLPEELGQLQNLDSLILNNNILVGEIPAQLANCFSLN---IL
                      *****:  **:* * :.* :*:** ***:  *

Erecta_sorghum        NVSYNNLAGVVPADNNFTRFSPD----------------SFLGNPGLCGYWLGSSCRSTG
Erecta_maize          NVSYNNLAGAVPTDNNFTRFSHD----------------SFLGNPGLCGYWLGSSCRSTG
Erecta_Arabidopsis    NVSHNNLVGDIPKNNNFSRFSPD----------------SFIGNPGLCGSWLNSPCHDSR
Erecta_rice           EFVIQQFIWTCPDGKELLEIPNGKHLLISDCNQYINHKCSFLGNPLLHVYCQDSSCGHS-
Erecta_wheat          NLSHNNFSGHVPFAKNFSKFPGE----------------SFLGNPMLSVHCKDSSCGNS-
                      :.  :::    *  ::: .:.                  **:*** *     .*.*  :

Erecta_sorghum        HHEKPPISKAAIIGVAVGGLVILLMILVAVCRPHRPPAFKDVTVSKPVRNAPPKLVILHM
Erecta_maize          HRDKPPISKAAIIGVAVGGLVILLMILVAVCRPHHPPAFKDATVSKPVSNGPPKLVILHM
Erecta_Arabidopsis    RTVRVSISRAAILGIAIGGLVILLMVLIAACRPHNPPPFLDGSLDKPVTYSTPKLVILHM
Erecta_rice           HGQRVNISKTAIACIILGFIILLCVLLLAIYKTNQPQPLVKGS-DKPVQ-GPPKLVVLQM
Erecta_wheat          HGSKVNT-RTAIACIISGFVILLCVLLLAIYKTKRPQPPIKAS-DKPGQ-GPPKIVLLQM
                      :  :    ::** :  :  * :::* ::*:*  :.:.* .  . : .**   ..**:*:*:*

Erecta_sorghum        NMALHVYDDIMRMTENLSEKYIIGYGASSTVYKCVLKNCKPVAIKKLYAHYPQSLKEFET
Erecta_maize          NMALHVFDDIMRMTENLSEKYIIGYGASSTVYKCVLKNCKPVAIKKLYAHYLQSLKEFET
Erecta_Arabidopsis    NMALHVYEDIMRMTENLSEKYIIGHGASSTVYKCVLKNCKPVAIKRLYSHNPQSMKQFET
Erecta_rice           DMAIHTYEDIMRLTENLSEKYIIGYGASSTVYKCELKSGKAIAVKRLYSQYNHSLREFET
Erecta_wheat          DMAIHTYDDIMRLTENLSEKYIIGYGASSTVYKCVLKSGKAIAVKRK--QYNHGAREFET
                      :**:*.::****:*********** *********:********* **. *.:*:*:   :  :. ::***

Erecta_sorghum        ELETVGSIKHRNLVSLQGYSLSPVGNLLFYDYMECGSLWDVLHEGSSKKKKLDWETRLRI
Erecta_maize          ELETVGSIKHRNLVSLQGYSLSPVGNLLFYAYMESGSLWDVLHEGSSKKNKLDWVTRLRI
Erecta_Arabidopsis    ELEMLSSIKHRNLVSLQAYSLSHLGSLLFYDYLENGSLWDLLH-GPTKKKTLDWDTRLKI
Erecta_rice           ELETIGSIRHRNLVSLHGFSLSPHGNLLFYDYMENGSLWDLLH-GPSKKVKLNWDTRLRI
Erecta_wheat          ELETVGSIRHRNLVSLHGFSLSPNGNLLFYDYMENGSLWDLLH-GPSKKVKLDWDTRLKI
                      *** :.**:*******:.:***  *.**** *:* ****:**  *.:** .*:* ***:*

Erecta_sorghum        ALGAAQGLAYLHHDCSPRIIHRDVKSKNILLDKDYEAHLTDFGIAKSLCVSKTHTSTYVM
Erecta_maize          ALGAAQGLAYLHHDCSPRIIHRDVKSKNILLDKDYEAHLTDFGIAKSLCVSKTHTSTYVM
Erecta_Arabidopsis    AYGAAQGLAYLHHDCSPRIIHRDVKSSNILLDKDLEARLTDFGIAKSLCVSKSHTSTYVM
Erecta_rice           AVGAAQGLAYLHHDCNPRIIHRDVKSSNILLDENFEAHLSDFGIAKCVPSAKSHASTYVL
Erecta_wheat          AVGAAQGLAYLHHDCNPRIVHRDVKSSNILLDEHFEAHLSDFGIAKCVPAAKTHASTYVL
                      * ************.***:******.*****:. **:*:*******.:  :*:*:****:

Erecta_sorghum        GTIGYIDPEYARTSRLNEKSDVYR----LWHCSAG--AADWQEASGQRILSKTASNEVMD
Erecta_maize          GTIGYIDPEYARTSRLNEKSDVYSYGIVLLELLTGKKPVDNECNLHHLILSKTASNEVME
Erecta_Arabidopsis    GTIGYIDPEYARTSRLTEKSDVYSYGIVLLELLTRRKAVDDESNLHHLIMSKTGNNEVME
Erecta_rice           GTIGYIDPEYARTSRLNEKSDVYSFGIVLLELLTGKKAVDNESNLHQLILSKADDNTVME
Erecta_wheat          GTIGYIDPEYARTSQLNEKSDVYS------------------------------------
                      **************:*.******

Erecta_sorghum        TVDPDIGDTCKDLGEVKKLFQLALLCTKRQPSDRPTMHEVVRVLDCLVNPDPPPKP----
Erecta_maize          TVDPDVGDTCKDLGEVKKLFQLALLCTKRQPSDRPTMHEVVRVLDCLVNPEPPPQPQQQQ
Erecta_Arabidopsis    MADPDITSTCKDLGVVKKVFQLALLCTKRQPNDRPTMHQVTRVLGSFMLSEQPPAAT---
Erecta_rice           AVDSEVSVTCTDMGLVRKAFQLALLCTKRHPSDRPTMHEVARVLLSLLPASAMTTP----
Erecta_wheat          ------------------------------------------------------------

Erecta_sorghum        -----SAHQLPQPSPAVPSYINEYVSLRGTGALSCANSTSTSDAELFLKFGEAISQNME
Erecta_maize          QKAHAHHQLP-PQPSPPAYVDEYVSLRGTGALSCANSSSTSDAELFLKFGEAISQNMV
Erecta_Arabidopsis    -------DTS-ATLAGSCYVDEYANLKTPHSVNCS-SMSASDAQLFLRFGQVISQNSE
Erecta_rice           -------KTV-DYSRLLASTTTAADMRGHDVTDIG-DNSSSDEQWFVRFGEVISKHTM
Erecta_wheat          ------------------------------------------------------------
```

Figure 13

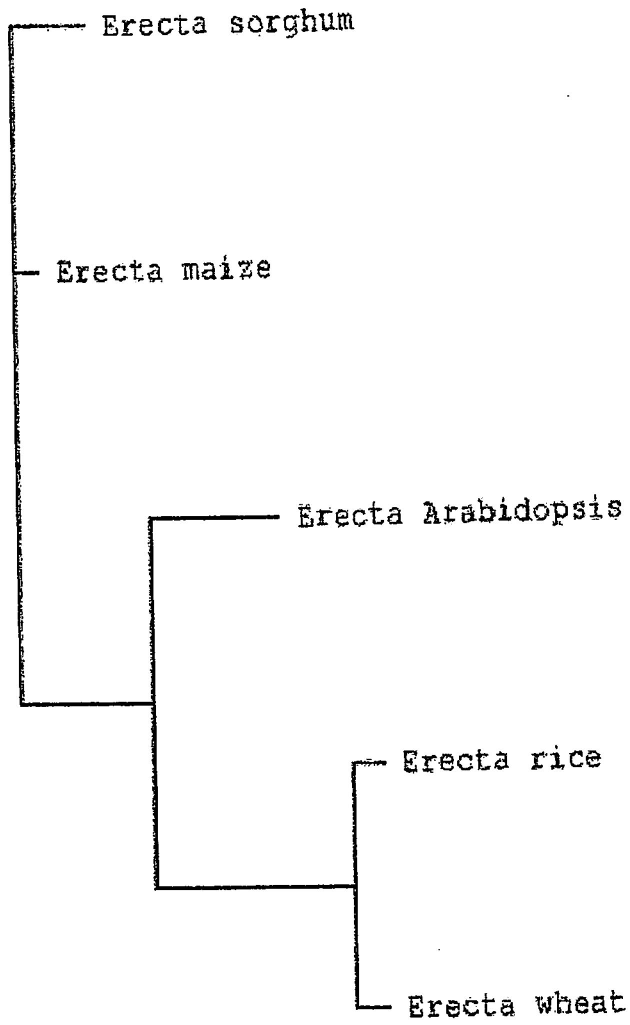
Erecta sorghum
Erecta maize
Erecta Arabidopsis
Erecta rice
Erecta wheat

FIGURE 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4645682 A
- US 3791839 A, Cushman
- US 3847641 A
- US 3826671 A, Petrucco
- US 4671816 A
- SU 1282492
- SU 1253559
- SU 1098934
- US 5589437 A
- US 4943315 A, Schulz
- JP 3184966 A
- US 2002040489 A
- WO 0102541 A
- US 5122466 A, Stomp
- US 4945050 A, Sanford and Wolf
- EP 672752 A
- AU 738153
- EP 856060 A1
- CA 2230216
- US 5177010 A
- US 5981840 A
- US 20020002711 A1
- AU PS3339


### Non-patent literature cited in the description


- *Helv. Chim. Acta,* 1988, vol. 71, 931
- **Kozlowski.** Tree Growth and Environmental Stresses (Univ. Washington Press, 1979
- Stable isotopes and plant carbon-water relations. **Masle et al.** Physiol. Ser. Acad. Press, 1993, 371-386
- **Hall et al.** *Plant Breeding Reviews,* 1994, vol. 4, 81-113
- **Farquhar et al.** Breaking the Yield Barrier. IRRI, vol. 95, 101
- **Rebetzke et al.** *Crop Science,* 2002, vol. 42, 739-745
- **Torii et al.** *The Plant Cell,* 1996, vol. 8, 735
- **Thumma et al.** *Journal of Experimental Botany,* 2001, vol. 52 (355), 203-214
- **Torii et al.** *The Plant Cell,* 1996, vol. 8, 735-746
- **Edwards et al.** *Genetics,* 1987, vol. 116, 113-125
- **Paterson et al.** *Nature,* 1988, vol. 335, 721-726
- **Masle et al.** *op. cit,* 1993
- **Lease et al.** *New Phytologist,* 2001, vol. 151, 133-143
- **Torii et al.** *The Plant Cell,* vol. 8 (73), 5-746
- **Devereaux et al.** *Nucl. Acids Res.,* 1984, vol. 12, 387-395
- **Needleman ; Wunsch.** *J. Mol. Biol.,* 1970, vol. 48, 443-453
- **Thompson et al.** *Nucl. Acids Res.,* 1994, vol. 22, 4673-4680
- **Ausubel et al.** Current Protocols in Molecular Biology. Greene/Wiley, 1992
- **Edwards et al.** *Genetics,* 1987, vol. 116, 113-125
- **Haley ; Knott.** *Heredity,* 1992, vol. 69, 315-324
- **Jiang ; Zeng.** *Genetics,* 1995, vol. 140, 1111-1127
- **Lander ; Botstein.** *Genetics,* 1989, vol. 121, 185-199
- **Jansen ; Stam.** *Genetics,* 1994, vol. 136, 1447-1455
- Biometrics in Plant Breeding: Applications of Molecular Markers. **Utz ; Melchinger.** Proc. Ninth Meeting of the EUCARPIA Section Biometrics in Plant Breeding. The Netherlands, 06 July 1994
- **Zeng.** *Genetics,* 1994, vol. 136, 1457-1468
- **Michelmoore et al.** *Proc. Natl Acad. Sci. (USA),* 1991, vol. 88, 9828-9832
- **Lister ; Dean.** *Plant J.,* 1993, vol. 4, 745-750
- **Lincoln et al.** Constructing genetic linkage maps with MAPMAKER/EXP version 3.0: A tutorial and reference manual. Whitehead Institute for Biomedical Research, 1993
- **Utz ; Melchinger.** *GENETICS,* 1994
- **Utz ; Melchinger.** PLABlocus Version 1.0. A computer program to map QTL, Institut für Pflanzenzüchtung. *Saatgutforschung und Populationsgenetik,* 1995, http://www.uni-hohenheim.de/∼ipspwww/soft.html
- **Carlborg et al.** *Genetics,* 2000
- **Grandillo et al.** *Theor. Appl. Genet.,* 1999, vol. 99, 978-987

- **Farquhar et al.** *Aust. J. Plant Physiol.,* 1982, vol. 9, 121-137
- **Farquhar ; Richards.** *Aust. J. Plant Physiol.,* 1984, vol. 11, 539-552
- **Farquhar et al.** *Ann. Rev. Plant Physiol.,* 1989, vol. 40, 388-397
- **Thumma et al.** *Proc. 9th Aust. Agronomy Conf.,* 1998
- **Farquhar.** *Australian Journal of Plant Physiology,* 1983, vol. 10, 205-226
- **Henderson et al.** *Aust. J. Plant Physiol.,* 1992, vol. 19, 263-285
- **Virgona et al.** *Aust. J. Plant Physiol.,* 1994, vol. 17, 207-214
- **Wright et al.** *Crop Sci,* vol. 34, 92-97
- **McPherson et al.** PCR A Practical Approach. IRL Press, Oxford University Press, 1991
- **Krens et al.** *Nature,* 1982, vol. 296, 72-74
- **Paszkowsk et al.** *EMBO J.,* 1984, vol. 3, 2717-2722
- **Armstrong et al.** *Plant Cell Rep.,* 1990, vol. 9, 335-339
- **Fromm et al.** *Proc. Natl. Acad. Sci. (USA),* 1985, vol. 82, 5824-5828
- **Crossway et al.** *Mol. Gen. Genet.,* 1986, vol. 202, 179-185
- **Christou et al.** *Plant Physiol.,* 1988, vol. 87, 671-674
- **Sanford.** *Part. Sci. Technol.,* 1988, vol. 5, 27-37
- **An et al.** *EMBO J.,* 1985, vol. 4, 277-284
- **Herrera-Estella et al.** *Nature,* 1983, vol. 303, 209-213
- **Herrera-Estella et al.** *EMBO J.,* 1983, vol. 2, 987-995
- **Herrera-Estella et al.** Plant Genetic Engineering. Cambridge University Press, 1985, 63-93
- **Bechtold et al.** *CR Acad. Sci. (Paris, Sciences de la vie/ Life Sciences),* 1993, vol. 316, 1194-1199
- **Clough et al.** *Plant J,* 1998, vol. 16, 735-74
- **McElroy et al.** *Mol and Gen Genetics,* 1991, vol. 231 (1), 150-160
- **Kasuga et al.** *Nature Biotechnology,* 1999, vol. 17, 287-291
- **Kay et al.** *Science,* 1987, vol. 236, 4805
- **Vidal ; Legrain.** *Nuc/. Acid Res,* 1999, vol. 27 (4), 919-929
- **Sambrook et al.** Molecular Cloning: A laboratory Manual. Cold Spring Harbour, 1989
- **Scopes.** Protein Purification: Principles and Practice. Springer Verlag, 1994
- **J. Finer.** *Plant Cell Report,* 1992, vol. 11, 323-328
- **Armstrong.** Maize Handbook. 1994, 665-671
- **Klein et al.** *Nature,* 1987, vol. 327, 70-73
- **Finer et al.** *Plant Cell Report,* 1992, vol. 11, 323-328
- **Vain.** *Plant Cell tissue and organ Culture,* 1989, vol. 18, 143-151
- *Methods of Molecular Biology: Plant gene transfer and expression protocols,* 1995, vol. 49, 113-123
- **Barcelo ; Lazzeri.** *Methods of Molecular Biology,* 1995, vol. 49, 113-123
- **Ishida.** *Nature Biotechnology,* 1996, vol. 14, 754-750
- **Scopes.** Protein purification: principles and practice. Springer Verlag, 1994
- **Hajdukiewics et al.** *Plant Mol Biol,* 1994, vol. 25, 989-994
- **Ditta et al.** *PNAS,* 1980, vol. 77, 7347-7351
- **Clough ; Bent.** *The Plant Journal,* 1998, vol. 16, 735-743
- **Hirochika.** *Current Opinion in Plant Biology,* 2001, vol. 4, 118-122
- **Hajdukiewicz et al.** *Plant Mol Biol,* 1994, vol. 25, 989-994
- **Ueda et al.** *Protoplasma,* 1999, vol. 206, 201-206
- **Olszewski et al.** *Nucl. Acid Res,* 1988, vol. 16, 10765-10782
- **Hellens et al.** *Plant Mol Biol,* 2000, vol. 42, 819-832
- **Ditta et al.** *Proc. Natl Acad. Sci.,* 1980, vol. 77, 7347-7351
- **Wang et al.** *J Gen and Breed,* 1997, vol. 51, 325-334
- **Pellegrineschi et al.** *Genome,* 2002, vol. 45 (2), 421-30
- **Gordon-Kamm et al.** *Plant Cell,* 1990, vol. 2 (7), 603-618

SEQUENCE LISTING

<110> The Australian National University

<120> METHOD OF PRODUCING PLANTS HAVING ENHANCED TRANSPIRATION EFFICIENCY AND PLANTS PRODUCED THEREFROM

<130> 94948/MRO

<150> AU PS3339
<151> 2002-07-02

<160> 45

<170> PatentIn version 3.1

<210> 1
<211> 3176
<212> DNA
<213> Arabidopsis thaliana ERECTA allele

<400> 1

```
gtttcttctt catggagact tgaaagcttt taaagtatat ctaaaaacgc agtcgtttta      60
agactgtgtg tgagaaatgg ctctgtttag agatattgtt cttcttgggt ttctcttctg     120
cttgagctta gtagctactg tgacttcaga ggagggagca acgttgctgg agattaagaa     180
gtcattcaaa gatgtgaaca atgttcttta tgactggaca acttcacctt cttcggatta     240
ttgtgtctgg agaggtgtgt cttgtgaaaa tgtcaccttc aatgttgttg ctcttaattt     300
gtcagatttg aatcttgatg gagaaatctc acctgctatt ggagatctca agagtctctt     360
gtcaattgat ctgcgaggta atcgcttgtc tggacaaatc cctgatgaga ttggtgactg     420
ttcttctttg caaaacttag acttatcctt caatgaatta agtggtgaca taccgttttc     480
gatttcgaag ttgaagcaac ttgagcagct gattctgaag aataaccaat tgataggacc     540
gatcccttca acactttcac agattccaaa cctgaaaatt ctggacttgg cacagaataa     600
actcagtggt gagataccaa gacttattta ctggaatgaa gttcttcagt atcttgggtt     660
```

gcgaggaaac aacttagtcg gtaacatttc tccagatttg tgtcaactga ctggtctttg 720
gtattttgac gtaagaaaca acagtttgac tggtagtata cctgagacga taggaaattg 780
cactgccttc caggttttgg acttgtccta caatcagcta actggtgaga tccctttga 840
catcggcttc ctgcaagttg caacattatc attgcaaggc aatcaactct ctgggaagat 900
tccatcagtg attggtctca tgcaagccct tgcagtctta gatctaagtg gcaacttgtt 960
gagtggatct attcctccga ttctcggaaa tcttactttc accgagaaat tgtatttgca 1020
cagtaacaag ctgactggtt caattccacc tgagcttgga aacatgtcaa aactccatta 1080
cctggaactc aatgataatc atctcacggg tcatatacca ccagagcttg ggaagcttac 1140
tgacttgttt gatctgaatg tggccaacaa tgatctggaa ggacctatac ctgatcatct 1200
gagctcttgc acaaatctaa acagcttaaa tgttcatggg aacaagttta gtggcactat 1260
accccgagca tttcaaaagc tagaaagtat gacttacctt aatctgtcca gcaacaatat 1320
caaaggtcca atcccggttg agctatctcg tatcggtaac ttagatacat tggatctttc 1380
caacaacaag ataaatggaa tcattccttc ttcccttggt gatttggagc atcttctcaa 1440
gatgaacttg agtagaaatc atataactgg tgtagttcca ggcgactttg gaaatctaag 1500
aagcatcatg gaaatagatc tttcaaataa tgatatctct ggcccaattc cagaagagct 1560
taaccaatta cagaacataa ttttgctgag actggaaaat aataacctga ctggtaatgt 1620
tggttcatta gccaactgtc tcagtctcac tgtattgaat gtatctcata acaacctcgt 1680
aggtgatatc cctaagaaca ataacttctc aagattttca ccagacagct tcattggcaa 1740
tcctggtctt tgcggtagtt ggctaaactc accgtgtcat gattctcgtc gaactgtacg 1800
agtgtcaatc tctagagcag ctattcttgg aatagctatt gggggacttg tgatccttct 1860
catggtctta atagcagctt gccgaccgca taatcctcct ccttttcttg atggatcact 1920

tgacaaacca gtaacttatt cgacaccgaa gctcgtcatc cttcatatga acatggcact 1980
ccacgtttac gaggatatca tgagaatgac agagaatcta agtgagaagt atatcattgg 2040
gcacggagca tcaagcactg tatacaaatg tgttttgaag aattgtaaac cggttgcgat 2100
taagcggctt tactctcaca acccacagtc aatgaaacag tttgaaacag aactcgagat 2160
gctaagtagc atcaagcaca gaaatcttgt gagcctacaa gcttattccc tctctcactt 2220
ggggagtctt ctgttctatg actatttgga aaatggtagc ctctgggatc ttcttcatgg 2280
ccctacgaag aaaaagactc ttgattggga cacacggctt aagatagcat atggtgcagc 2340
acaaggttta gcttatctac accatgactg tagtccaagg atcattcaca gagacgtgaa 2400
gtcgtccaac attctcttgg acaaagactt agaggctcgt ttgacagatt ttggaatagc 2460
gaaaagcttg tgtgtgtcaa agtcacatac ttcaacttac gtgatgggca cgataggtta 2520
catagacccc gagtatgctc gcacttcacg gctcactgag aaatccgatg tctacagtta 2580
tggaatagtc cttcttgagt tgttaacccg aaggaaagcc gttgatgacg aatccaatct 2640
ccaccatctg ataatgtcaa agacggggaa caatgaagtg atggaaatgg cagatccaga 2700
catcacatcg acgtgtaaag atctcggtgt ggtgaagaaa gttttccaac tggcactcct 2760
atgcaccaaa agacagccga atgatcgacc cacaatgcac caggtgactc gtgttctcgg 2820
cagttttatg ctatcggaac aaccacctgc tgcgactgac acgtcagcga cgctggctgg 2880
ttcgtgctac gtcgatgagt atgcaaatct caagactcct cattctgtca attgctcttc 2940
catgagtgct tctgatgctc aactgtttct tcggtttgga caagttattt ctcagaacag 3000
tgagtagttt ttcgttagga ggagaatctt taaaacggta tcttttcgtt gcgttaagct 3060
gttagaaaaa ttaatgtctc atgtaaagta ttatgcactg ccttattatt attagacaag 3120
tgtgtggtgt gaatatgtct tcagactggc acttagactt cctataagtt cttgcc 3176

<210> 2

<211> 976
<212> PRT
<213> Arabidopsis thaliana ERECTA allele

<400> 2

```
Met Ala Leu Phe Arg Asp Ile Val Leu Leu Gly Phe Leu Phe Cys Leu
1               5                   10                  15

Ser Leu Val Ala Thr Val Thr Ser Glu Glu Gly Ala Thr Leu Leu Glu
            20                  25                  30

Ile Lys Lys Ser Phe Lys Asp Val Asn Asn Val Leu Tyr Asp Trp Thr
        35                  40                  45

Thr Ser Pro Ser Ser Asp Tyr Cys Val Trp Arg Gly Val Ser Cys Glu
    50                  55                  60

Asn Val Thr Phe Asn Val Val Ala Leu Asn Leu Ser Asp Leu Asn Leu
65                  70                  75                  80

Asp Gly Glu Ile Ser Pro Ala Ile Gly Asp Leu Lys Ser Leu Leu Ser
                85                  90                  95

Ile Asp Leu Arg Gly Asn Arg Leu Ser Gly Gln Ile Pro Asp Glu Ile
            100                 105                 110

Gly Asp Cys Ser Ser Leu Gln Asn Leu Asp Leu Ser Phe Asn Glu Leu
        115                 120                 125

Ser Gly Asp Ile Pro Phe Ser Ile Ser Lys Leu Lys Gln Leu Glu Gln
    130                 135                 140
```

Leu Ile Leu Lys Asn Asn Gln Leu Ile Gly Pro Ile Pro Ser Thr Leu
145 150 155 160

Ser Gln Ile Pro Asn Leu Lys Ile Leu Asp Leu Ala Gln Asn Lys Leu
165 170 175

Ser Gly Glu Ile Pro Arg Leu Ile Tyr Trp Asn Glu Val Leu Gln Tyr
180 185 190

Leu Gly Leu Arg Gly Asn Asn Leu Val Gly Asn Ile Ser Pro Asp Leu
195 200 205

Cys Gln Leu Thr Gly Leu Trp Tyr Phe Asp Val Arg Asn Asn Ser Leu
210 215 220

Thr Gly Ser Ile Pro Glu Thr Ile Gly Asn Cys Thr Ala Phe Gln Val
225 230 235 240

Leu Asp Leu Ser Tyr Asn Gln Leu Thr Gly Glu Ile Pro Phe Asp Ile
245 250 255

Gly Phe Leu Gln Val Ala Thr Leu Ser Leu Gln Gly Asn Gln Leu Ser
260 265 270

Gly Lys Ile Pro Ser Val Ile Gly Leu Met Gln Ala Leu Ala Val Leu
275 280 285

Asp Leu Ser Gly Asn Leu Leu Ser Gly Ser Ile Pro Pro Ile Leu Gly
290 295 300

Asn Leu Thr Phe Thr Glu Lys Leu Tyr Leu His Ser Asn Lys Leu Thr
305 310 315 320

Gly Ser Ile Pro Pro Glu Leu Gly Asn Met Ser Lys Leu His Tyr Leu
325 330 335

Glu Leu Asn Asp Asn His Leu Thr Gly His Ile Pro Pro Glu Leu Gly
340 345 350

Lys Leu Thr Asp Leu Phe Asp Leu Asn Val Ala Asn Asn Asp Leu Glu
355 360 365

Gly Pro Ile Pro Asp His Leu Ser Ser Cys Thr Asn Leu Asn Ser Leu
370 375 380

Asn Val His Gly Asn Lys Phe Ser Gly Thr Ile Pro Arg Ala Phe Gln
385 390 395 400

Lys Leu Glu Ser Met Thr Tyr Leu Asn Leu Ser Ser Asn Asn Ile Lys
405 410 415

Gly Pro Ile Pro Val Glu Leu Ser Arg Ile Gly Asn Leu Asp Thr Leu
420 425 430

Asp Leu Ser Asn Asn Lys Ile Asn Gly Ile Ile Pro Ser Ser Leu Gly
435 440 445

Asp Leu Glu His Leu Leu Lys Met Asn Leu Ser Arg Asn His Ile Thr
450 455 460

Gly Val Val Pro Gly Asp Phe Gly Asn Leu Arg Ser Ile Met Glu Ile
465 470 475 480

Asp Leu Ser Asn Asn Asp Ile Ser Gly Pro Ile Pro Glu Glu Leu Asn
485 490 495

Gln Leu Gln Asn Ile Ile Leu Leu Arg Leu Glu Asn Asn Asn Leu Thr
500 505 510

Gly Asn Val Gly Ser Leu Ala Asn Cys Leu Ser Leu Thr Val Leu Asn
515 520 525

Val Ser His Asn Asn Leu Val Gly Asp Ile Pro Lys Asn Asn Asn Phe
530 535 540

Ser Arg Phe Ser Pro Asp Ser Phe Ile Gly Asn Pro Gly Leu Cys Gly
545 550 555 560

Ser Trp Leu Asn Ser Pro Cys His Asp Ser Arg Arg Thr Val Arg Val
565 570 575

Ser Ile Ser Arg Ala Ala Ile Leu Gly Ile Ala Ile Gly Gly Leu Val
580 585 590

Ile Leu Leu Met Val Leu Ile Ala Ala Cys Arg Pro His Asn Pro Pro
595 600 605

Pro Phe Leu Asp Gly Ser Leu Asp Lys Pro Val Thr Tyr Ser Thr Pro
610 615 620

Lys Leu Val Ile Leu His Met Asn Met Ala Leu His Val Tyr Glu Asp
625 630 635 640

Ile Met Arg Met Thr Glu Asn Leu Ser Glu Lys Tyr Ile Ile Gly His
645 650 655

Gly Ala Ser Ser Thr Val Tyr Lys Cys Val Leu Lys Asn Cys Lys Pro

660 665 670

Val Ala Ile Lys Arg Leu Tyr Ser His Asn Pro Gln Ser Met Lys Gln
675 680 685

Phe Glu Thr Glu Leu Glu Met Leu Ser Ser Ile Lys His Arg Asn Leu
690 695 700

Val Ser Leu Gln Ala Tyr Ser Leu Ser His Leu Gly Ser Leu Leu Phe
705 710 715 720

Tyr Asp Tyr Leu Glu Asn Gly Ser Leu Trp Asp Leu Leu His Gly Pro
725 730 735

Thr Lys Lys Lys Thr Leu Asp Trp Asp Thr Arg Leu Lys Ile Ala Tyr
740 745 750

Gly Ala Ala Gln Gly Leu Ala Tyr Leu His His Asp Cys Ser Pro Arg
755 760 765

Ile Ile His Arg Asp Val Lys Ser Ser Asn Ile Leu Leu Asp Lys Asp
770 775 780

Leu Glu Ala Arg Leu Thr Asp Phe Gly Ile Ala Lys Ser Leu Cys Val
785 790 795 800

Ser Lys Ser His Thr Ser Thr Tyr Val Met Gly Thr Ile Gly Tyr Ile
805 810 815

Asp Pro Glu Tyr Ala Arg Thr Ser Arg Leu Thr Glu Lys Ser Asp Val
820 825 830

Tyr Ser Tyr Gly Ile Val Leu Leu Glu Leu Leu Thr Arg Arg Lys Ala
835 840 845

Val Asp Asp Glu Ser Asn Leu His His Leu Ile Met Ser Lys Thr Gly
850 855 860

Asn Asn Glu Val Met Glu Met Ala Asp Pro Asp Ile Thr Ser Thr Cys
865 870 875 880

Lys Asp Leu Gly Val Val Lys Lys Val Phe Gln Leu Ala Leu Leu Cys
885 890 895

Thr Lys Arg Gln Pro Asn Asp Arg Pro Thr Met His Gln Val Thr Arg
900 905 910

Val Leu Gly Ser Phe Met Leu Ser Glu Gln Pro Pro Ala Ala Thr Asp
915 920 925

Thr Ser Ala Thr Leu Ala Gly Ser Cys Tyr Val Asp Glu Tyr Ala Asn
930 935 940

Leu Lys Thr Pro His Ser Val Asn Cys Ser Ser Met Ser Ala Ser Asp
945 950 955 960

Ala Gln Leu Phe Leu Arg Phe Gly Gln Val Ile Ser Gln Asn Ser Glu
965 970 975

<210> 3
<211> 3000
<212> DNA
<213> rice ERECTA

<400> 3

atggcggcgg cgagggcgcc gtggctgtgg tggtgggtgg tggtggttgt tggtgtggcg 60

gtggcggagg cggcctccgg aggaggagga gggggagatg gggaggggaa ggcgctgatg 120

ggcgtgaagg ccggtttcgg gaacgcggcc aacgcgctcg tcgactggga cggcggcgcc 180

gaccactgcg cgtggcgcgg cgtcacctgc gacaacgcct ccttcgccgt cctcgccctg 240

aacttgtcaa atctaaacct aggaggtgag atctcgccgg ccatcggaga gctcaagaat 300

ctacagttcg ttgatctcaa ggggaacaag ctcactggcc aaatcccaga tgagattggg 360

gactgcatct ccttaaaata tttggatttg tctggcaact tgctgtatgg agacatcccc 420

ttctccatct ccaagctcaa gcagcttgag gagctgattt tgaagaacaa ccagctcacg 480

ggacccatcc cttccacatt gtcccaaatt ccaaatctca agacattgga cctggcacag 540

aaccagctta caggcgatat cccaaggctc atatactgga atgaagttct gcaataccta 600

ggtttgaggg gtaactcact gactggaact ttgtcacctg acatgtgcca actgactggc 660

ctgtggtact ttgatgtaag gggaaacaat ctcacaggga ccattccaga gagcataggg 720

aactgcacca gctttgagat tctggacatt tcgtataacc aaatctctgg agaaatacct 780

tacaacatag gctttcttca agtagccaca ctgtcacttc aaggaaatag actgactggg 840

aaaattccag atgtgattgg cctgatgcaa gctcttgctg ttctagacct gagtgagaac 900

gagctggtag ggcccattcc ttctatactg ggcaatctat cctatactgg aaaactatat 960

ttacatggga acaaacttac tggagtcata ccgccggagc ttgggaacat gagtaaactt 1020

agctacctac aactgaatga taatgaattg gtgggcacaa ttccagcaga gcttggcaaa 1080

cttgaagagc tttttgaact aaatcttgcc aacaacaatc ttcaaggtcc tattcctgca 1140

aacatcagtt cttgcactgc tctaaacaaa ttcaatgttt atggcaataa gctaaatggt 1200

tctattcctg ctggtttcca gaagttggag agtctgactt acttgaacct atcttcaaac 1260

aatttcaaag gcaatattcc ttctgagctt ggtcacatca tcaacttgga cacattggat 1320

ctttcctaca atgaattctc tggaccagtt cctgctacca ttggtgatct agagcacctt 1380

cttgaactga atttgagtaa gaaccatctt gatgggccag ttcctgctga gtttggaaac 1440

ttgagaagcg tccaagtaat tgatatgtcc aacaacaact tatctggtag tctgcccgag 1500

gaacttggac aacttcaaaa ccttgatagc ctgattctta acaacaacaa tttggttggg 1560

gagatccctg ctcaattggc caactgcttc agcttaaata accttgcatt tcaggaattt 1620

gtcatacaac aatttatctg gacatgtccc gatggcaaag aacttctcga aattcccaat 1680

ggaaagcatc ttctaatttc tgattgcaac cagtacataa atcataaatg cagcttcttg 1740

ggtaatccat tactgcatgt ttactgccaa gattccagct gtggacactc tcatggacaa 1800

agagttaata tttcaaagac agcaattgct tgcattatct taggctttat catattgctc 1860

tgcgttctgc tgttggctat atataaaaca aatcaaccac agccacttgt caaaggatcc 1920

gataagccag tgcaaggacc tccaaagcta gttgttctcc agatggacat ggctatccat 1980

acttacgagg acatcatgag gctgacagag aatttgagcg agaaatacat cattggctat 2040

ggcgcctcaa gcactgtcta caaatgtgaa ctcaagagcg gcaaggccat tgctgtcaag 2100

cggctttaca gtcagtataa ccatagcctc cgagagtttg aaacagaact agagacaatt 2160

ggcagcatac ggcacaggaa tcttgttagc ctccatggct tctcgctatc tccacatgga 2220

aacttgctct tctatgatta catggaaaat ggttccttgt gggatcttct ccacggtcca 2280

tcaaagaaag tgaagctcaa ctgggacaca agactgagga tcgcggtcgg agctgcacaa 2340

gggctggcct atctccacca tgactgcaac cctcgcataa tccacagaga tgtcaagtcc 2400

tccaacatcc tgctcgacga gaacttcgaa gcgcacctct cagatttcgg catagccaaa 2460

tgtgtcccct ctgccaagtc ccatgcctcc acttatgtgc taggaaccat cggctacatt 2520

gatccggagt atgccaggac ttccaggctc aatgagaaat ctgatgtgta cagcttcggc 2580

atcgtccttc tggaattgct cacagggaag aaggccgtcg acaacgaatc gaacttgcat 2640

caattgatac tctccaaagc tgatgacaac acagtcatgg aggcagtgga ctcggaggtg 2700

tcagtgacgt gcacggacat gggactggtc aggaaggcct tccagctcgc ccttctgtgc 2760

accaagaggc acccttcaga ccggccgacc atgcacgagg ttgcaagggt gctgctctcc 2820

ctgctgccgg cctccgccat gacaacgccc aagacggtgg actactcccg gttgctggcg 2880

tcgacgacga cggcggccga catgcgaggg cacgacgtga ccgacatcgg cgacaacagc 2940

tcctccgacg agcagtggtt cgtcaggttc ggcgaggtca tatccaagca cacaatgtga 3000

<210> 4
<211> 999
<212> PRT
<213> rice ERECTA

<400> 4

Met Ala Ala Ala Arg Ala Pro Trp Leu Trp Trp Trp Val Val Val Val
1               5                   10                  15

Val Gly Val Ala Val Ala Glu Ala Ala Ser Gly Gly Gly Gly Gly Gly
            20                  25                  30

Asp Gly Glu Gly Lys Ala Leu Met Gly Val Lys Ala Gly Phe Gly Asn
        35                  40                  45

Ala Ala Asn Ala Leu Val Asp Trp Asp Gly Gly Ala Asp His Cys Ala
    50                  55                  60

Trp Arg Gly Val Thr Cys Asp Asn Ala Ser Phe Ala Val Leu Ala Leu
65                  70                  75                  80

Asn Leu Ser Asn Leu Asn Leu Gly Gly Glu Ile Ser Pro Ala Ile Gly
                85                  90                  95

Glu Leu Lys Asn Leu Gln Phe Val Asp Leu Lys Gly Asn Lys Leu Thr
100 105 110

Gly Gln Ile Pro Asp Glu Ile Gly Asp Cys Ile Ser Leu Lys Tyr Leu
115 120 125

Asp Leu Ser Gly Asn Leu Leu Tyr Gly Asp Ile Pro Phe Ser Ile Ser
130 135 140

Lys Leu Lys Gln Leu Glu Glu Leu Ile Leu Lys Asn Asn Gln Leu Thr
145 150 155 160

Gly Pro Ile Pro Ser Thr Leu Ser Gln Ile Pro Asn Leu Lys Thr Leu
165 170 175

Asp Leu Ala Gln Asn Gln Leu Thr Gly Asp Ile Pro Arg Leu Ile Tyr
180 185 190

Trp Asn Glu Val Leu Gln Tyr Leu Gly Leu Arg Gly Asn Ser Leu Thr
195 200 205

Gly Thr Leu Ser Pro Asp Met Cys Gln Leu Thr Gly Leu Trp Tyr Phe
210 215 220

Asp Val Arg Gly Asn Asn Leu Thr Gly Thr Ile Pro Glu Ser Ile Gly
225 230 235 240

Asn Cys Thr Ser Phe Glu Ile Leu Asp Ile Ser Tyr Asn Gln Ile Ser
245 250 255

Gly Glu Ile Pro Tyr Asn Ile Gly Phe Leu Gln Val Ala Thr Leu Ser

260 265 270

Leu Gln Gly Asn Arg Leu Thr Gly Lys Ile Pro Asp Val Ile Gly Leu
275 280 285

Met Gln Ala Leu Ala Val Leu Asp Leu Ser Glu Asn Glu Leu Val Gly
290 295 300

Pro Ile Pro Ser Ile Leu Gly Asn Leu Ser Tyr Thr Gly Lys Leu Tyr
305 310 315 320

Leu His Gly Asn Lys Leu Thr Gly Val Ile Pro Pro Glu Leu Gly Asn
325 330 335

Met Ser Lys Leu Ser Tyr Leu Gln Leu Asn Asp Asn Glu Leu Val Gly
340 345 350

Thr Ile Pro Ala Glu Leu Gly Lys Leu Glu Glu Leu Phe Glu Leu Asn
355 360 365

Leu Ala Asn Asn Asn Leu Gln Gly Pro Ile Pro Ala Asn Ile Ser Ser
370 375 380

Cys Thr Ala Leu Asn Lys Phe Asn Val Tyr Gly Asn Lys Leu Asn Gly
385 390 395 400

Ser Ile Pro Ala Gly Phe Gln Lys Leu Glu Ser Leu Thr Tyr Leu Asn
405 410 415

Leu Ser Ser Asn Asn Phe Lys Gly Asn Ile Pro Ser Glu Leu Gly His
420 425 430

Ile Ile Asn Leu Asp Thr Leu Asp Leu Ser Tyr Asn Glu Phe Ser Gly
435 440 445

Pro Val Pro Ala Thr Ile Gly Asp Leu Glu His Leu Leu Glu Leu Asn
450 455 460

Leu Ser Lys Asn His Leu Asp Gly Pro Val Pro Ala Glu Phe Gly Asn
465 470 475 480

Leu Arg Ser Val Gln Val Ile Asp Met Ser Asn Asn Asn Leu Ser Gly
485 490 495

Ser Leu Pro Glu Glu Leu Gly Gln Leu Gln Asn Leu Asp Ser Leu Ile
500 505 510

Leu Asn Asn Asn Asn Leu Val Gly Glu Ile Pro Ala Gln Leu Ala Asn
515 520 525

Cys Phe Ser Leu Asn Asn Leu Ala Phe Gln Glu Phe Val Ile Gln Gln
530 535 540

Phe Ile Trp Thr Cys Pro Asp Gly Lys Glu Leu Leu Glu Ile Pro Asn
545 550 555 560

Gly Lys His Leu Leu Ile Ser Asp Cys Asn Gln Tyr Ile Asn His Lys
565 570 575

Cys Ser Phe Leu Gly Asn Pro Leu Leu His Val Tyr Cys Gln Asp Ser
580 585 590

Ser Cys Gly His Ser His Gly Gln Arg Val Asn Ile Ser Lys Thr Ala
595 600 605

Ile Ala Cys Ile Ile Leu Gly Phe Ile Ile Leu Leu Cys Val Leu Leu
610 615 620

Leu Ala Ile Tyr Lys Thr Asn Gln Pro Gln Pro Leu Val Lys Gly Ser
625 630 635 640

Asp Lys Pro Val Gln Gly Pro Pro Lys Leu Val Val Leu Gln Met Asp
645 650 655

Met Ala Ile His Thr Tyr Glu Asp Ile Met Arg Leu Thr Glu Asn Leu
660 665 670

Ser Glu Lys Tyr Ile Ile Gly Tyr Gly Ala Ser Ser Thr Val Tyr Lys
675 680 685

Cys Glu Leu Lys Ser Gly Lys Ala Ile Ala Val Lys Arg Leu Tyr Ser
690 695 700

Gln Tyr Asn His Ser Leu Arg Glu Phe Glu Thr Glu Leu Glu Thr Ile
705 710 715 720

Gly Ser Ile Arg His Arg Asn Leu Val Ser Leu His Gly Phe Ser Leu
725 730 735

Ser Pro His Gly Asn Leu Leu Phe Tyr Asp Tyr Met Glu Asn Gly Ser
740 745 750

Leu Trp Asp Leu Leu His Gly Pro Ser Lys Lys Val Lys Leu Asn Trp
755 760 765

Asp Thr Arg Leu Arg Ile Ala Val Gly Ala Ala Gln Gly Leu Ala Tyr
770 775 780

Leu His His Asp Cys Asn Pro Arg Ile Ile His Arg Asp Val Lys Ser
785                 790                 795                 800

Ser Asn Ile Leu Leu Asp Glu Asn Phe Glu Ala His Leu Ser Asp Phe
                805                 810                 815

Gly Ile Ala Lys Cys Val Pro Ser Ala Lys Ser His Ala Ser Thr Tyr
            820                 825                 830

Val Leu Gly Thr Ile Gly Tyr Ile Asp Pro Glu Tyr Ala Arg Thr Ser
        835                 840                 845

Arg Leu Asn Glu Lys Ser Asp Val Tyr Ser Phe Gly Ile Val Leu Leu
    850                 855                 860

Glu Leu Leu Thr Gly Lys Lys Ala Val Asp Asn Glu Ser Asn Leu His
865                 870                 875                 880

Gln Leu Ile Leu Ser Lys Ala Asp Asp Asn Thr Val Met Glu Ala Val
                885                 890                 895

Asp Ser Glu Val Ser Val Thr Cys Thr Asp Met Gly Leu Val Arg Lys
            900                 905                 910

Ala Phe Gln Leu Ala Leu Leu Cys Thr Lys Arg His Pro Ser Asp Arg
        915                 920                 925

Pro Thr Met His Glu Val Ala Arg Val Leu Leu Ser Leu Leu Pro Ala
    930                 935                 940

Ser Ala Met Thr Thr Pro Lys Thr Val Asp Tyr Ser Arg Leu Leu Ala

945 950 955 960

Ser Thr Thr Thr Ala Ala Asp Met Arg Gly His Asp Val Thr Asp Ile
965 970 975

Gly Asp Asn Ser Ser Ser Asp Glu Gln Trp Phe Val Arg Phe Gly Glu
980 985 990

Val Ile Ser Lys His Thr Met
995

<210> 5
<211> 2766
<212> DNA
<213> sorghum ERECTA

<400> 5

atgacgacga cggccgcccg tgctctcgtc gccctcctcc tcgtcgccgt cgccgtcgcc 60

gacgatgggg cgacgctggt ggagatcaag aagtccttcc gcaacgtcgg caacgtactg 120

tacgattggg ccggcgacga ctactgctcc tggcgcggcg tcctgtgcga caacgtcaca 180

ttcgccgtcg ctgcgctcaa cctctctggc ctcaaccttg agggcgagat ctctccagcc 240

gtcggcagcc tcaagagcct cgtctccatc gatctgaagt caaatgggct atccgggcag 300

atccctgatg agattggtga ttgttcatca cttaggacgc tggacttttc tttcaacaac 360

ttggatggcg acataccatt ttctatatca aagctgaagc acctggagaa cttgatattg 420

aagaacaacc agctgattgg tgcgatccca tcaacattgt cacagctccc aaatttgaag 480

attttggatt tggcacaaaa caaactgact ggggagatac caaggcttat ctactggaat 540

gaggttcttc aatatcttga tgtgaagaac aatagcttga ccggggtgat accagacacc 600

attgggaact gtacaagttt tcaagtcttg gatttgtctt acaaccgctt tactggacca 660

atcccattca acattggttt cctacaagtg gctacactat ccttgcaagg gaacaagttc 720
accggtccaa ttccttcagt aattggtctt atgcaggctc tcgctgttct agatctgagt 780
tacaaccaat tatctggtcc tataccatca atactaggca acttgacata cactgagaag 840
ctgtacatcc aaggcaataa gttaactggg tcgataccac cagagttagg aaatatgtca 900
acacttcatt acctagaact gaacgataat caacttactg ggtcaattcc accagagctt 960
ggaaggctaa caggcttgtt tgacctgaac cttgcgaata accacctgga aggaccaatt 1020
cctgacaacc taagttcatg tgtgaatctc aatagcttca atgcttatgg caacaagtta 1080
aatgggacca ttcctcgttc gttgcggaaa cttgaaagca tgacctattt aaatctgtca 1140
tcaaacttca taagtggctc tattcctatt gagttatcaa ggatcaacaa tttggacacg 1200
ctggatttat cctgtaacat gatgactggt ccaattccat catcaattgg cagcctagag 1260
catctattga gacttaactt gagcaagaat ggtctagttg gattcatccc cgcggagttt 1320
ggtaatttga ggagtgtcat ggagattgat ttatcctata atcaccttgg tggcctgatt 1380
cctcaagaac ttgaaatgct gcaaaacctg atgttgctaa atgtgtcgta caataatttg 1440
gctggtgttg tccctgctga caacaacttc acacggtttt cacctgacag ctttttaggt 1500
aatcctggac tctgtggata ctggcttggt tcgtcgtgtc gttccactgg ccaccacgag 1560
aaaccgccta tctcaaaggc tgccataatt ggtgttgctg tgggtggact tgttatcctc 1620
ttgatgatct tagtagctgt ttgcaggcca catcgtccac ctgcttttaa agatgtcact 1680
gtaagcaagc cagtgagaaa tgctcccccc aagctggtga tccttcatat gaacatggcc 1740
cttcatgtat acgatgacat aatgaggatg actgagaact tgagtgagaa atacatcatt 1800
ggatacgggg cgtcaagtac agtttataaa tgtgtcctaa agaattgcaa accggtggca 1860
ataaaaaagc tgtatgccca ctacccacag agccttaagg aatttgaaac tgagcttgag 1920
actgttggta gcatcaagca ccggaatcta gtcagccttc aagggtactc attatcacct 1980

gttgggaacc tcctctttta tgattatatg gaatgtggca gcttatggga tgttttacat 2040

gaaggttcat ccaagaagaa aaaacttgac tgggagactc gcctacggat tgctcttggt 2100

gcagctcaag gccttgctta ccttcaccat gactgcagtc cacggataat tcatcgggat 2160

gtaaaatcaa agaatatact ccttgacaaa gattatgagg cccatcttac agactttgga 2220

attgctaaga gcttatgtgt ctcaaaaact cacacatcaa cctatgtcat gggaactatt 2280

ggctacattg atcctgagta cgcccgcact tcccgtctca acgaaaagtc tgatgtctac 2340

aggctatggc attgttctgc tggagctgct gactggcaag aagccagtgg acaacgaatc 2400

ctatcgaaga cggcaagcaa cgaggtcatg gataccgtgg accctgacat cggggacacc 2460

tgcaaggacc tcggcgaggt gaagaagctg ttccagctgg cgctcctttg caccaagcgg 2520

caaccctcgg accgaccgac gatgcacgag gtggtgcgcg tcctggactg cctggtgaac 2580

ccggacccgc cgccaaagcc gtcggcgcac cagctgccgc agccgtcgcc agccgtgcca 2640

agctacatca acgagtacgt cagcctgcgg ggcaccggcg ctctctcctg cgccaactcg 2700

accagcacct cggacgccga gctgttcctc aagttcggcg aggccatctc gcagaacatg 2760

gagtag 2766

<210> 6
<211> 921
<212> PRT
<213> Sorghum ERECTA

<400> 6

Met Thr Thr Thr Ala Ala Arg Ala Leu Val Ala Leu Leu Leu Val Ala
1 5 10 15

Val Ala Val Ala Asp Asp Gly Ala Thr Leu Val Glu Ile Lys Lys Ser
20 25 30

Phe Arg Asn Val Gly Asn Val Leu Tyr Asp Trp Ala Gly Asp Asp Tyr
35 40 45

Cys Ser Trp Arg Gly Val Leu Cys Asp Asn Val Thr Phe Ala Val Ala
50 55 60

Ala Leu Asn Leu Ser Gly Leu Asn Leu Glu Gly Glu Ile Ser Pro Ala
65 70 75 80

Val Gly Ser Leu Lys Ser Leu Val Ser Ile Asp Leu Lys Ser Asn Gly
85 90 95

Leu Ser Gly Gln Ile Pro Asp Glu Ile Gly Asp Cys Ser Ser Leu Arg
100 105 110

Thr Leu Asp Phe Ser Phe Asn Asn Leu Asp Gly Asp Ile Pro Phe Ser
115 120 125

Ile Ser Lys Leu Lys His Leu Glu Asn Leu Ile Leu Lys Asn Asn Gln
130 135 140

Leu Ile Gly Ala Ile Pro Ser Thr Leu Ser Gln Leu Pro Asn Leu Lys
145 150 155 160

Ile Leu Asp Leu Ala Gln Asn Lys Leu Thr Gly Glu Ile Pro Arg Leu
165 170 175

Ile Tyr Trp Asn Glu Val Leu Gln Tyr Leu Asp Val Lys Asn Asn Ser
180 185 190

Leu Thr Gly Val Ile Pro Asp Thr Ile Gly Asn Cys Thr Ser Phe Gln

195 200 205

Val Leu Asp Leu Ser Tyr Asn Arg Phe Thr Gly Pro Ile Pro Phe Asn
210 215 220

Ile Gly Phe Leu Gln Val Ala Thr Leu Ser Leu Gln Gly Asn Lys Phe
225 230 235 240

Thr Gly Pro Ile Pro Ser Val Ile Gly Leu Met Gln Ala Leu Ala Val
245 250 255

Leu Asp Leu Ser Tyr Asn Gln Leu Ser Gly Pro Ile Pro Ser Ile Leu
260 265 270

Gly Asn Leu Thr Tyr Thr Glu Lys Leu Tyr Ile Gln Gly Asn Lys Leu
275 280 285

Thr Gly Ser Ile Pro Pro Glu Leu Gly Asn Met Ser Thr Leu His Tyr
290 295 300

Leu Glu Leu Asn Asp Asn Gln Leu Thr Gly Ser Ile Pro Pro Glu Leu
305 310 315 320

Gly Arg Leu Thr Gly Leu Phe Asp Leu Asn Leu Ala Asn Asn His Leu
325 330 335

Glu Gly Pro Ile Pro Asp Asn Leu Ser Ser Cys Val Asn Leu Asn Ser
340 345 350

Phe Asn Ala Tyr Gly Asn Lys Leu Asn Gly Thr Ile Pro Arg Ser Leu
355 360 365

Arg Lys Leu Glu Ser Met Thr Tyr Leu Asn Leu Ser Ser Asn Phe Ile
370 375 380

Ser Gly Ser Ile Pro Ile Glu Leu Ser Arg Ile Asn Asn Leu Asp Thr
385 390 395 400

Leu Asp Leu Ser Cys Asn Met Met Thr Gly Pro Ile Pro Ser Ser Ile
405 410 415

Gly Ser Leu Glu His Leu Leu Arg Leu Asn Leu Ser Lys Asn Gly Leu
420 425 430

Val Gly Phe Ile Pro Ala Glu Phe Gly Asn Leu Arg Ser Val Met Glu
435 440 445

Ile Asp Leu Ser Tyr Asn His Leu Gly Gly Leu Ile Pro Gln Glu Leu
450 455 460

Glu Met Leu Gln Asn Leu Met Leu Leu Asn Val Ser Tyr Asn Asn Leu
465 470 475 480

Ala Gly Val Val Pro Ala Asp Asn Asn Phe Thr Arg Phe Ser Pro Asp
485 490 495

Ser Phe Leu Gly Asn Pro Gly Leu Cys Gly Tyr Trp Leu Gly Ser Ser
500 505 510

Cys Arg Ser Thr Gly His His Glu Lys Pro Pro Ile Ser Lys Ala Ala
515 520 525

Ile Ile Gly Val Ala Val Gly Gly Leu Val Ile Leu Leu Met Ile Leu
530 535 540

Val Ala Val Cys Arg Pro His Arg Pro Pro Ala Phe Lys Asp Val Thr
545 550 555 560

Val Ser Lys Pro Val Arg Asn Ala Pro Pro Lys Leu Val Ile Leu His
565 570 575

Met Asn Met Ala Leu His Val Tyr Asp Asp Ile Met Arg Met Thr Glu
580 585 590

Asn Leu Ser Glu Lys Tyr Ile Ile Gly Tyr Gly Ala Ser Ser Thr Val
595 600 605

Tyr Lys Cys Val Leu Lys Asn Cys Lys Pro Val Ala Ile Lys Lys Leu
610 615 620

Tyr Ala His Tyr Pro Gln Ser Leu Lys Glu Phe Glu Thr Glu Leu Glu
625 630 635 640

Thr Val Gly Ser Ile Lys His Arg Asn Leu Val Ser Leu Gln Gly Tyr
645 650 655

Ser Leu Ser Pro Val Gly Asn Leu Leu Phe Tyr Asp Tyr Met Glu Cys
660 665 670

Gly Ser Leu Trp Asp Val Leu His Glu Gly Ser Ser Lys Lys Lys Lys
675 680 685

Leu Asp Trp Glu Thr Arg Leu Arg Ile Ala Leu Gly Ala Ala Gln Gly
690 695 700

Leu Ala Tyr Leu His His Asp Cys Ser Pro Arg Ile Ile His Arg Asp
705 710 715 720

Val Lys Ser Lys Asn Ile Leu Leu Asp Lys Asp Tyr Glu Ala His Leu
725 730 735

Thr Asp Phe Gly Ile Ala Lys Ser Leu Cys Val Ser Lys Thr His Thr
740 745 750

Ser Thr Tyr Val Met Gly Thr Ile Gly Tyr Ile Asp Pro Glu Tyr Ala
755 760 765

Arg Thr Ser Arg Leu Asn Glu Lys Ser Asp Val Tyr Arg Leu Trp His
770 775 780

Cys Ser Ala Gly Ala Ala Asp Trp Gln Glu Ala Ser Gly Gln Arg Ile
785 790 795 800

Leu Ser Lys Thr Ala Ser Asn Glu Val Met Asp Thr Val Asp Pro Asp
805 810 815

Ile Gly Asp Thr Cys Lys Asp Leu Gly Glu Val Lys Lys Leu Phe Gln
820 825 830

Leu Ala Leu Leu Cys Thr Lys Arg Gln Pro Ser Asp Arg Pro Thr Met
835 840 845

His Glu Val Val Arg Val Leu Asp Cys Leu Val Asn Pro Asp Pro Pro
850 855 860

Pro Lys Pro Ser Ala His Gln Leu Pro Gln Pro Ser Pro Ala Val Pro
865 870 875 880

Ser Tyr Ile Asn Glu Tyr Val Ser Leu Arg Gly Thr Gly Ala Leu Ser

885 890 895

Cys Ala Asn Ser Thr Ser Thr Ser Asp Ala Glu Leu Phe Leu Lys Phe
900 905 910

Gly Glu Ala Ile Ser Gln Asn Met Glu
915 920

<210> 7
<211> 2751
<212> DNA
<213> Arabidopsis thaliana ERECTA homolog

<400> 7

```
atggcgataa aggcttcatt cagcaacgtg gcgaatatgc ttcttgattg ggacgatgtt      60
cataaccacg acttttgttc ttggagaggt gtcttctgtg ataacgttag cctcaatgtt     120
gtctctctta atctgtcaaa cctgaatctt ggtggagaga tatcatctgc ccttggagat     180
ttgatgaatc tgcaatcaat agacttgcaa ggaaataaat tgggtggtca aattccagat     240
gagattggaa actgtgtttc tcttgcttat gtggatttct ccaccaattt gttgtttgga     300
gacataccgt tttcaatctc taaactcaaa cagctgacct taactcagat tccaaacctt     360
aagacccttg acctcgcaag aaaccagctt actggtgaga taccaaggtt actctactgg     420
aatgaagttt tacagtatct cggtttacgt gggaatatgt taactgggac attgtctcct     480
gatatgtgtc agctgacggg tctgtggtac tttgatgtga gaggcaacaa ccttactgga     540
actatcccag agagcattgg caattgcaca agctttgaga tcttggatgt atcttataat     600
cagattaccg gagttatacc ctacaatatt ggtttcctcc aagtagctac tctgtcactt     660
caaggaaaca agttgactgg cagaattccg gaagtgattg gtctgatgca ggctcttgct     720
gtattggatt tgagtgacaa tgaattaact gggcctattc caccaatact tgggaatctg     780
```

tcattcactg gaaaactgta tctccatggc aacaagctca ctggacaaat cccacccgag 840

ctaggcaata tgtcacgact cagctatttg caactaaatg ataatgaact agtgggaaag 900

atcccacctg agcttgggaa gctggaacaa ttgttcgaac tgaatcttgc gaacaacaat 960

cttgtagggc tgattccatc taacattagt tcctgtgctg ccttgaatca attcaatgtt 1020

catgggaact tcttgagtgg agctgtacca cttgaattcc ggaatcttgg aagcttgact 1080

tatctaaatc tttcctcaaa cagtttcaag ggcaaaatac ctgctgagct tggccatatc 1140

atcaatcttg atacattgga tctgtctggc aacaatttct caggctcaat tccattaaca 1200

cttggtgatc ttgagcatct tctcatctta aacttgagca gaaatcatct gaatggcaca 1260

ttgcctgcag aattcgggaa cctccgaagc attcagatca tcgatgtgtc atttaatttt 1320

cttgccggtg ttattccaac tgaacttggc cagttgcaga acataaactc tctgatactg 1380

aacaacaaca agattcatgg gaaaatccct gatcagctaa ctaactgctt cagtcttgcc 1440

aatctgaaca tctccttcaa taatctttct ggaataatcc cacctatgaa gaactttaca 1500

cgtttttccc cggccagctt ctttggaaat ccatttctct gcgggaactg ggttggatca 1560

atctgtggcc catctttacc taagtcacaa gtattcacca gagttgccgt gatttgtatg 1620

gttctcggtt tcatcactct catatgcatg atattcattg cggtttacaa gtcaaagcag 1680

cagaaaccag tcttgaaagg ctcttcaaaa caacctgaag ggtcaacgaa gctggtgatt 1740

cttcacatgg acatggctat tcacacgttt gatgatatca tgagagttac agaaaacctc 1800

gatgagaaat acatcattgg atacggtgct tctagcacag tttacaagtg cacctccaaa 1860

acttcccgac ctattgccat taagcgaatc tacaatcagt atcccagcaa cttccgcgag 1920

tttgaaacag agctcgagac cattgggagc atcagacaca gaaacatagt aagcttgcac 1980

ggatacgcct tatctccctt tggcaacctc ctcttctacg actacatgga aaatggctct 2040

ctttgggatc ttctccatgg gcctgggaag aaggtgaagc ttgactggga aacaaggctg 2100

aagatagctg ttggagctgc gcaaggactt gcatatcttc accatgactg cacacctagg 2160

ataatccatc gagacatcaa gtcatcaaac atactccttg atgggaattt cgaagcgcgt 2220

ttgtcagatt ttgggattgc caagagcata ccagccacca aaacttatgc ttcaacctat 2280

gttcttggaa ccattggata tattgaccca gagtatgctc gaacttcgcg tctgaacgag 2340

aagtctgata tctacagttt cggtattgtc cttcttgagc ttctaaccgg caagaaggct 2400

gtggataacg aggccaactt gcatcaaatg attctatcaa aggcggatga taacacagta 2460

atggaagctg ttgatgcaga ggtctcagtg acttgcatgg actcaggaca catcaagaaa 2520

acatttcagc tagctctctt gtgcaccaag cgaaatcctt tggagagacc caccatgcag 2580

gaggtctcta gggttctgct ctcacttgtc ccgtctccac ctccaaagaa gttaccgtcg 2640

cctgcaaaag tacaggaagg ggaagaacgg cgtgagagcc actcttcaga tacaacaacc 2700

ccacagtggt ttgttcagtt ccgtgaagat atctccaaaa gtagcttata a 2751

<210> 8
<211> 932
<212> PRT
<213> Arabidopsis thaliana ERECTA homolog

<400> 8

Met Ala Ile Lys Ala Ser Phe Ser Asn Val Ala Asn Met Leu Leu Asp
1 5 10 15

Trp Asp Asp Val His Asn His Asp Phe Cys Ser Trp Arg Gly Val Phe
20 25 30

Cys Asp Asn Val Ser Leu Asn Val Val Ser Leu Asn Leu Ser Asn Leu
35 40 45

Asn Leu Gly Gly Glu Ile Ser Ser Ala Leu Gly Asp Leu Met Asn Leu
50 55 60

Gln Ser Ile Asp Leu Gln Gly Asn Lys Leu Gly Gly Gln Ile Pro Asp
65 70 75 80

Glu Ile Gly Asn Cys Val Ser Leu Ala Tyr Val Asp Phe Ser Thr Asn
85 90 95

Leu Leu Phe Gly Asp Ile Pro Phe Ser Ile Ser Lys Leu Lys Gln Leu
100 105 110

Glu Phe Leu Asn Leu Lys Asn Asn Gln Leu Thr Gly Pro Ile Pro Ala
115 120 125

Thr Leu Thr Gln Ile Pro Asn Leu Lys Thr Leu Asp Leu Ala Arg Asn
130 135 140

Gln Leu Thr Gly Glu Ile Pro Arg Leu Leu Tyr Trp Asn Glu Val Leu
145 150 155 160

Gln Tyr Leu Gly Leu Arg Gly Asn Met Leu Thr Gly Thr Leu Ser Pro
165 170 175

Asp Met Cys Gln Leu Thr Gly Leu Trp Tyr Phe Asp Val Arg Gly Asn
180 185 190

Asn Leu Thr Gly Thr Ile Pro Glu Ser Ile Gly Asn Cys Thr Ser Phe
195 200 205

Glu Ile Leu Asp Val Ser Tyr Asn Gln Ile Thr Gly Val Ile Pro Tyr
210 215 220

Asn Ile Gly Phe Leu Gln Val Ala Thr Leu Ser Leu Gln Gly Asn Lys
225 230 235 240

Leu Thr Gly Arg Ile Pro Glu Val Ile Gly Leu Met Gln Ala Leu Ala
245 250 255

Val Leu Asp Leu Ser Asp Asn Glu Leu Thr Gly Pro Ile Pro Pro Ile
260 265 270

Leu Gly Asn Leu Ser Phe Thr Gly Lys Leu Tyr Leu His Gly Asn Lys
275 280 285

Leu Thr Gly Gln Ile Pro Pro Glu Leu Gly Asn Met Ser Arg Leu Ser
290 295 300

Tyr Leu Gln Leu Asn Asp Asn Glu Leu Val Gly Lys Ile Pro Pro Glu
305 310 315 320

Leu Gly Lys Leu Glu Gln Leu Phe Glu Leu Asn Leu Ala Asn Asn Asn
325 330 335

Leu Val Gly Leu Ile Pro Ser Asn Ile Ser Ser Cys Ala Ala Leu Asn
340 345 350

Gln Phe Asn Val His Gly Asn Phe Leu Ser Gly Ala Val Pro Leu Glu
355 360 365

Phe Arg Asn Leu Gly Ser Leu Thr Tyr Leu Asn Leu Ser Ser Asn Ser
370 375 380

Phe Lys Gly Lys Ile Pro Ala Glu Leu Gly His Ile Ile Asn Leu Asp
385 390 395 400

Thr Leu Asp Leu Ser Gly Asn Asn Phe Ser Gly Ser Ile Pro Leu Thr
405 410 415

Leu Gly Asp Leu Glu His Leu Leu Ile Leu Asn Leu Ser Arg Asn His
420 425 430

Leu Asn Gly Thr Leu Pro Ala Glu Phe Gly Asn Leu Arg Ser Ile Gln
435 440 445

Ile Ile Asp Val Ser Phe Asn Phe Leu Ala Gly Val Ile Pro Thr Glu
450 455 460

Leu Gly Gln Leu Gln Asn Ile Asn Ser Leu Ile Leu Asn Asn Asn Lys
465 470 475 480

Ile His Gly Lys Ile Pro Asp Gln Leu Thr Asn Cys Phe Ser Leu Ala
485 490 495

Asn Leu Asn Ile Ser Phe Asn Asn Leu Ser Gly Ile Ile Pro Pro Met
500 505 510

Lys Asn Phe Thr Arg Phe Ser Pro Ala Ser Phe Phe Gly Asn Pro Phe
515 520 525

Leu Cys Gly Asn Trp Val Gly Ser Ile Cys Gly Pro Ser Leu Pro Lys
530 535 540

Ser Gln Val Phe Thr Arg Val Ala Val Ile Cys Met Val Leu Gly Phe
545 550 555 560

Ile Thr Leu Ile Cys Met Ile Phe Ile Ala Val Tyr Lys Ser Lys Gln

565 570 575

Gln Lys Pro Val Leu Lys Gly Ser Ser Lys Gln Pro Glu Gly Ser Thr
580 585 590

Lys Leu Val Ile Leu His Met Asp Met Ala Ile His Thr Phe Asp Asp
595 600 605

Ile Met Arg Val Thr Glu Asn Leu Asp Glu Lys Tyr Ile Ile Gly Tyr
610 615 620

Gly Ala Ser Ser Thr Val Tyr Lys Cys Thr Ser Lys Thr Ser Arg Pro
625 630 635 640

Ile Ala Ile Lys Arg Ile Tyr Asn Gln Tyr Pro Ser Asn Phe Arg Glu
645 650 655

Phe Glu Thr Glu Leu Glu Thr Ile Gly Ser Ile Arg His Arg Asn Ile
660 665 670

Val Ser Leu His Gly Tyr Ala Leu Ser Pro Phe Gly Asn Leu Leu Phe
675 680 685

Tyr Asp Tyr Met Glu Asn Gly Ser Leu Trp Asp Leu Leu His Gly Pro
690 695 700

Gly Lys Lys Val Lys Leu Asp Trp Glu Thr Arg Leu Lys Ile Ala Val
705 710 715 720

Gly Ala Ala Gln Gly Leu Ala Tyr Leu His His Asp Cys Thr Pro Arg
725 730 735

Ile Ile His Arg Asp Ile Lys Ser Ser Asn Ile Leu Leu Asp Gly Asn
740 745 750

Phe Glu Ala Arg Leu Ser Asp Phe Gly Ile Ala Lys Ser Ile Pro Ala
755 760 765

Thr Lys Thr Tyr Ala Ser Thr Tyr Val Leu Gly Thr Ile Gly Tyr Ile
770 775 780

Asp Pro Glu Tyr Ala Arg Thr Ser Arg Leu Asn Glu Lys Ser Asp Ile
785 790 795 800

Tyr Ser Phe Gly Ile Val Leu Leu Glu Leu Leu Thr Gly Lys Lys Ala
805 810 815

Val Asp Asn Glu Ala Asn Leu His Gln Met Ile Leu Ser Lys Ala Asp
820 825 830

Asp Asn Thr Val Met Glu Ala Val Asp Ala Glu Val Ser Val Thr Cys
835 840 845

Met Asp Ser Gly His Ile Lys Lys Thr Phe Gln Leu Ala Leu Leu Cys
850 855 860

Thr Lys Arg Asn Pro Leu Glu Arg Pro Thr Met Gln Glu Val Ser Arg
865 870 875 880

Val Leu Leu Ser Leu Val Pro Ser Pro Pro Pro Lys Lys Leu Pro Ser
885 890 895

Pro Ala Lys Val Gln Glu Gly Glu Glu Arg Arg Glu Ser His Ser Ser
900 905 910

Asp Thr Thr Thr Pro Gln Trp Phe Val Gln Phe Arg Glu Asp Ile Ser
915 920 925

Lys Ser Ser Leu
930

<210> 9
<211> 2901
<212> DNA
<213> Arabidopsis thaliana ERECTA homolog

<400> 9

```
atgaaggaga agatgcagcg aatggtttta tctttagcaa tggtgggttt tatggttttt     60
ggtgttgctt cggctatgaa caacgaaggg aaagctctga tggcgataaa aggctctttc    120
agcaacttag tgaatatgct tttggattgg gacgatgttc acaacagtga cttgtgttct    180
tggcgaggtg ttttctgcga caacgttagc tactccgttg tctctctgaa tttgtccagt    240
ctgaatcttg gaggggagat atctccagct attggagacc tacggaattt gcaatcaata    300
gacttgcaag gtaataaact agcaggtcaa attccagatg agattggaaa ctgtgcttct    360
cttgtttatc tggatttgtc cgagaatctg ttatatggag acataccttt ctcaatctct    420
aaactcaagc agcttgaaac tctgaatctg aagaacaatc agctcacagg tcctgtacca    480
gcaaccttaa cccagattcc aaaccttaag agacttgatc ttgctggcaa tcatctaacg    540
ggtgagatat cgagattgct ttactggaat gaagttttgc agtatcttgg attacgaggg    600
aatatgttga ctggaacgtt atcttctgat atgtgtcagc taaccggttt gtggtacttt    660
gatgtgagag gaaataatct aactggaacc atcccggaga gcatcggaaa ttgcacaagc    720
tttcaaatcc tggacatatc ttataatcag ataacaggag agattcctta caatatcggc    780
ttcctccaag ttgctactct gtcacttcaa ggaaacagat tgacgggtag aattccagaa    840
```

gttattggtc taatgcaggc tcttgctgtt ttggatttga gtgacaatga gcttgttggt 900

cctatcccac cgatacttgg caatctctca tttaccggaa agttgtatct ccatggcaat 960

atgctcactg gtccaatccc ctctgagctt gggaatatgt cacgtctcag ctatttgcag 1020

ctaaacgaca ataaactagt gggaactatt ccacctgagc ttggaaagct ggagcaattg 1080

tttgaactga atcttgccaa caaccgttta gtagggccca taccatccaa cattagttca 1140

tgtgcagcct tgaatcaatt caatgttcat gggaacctct tgagtggatc tattccactg 1200

gcgtttcgca atctcgggag cttgacttat ctgaatcttt cgtcgaacaa tttcaaggga 1260

aaaataccag ttgagcttgg acatataatc aatcttgaca aactagatct gtctggcaat 1320

aacttctcag ggtctatacc attaacgctt ggcgatcttg aacaccttct catattaaat 1380

cttagcagaa accatcttag tggacaatta cctgcagagt ttgggaacct tcgaagcatt 1440

cagatgattg atgtatcatt caatctgctc tccggagtta ttccaactga acttggccaa 1500

ttgcagaatt taaactcttt aatattgaac aacaacaagc ttcatgggaa aattccagat 1560

cagcttacga actgcttcac tcttgtcaat ctgaatgtct ccttcaacaa tctctccggg 1620

atagtcccac caatgaaaaa cttctcacgt tttgctccag ccagctttgt tggaaatcca 1680

tatctttgtg gaaactgggt tggatctatt tgtggtcctt taccgaaatc tcgagtattc 1740

tccagaggtg ctttgatctg cattgttctt ggcgtcatca ctctcctatg tatgattttc 1800

cttgcagttt acaaatcaat gcagcagaag aagattctac aaggctcctc aaaacaagct 1860

gaagggttaa ccaagctagt gattctccac atggacatgg caattcatac atttgatgat 1920

atcatgagag tgactgagaa tcttaacgaa aagtttataa ttggatatgg tgcttctagc 1980

acggtataca aatgtgcatt aaaaagttcc cgacctattg ccattaagcg actctacaat 2040

cagtatccgc ataacttgcg ggaatttgag acagaacttg agaccattgg gagcattagg 2100

cacagaaaca tagtcagctt gcatggatat gccttgtctc ctactggcaa ccttcttttc 2160

tatgactaca tggaaaatgg atcactttgg gaccttcttc atgggtcatt gaagaaagtg 2220

aagcttgatt gggagacaag gttgaagata gcggttggag ctgcacaagg actagcctat 2280

cttcaccacg attgtactcc tcgaatcatt caccgtgaca tcaagtcatc gaacatactt 2340

cttgatgaga atttcgaagc acatttatct gatttcggga ttgctaagag cataccagct 2400

agcaaaaccc atgcctcgac ttatgttttg ggaacaattg gttatataga cccagagtat 2460

gctcgtactt cacgaatcaa tgagaaatcc gatatataca gcttcggtat tgttcttctt 2520

gagcttctca ctgggaagaa agcagtggat aacgaagcta acttgcatca actgatattg 2580

tcaaaggctg atgataatac tgtgatggaa gcagttgatc cagaggttac tgtgacttgt 2640

atggacttgg gacatatcag gaagacattt cagctggctc tcttatgcac aaagcgaaac 2700

cctttagaga gacccacaat gcttgaagtc tctagggttc tgctctctct tgtcccatct 2760

ctgcaagtag caaagaagct accttctctt gatcactcaa ccaaaaagct gcagcaagag 2820

aatgaagtta ggaatcctga tgcagaagca tctcaatggt ttgttcagtt ccgtgaagtc 2880

atctccaaaa gtagcatata a 2901

<210> 10
<211> 966
<212> PRT
<213> Arabidopsis thaliana ERECTA homolog

<400> 10

Met Lys Glu Lys Met Gln Arg Met Val Leu Ser Leu Ala Met Val Gly
1 5 10 15

Phe Met Val Phe Gly Val Ala Ser Ala Met Asn Asn Glu Gly Lys Ala
20 25 30

Leu Met Ala Ile Lys Gly Ser Phe Ser Asn Leu Val Asn Met Leu Leu
35 40 45

Asp Trp Asp Asp Val His Asn Ser Asp Leu Cys Ser Trp Arg Gly Val
50 55 60

Phe Cys Asp Asn Val Ser Tyr Ser Val Val Ser Leu Asn Leu Ser Ser
65 70 75 80

Leu Asn Leu Gly Gly Glu Ile Ser Pro Ala Ile Gly Asp Leu Arg Asn
85 90 95

Leu Gln Ser Ile Asp Leu Gln Gly Asn Lys Leu Ala Gly Gln Ile Pro
100 105 110

Asp Glu Ile Gly Asn Cys Ala Ser Leu Val Tyr Leu Asp Leu Ser Glu
115 120 125

Asn Leu Leu Tyr Gly Asp Ile Pro Phe Ser Ile Ser Lys Leu Lys Gln
130 135 140

Leu Glu Thr Leu Asn Leu Lys Asn Asn Gln Leu Thr Gly Pro Val Pro
145 150 155 160

Ala Thr Leu Thr Gln Ile Pro Asn Leu Lys Arg Leu Asp Leu Ala Gly
165 170 175

Asn His Leu Thr Gly Glu Ile Ser Arg Leu Leu Tyr Trp Asn Glu Val
180 185 190

Leu Gln Tyr Leu Gly Leu Arg Gly Asn Met Leu Thr Gly Thr Leu Ser
195 200 205

Ser Asp Met Cys Gln Leu Thr Gly Leu Trp Tyr Phe Asp Val Arg Gly
210 215 220

Asn Asn Leu Thr Gly Thr Ile Pro Glu Ser Ile Gly Asn Cys Thr Ser
225 230 235 240

Phe Gln Ile Leu Asp Ile Ser Tyr Asn Gln Ile Thr Gly Glu Ile Pro
245 250 255

Tyr Asn Ile Gly Phe Leu Gln Val Ala Thr Leu Ser Leu Gln Gly Asn
260 265 270

Arg Leu Thr Gly Arg Ile Pro Glu Val Ile Gly Leu Met Gln Ala Leu
275 280 285

Ala Val Leu Asp Leu Ser Asp Asn Glu Leu Val Gly Pro Ile Pro Pro
290 295 300

Ile Leu Gly Asn Leu Ser Phe Thr Gly Lys Leu Tyr Leu His Gly Asn
305 310 315 320

Met Leu Thr Gly Pro Ile Pro Ser Glu Leu Gly Asn Met Ser Arg Leu
325 330 335

Ser Tyr Leu Gln Leu Asn Asp Asn Lys Leu Val Gly Thr Ile Pro Pro
340 345 350

Glu Leu Gly Lys Leu Glu Gln Leu Phe Glu Leu Asn Leu Ala Asn Asn
355 360 365

Arg Leu Val Gly Pro Ile Pro Ser Asn Ile Ser Ser Cys Ala Ala Leu
370 375 380

Asn Gln Phe Asn Val His Gly Asn Leu Leu Ser Gly Ser Ile Pro Leu
385 390 395 400

Ala Phe Arg Asn Leu Gly Ser Leu Thr Tyr Leu Asn Leu Ser Ser Asn
405 410 415

Asn Phe Lys Gly Lys Ile Pro Val Glu Leu Gly His Ile Ile Asn Leu
420 425 430

Asp Lys Leu Asp Leu Ser Gly Asn Asn Phe Ser Gly Ser Ile Pro Leu
435 440 445

Thr Leu Gly Asp Leu Glu His Leu Leu Ile Leu Asn Leu Ser Arg Asn
450 455 460

His Leu Ser Gly Gln Leu Pro Ala Glu Phe Gly Asn Leu Arg Ser Ile
465 470 475 480

Gln Met Ile Asp Val Ser Phe Asn Leu Leu Ser Gly Val Ile Pro Thr
485 490 495

Glu Leu Gly Gln Leu Gln Asn Leu Asn Ser Leu Ile Leu Asn Asn Asn
500 505 510

Lys Leu His Gly Lys Ile Pro Asp Gln Leu Thr Asn Cys Phe Thr Leu
515 520 525

Val Asn Leu Asn Val Ser Phe Asn Asn Leu Ser Gly Ile Val Pro Pro
530 535 540

Met Lys Asn Phe Ser Arg Phe Ala Pro Ala Ser Phe Val Gly Asn Pro

545 550 555 560

Tyr Leu Cys Gly Asn Trp Val Gly Ser Ile Cys Gly Pro Leu Pro Lys
565 570 575

Ser Arg Val Phe Ser Arg Gly Ala Leu Ile Cys Ile Val Leu Gly Val
580 585 590

Ile Thr Leu Leu Cys Met Ile Phe Leu Ala Val Tyr Lys Ser Met Gln
595 600 605

Gln Lys Lys Ile Leu Gln Gly Ser Ser Lys Gln Ala Glu Gly Leu Thr
610 615 620

Lys Leu Val Ile Leu His Met Asp Met Ala Ile His Thr Phe Asp Asp
625 630 635 640

Ile Met Arg Val Thr Glu Asn Leu Asn Glu Lys Phe Ile Ile Gly Tyr
645 650 655

Gly Ala Ser Ser Thr Val Tyr Lys Cys Ala Leu Lys Ser Ser Arg Pro
660 665 670

Ile Ala Ile Lys Arg Leu Tyr Asn Gln Tyr Pro His Asn Leu Arg Glu
675 680 685

Phe Glu Thr Glu Leu Glu Thr Ile Gly Ser Ile Arg His Arg Asn Ile
690 695 700

Val Ser Leu His Gly Tyr Ala Leu Ser Pro Thr Gly Asn Leu Leu Phe
705 710 715 720

Tyr Asp Tyr Met Glu Asn Gly Ser Leu Trp Asp Leu Leu His Gly Ser
725 730 735

Leu Lys Lys Val Lys Leu Asp Trp Glu Thr Arg Leu Lys Ile Ala Val
740 745 750

Gly Ala Ala Gln Gly Leu Ala Tyr Leu His His Asp Cys Thr Pro Arg
755 760 765

Ile Ile His Arg Asp Ile Lys Ser Ser Asn Ile Leu Leu Asp Glu Asn
770 775 780

Phe Glu Ala His Leu Ser Asp Phe Gly Ile Ala Lys Ser Ile Pro Ala
785 790 795 800

Ser Lys Thr His Ala Ser Thr Tyr Val Leu Gly Thr Ile Gly Tyr Ile
805 810 815

Asp Pro Glu Tyr Ala Arg Thr Ser Arg Ile Asn Glu Lys Ser Asp Ile
820 825 830

Tyr Ser Phe Gly Ile Val Leu Leu Glu Leu Leu Thr Gly Lys Lys Ala
835 840 845

Val Asp Asn Glu Ala Asn Leu His Gln Leu Ile Leu Ser Lys Ala Asp
850 855 860

Asp Asn Thr Val Met Glu Ala Val Asp Pro Glu Val Thr Val Thr Cys
865 870 875 880

Met Asp Leu Gly His Ile Arg Lys Thr Phe Gln Leu Ala Leu Leu Cys
885 890 895

Thr Lys Arg Asn Pro Leu Glu Arg Pro Thr Met Leu Glu Val Ser Arg
900 905 910

Val Leu Leu Ser Leu Val Pro Ser Leu Gln Val Ala Lys Lys Leu Pro
915 920 925

Ser Leu Asp His Ser Thr Lys Lys Leu Gln Gln Glu Asn Glu Val Arg
930 935 940

Asn Pro Asp Ala Glu Ala Ser Gln Trp Phe Val Gln Phe Arg Glu Val
945 950 955 960

Ile Ser Lys Ser Ser Ile
965

<210> 11
<211> 636
<212> DNA
<213> partial wheat ERECTA

<400> 11

atgaattctg caatacctag gcttgagggg taactcactg actggaacct tgtcacctga 60

catgtgccaa ctcactggcc tgtggtactt tgatgtgagg ggcaacaatc taacaggaac 120

aattccacag agcataggga actgcactag ctttgagatt ctggacattt catataacaa 180

aatctctgga gaaatacctt acaacatagg tttccttcaa gtagctacac tgtcacttca 240

aggaaataga ctgactggga aaattccaga agtgattggc ctcatgcaag ctcttgctgt 300

tcttgatctg agcgaaaacg aactagtagg ggccattcct ccgatactcg gcaacctgtc 360

ctacactggc aaactatatt tgcatggcaa taaacttact ggtgaagtac cccggaact 420

tgggaacatg actaaactta gctacctgca actgaatgac aatgaattag tgggcgcaat 480

tccagctgag cttgggaaac ttgaagagct attcgaatta aatcttgcca acaacaatct 540

tgagggtcct attcctacaa acatcagttc ttgcactgca ctaaacaaat tcaatgttta 600

cggcaataga ttgaacggtt ctatccctgc tggttt 636

<210> 12
<211> 466
<212> DNA
<213> partial wheat ERECTA

<400> 12

ttcaatgttt atggcaatag attgaacggt tctatccctg ctggtttcca gaatttggag 60

agtttgacta acttgaattt atcctcaaac aattttaaag gccatatccc atctgaactt 120

ggtcatatca tcaatttgga cacactggat ctttcctaca atgaactctc tggaccagtt 180

cctgctacta ttggtgatct tgagcatctt cttcaactaa atttgagcaa aaaccatctt 240

agcgggtcag tgcctgctga gttcggaaac ttgagaagca tccaagtaat tgatttatcc 300

aacaacgcca tgtctggtta tctccctgaa gaactaggcc aacttcagaa ccttgatagt 360

ttgattctta acaacaatat tttggtcgga gagatccctg ctcagttggc taactgcttc 420

agcttaaaca tcttgaactt gtcacataac aacttttctg gacatg 466

<210> 13
<211> 372
<212> DNA
<213> partial wheat ERECTA

<220>
<221> misc_feature
<222> (62)..(62)
<223> not determined

<400> 13

ttgtcagcct tctggcttct cactctctcc caatggaaac ctgctcttct acgattacat 60

gngaaaacgg ttccttgtgg gatcttctcc atggtccatc aaagaaagtg aagcttgact 120

gggacacccg actgaggatc gcggtcggcg cggcacaagg gctggcctat ctgcaccatg 180

actgcaatct gcggatagtc cacagggacg tcaagtcctc caacatcctg ctcgacgagc 240

actttgaagc gcatctctcg gacttcggca tcgccaaatg cgtcccggca gccaagaccc 300

atgcgtccac atatgtgcta ggaaccatcg gctacatcga tccagagtac gcccggacgt 360

cgaggttgaa cg 372

<210> 14
<211> 357
<212> DNA
<213> partial wheat ERECTA

<400> 14

ttgactaact tgaatttatc ctcaaacaat tttaaaggcc atatcccatc tgaacttggt 60

catatcatca atttggacac actggatctt tcctacaatg aactctctgg accagttcct 120

gctactattg gtgatcttga gcatcttctt caactaaatt tgagcaaaaa ccatcttagc 180

gggtcagtgc ctgctgagtt cggaaacttg agaagcatcc aagtaattga tttatccaac 240

aacgccatgt ctggttatct ccctgaagaa ctaggccaac ttcagaacct tgatagtttg 300

attcttaaca acaatatttt ggtcggggag atccctgctc agttggctaa ctgcttc 357

<210> 15
<211> 314
<212> DNA
<213> partial wheat ERECTA

<400> 15

cacactggat ctttcctaca atgaattctc tggaccagac cctgctacta ttggtgatct 60

tgagcatgtt cttcagatta aatttgagca aaaaccatct tactgggcca atgcctgctg 120

agttctgaaa cttgagaagc atccaagtaa ttgatttatc caacaacgcc atgtctggtt 180

atctccctga agaactacgc caacttcaga atcttgatag tttgatgctt aacaacaata 240

ctttggttgg ggagatccct gctcatctgg ctaactgctt caacttaaac atcttgaact 300

tgccatataa caac 314

<210> 16
<211> 549
<212> DNA
<213> partial wheat ERECTA

<400> 16

catcatcggc tacggcgctt caagtaccgt gtataaatgt gtgctcaaga gtggcaaggc 60

cattgctgtg aagcggctct acagccaata caaccatggc gcccgtgagt ttgagacaga 120

gctggagaca gtcggtagca tccggcacag gaatcttgtc agccttcatg gcttctcact 180

ctctccaaat ggaaacctgc tcttctacga ttacatggaa aatggttcct tgtgggatct 240

tctccacggt ccatcaaaga aggtgaaact tgactgggac acccgactga gaatcgccgt 300

cggtgcggca caagggctgg catatcttca ccatgactgc aaccctcgga tagtccacag 360

ggacgtcaag tcctccaaca tcctgctcga cgagcacttt gaagcgcatc tctcggactt 420

cggcatcgcc aaatgcgtcc cagctgccaa gacccacgcg tccacctatg tgctaggaac 480

catcggctac atcgatccag agtacgcccg gacgtcccag ctgaacgaga aatctgatgt 540

gtacagctt 549

<210> 17
<211> 615
<212> DNA
<213> partial wheat ERECTA

<400> 17

ccacgcgtcg atcatcaatt gggacacacg ggatctttcc tacaatgaat tctccgggcc 60

```
agttcctgct actattggtg atctggagca tcttcttcaa ctaaatttga gcaaaaacca      120
tcttagtggg tctgtgcctg ctgagttcgg aaacttgaga agcatccaag taattgattt      180
atccaacaac gccatttctg gttatctccc tgaagaacta ggccaacttc agaaccttga      240
tagtttgatt cttaacaaca atactttggt tggggagatc cctgctcagt tggctaactg      300
cttcagctta aacatcttga acttgtcata taacaacttt tctggacatg tcccattcgc      360
taagaacttc tcaaagttcc ccggggaaag cttcttggga aatccgatgc tgagcgttca      420
ctgcaaagac tccagctgtg gcaactctca tggatcaaaa gtgaatactc ggacagcgat      480
tgcttgcatc atctcgggct tcgtcatatt gctctgtgtt ctgctattgg gcaatatata      540
aaacaaagcg accacagcca cctatcaaag catctgataa accagggcaa ggacctccaa      600
agatagtact cctcc                                                        615
```

<210> 18
<211> 719
<212> DNA
<213> partial wheat ERECTA

<400> 18

```
cgttcactgc aaagactcca gctgtggcaa ctctcatgga tcaaaagtga atattcggac       60
ggcgattgct tgcatcatct cgggcttcgt catactgcta tgtgttctgc tattggcaat      120
atataaaaca aagcgaccac agccacctat caaagcatct gataaaccag tgcaaggacc      180
tccaaagata gtactcctcc aaatggacat ggctatccat acctatgatg atattatgag      240
gctgacagag aatttgagcg agaaatacat catcggctac ggcgcttcaa gtaccgtgta      300
taaatgtgtg ctcaagagtg gcaaggccat tgctgtgaag cggctctaca gccaatacaa      360
ccatggcgcc cgtgagtttg agacagagct ggagacagtc ggtagcatcc ggcacaggaa      420
tcttgtcagc cttcatggct tctcactctc tccaaatgga aacctgctct tctacgatta      480
```

catggaaaat ggttccttgt gggatcttct ccacggtcca tcaaagaagg tgaaacttga 540

ctgggacacc cgactgagaa tcgccgtcgg tgcggcacaa gggctggcat atcttcacca 600

tgactgcaac cctcggatag tccacaggga cgtcaagtcc tccaacatcc tgctcgacga 660

gcactttgaa gcgcatctct cggacttcgg catcgcccaa tgcgtcccca gctgccaag 719

<210> 19
<211> 1346
<212> DNA
<213> wheat ERECTA

<400> 19

ttcaatgttt atggcaatag attgaacggt tctatccctg ctggtttcca gaatttggag 60

agtttgacta acttgaattt atcctcaaac aattttaaag gccatatccc atctgaactt 120

ggtcatatca tcaatttgga cacactggat ctttcctaca atgaactctc tggaccagtt 180

cctgctacta ttggtgatct tgagcatctt cttcaactaa atttgagcaa aaaccatctt 240

agcgggtcag tgcctgctga gttcggaaac ttgagaagca tccaagtaat tgatttatcc 300

aacaacgcca tgtctggtta tctccctgaa gaactaggcc aacttcagaa ccttgatagt 360

ttgattctta acaacaatat tttggtcggg gagatccctg ctcagttggc taactgcttc 420

agcttaaaca tcttgaactt gtcacataac aacttttctg gacatgtccc attcgctaag 480

aacttctcaa agttccccgg ggaaagcttc ttgggaaatc cgatgctgag cgttcactgc 540

aaagactcca gctgtggcaa ctctcatgga tcaaaagtga atactcggac agcgattgct 600

tgcatcatct cgggcttcgt catactgcta tgtgttctgc tattggcaat atataaaaca 660

aagcgaccac agccacctat caaagcatct gataaaccag ggcaaggacc tccaaagata 720

gtactcctcc aaatggacat ggctatccat acctatgatg atattatgag gctgacagag 780

aatttgagcg agaaatacat catcggctac ggcgcttcaa gtaccgtgta taaatgtgtg 840

ctcaagagtg gcaaggccat tgctgtgaag cggctctaca gccaatacaa ccatggcgcc 900

cgtgagtttg agacagagct ggagacagtc ggtagcatcc ggcacaggaa tcttgtcagc 960

cttcatggct tctcactctc tccaaatgga aacctgctct tctacgatta catggaaaat 1020

ggttccttgt gggatcttct ccacggtcca tcaaagaagg tgaaacttga ctgggacacc 1080

cgactgagaa tcgccgtcgg tgcggcacaa gggctggcat atcttcacca tgactgcaac 1140

cctcggatag tccacaggga cgtcaagtcc tccaacatcc tgctcgacga gcactttgaa 1200

gcgcatctct cggacttcgg catcgccaaa tgcgtcccag ctgccaagac ccacgcgtcc 1260

acatatgtgc taggaaccat cggctacatc gatccagagt acgcccggac gtcccagctg 1320

aacgagaaat ctgatgtgta cagctt 1346

<210> 20
<211> 448
<212> PRT
<213> wheat ERECTA

<400> 20

Phe Asn Val Tyr Gly Asn Arg Leu Asn Gly Ser Ile Pro Ala Gly Phe
1 5 10 15

Gln Asn Leu Glu Ser Leu Thr Asn Leu Asn Leu Ser Ser Asn Asn Phe
20 25 30

Lys Gly His Ile Pro Ser Glu Leu Gly His Ile Ile Asn Leu Asp Thr
35 40 45

Leu Asp Leu Ser Tyr Asn Glu Leu Ser Gly Pro Val Pro Ala Thr Ile
50 55 60

Gly Asp Leu Glu His Leu Leu Gln Leu Asn Leu Ser Lys Asn His Leu
65 70 75 80

Ser Gly Ser Val Pro Ala Glu Phe Gly Asn Leu Arg Ser Ile Gln Val
85 90 95

Ile Asp Leu Ser Asn Asn Ala Met Ser Gly Tyr Leu Pro Glu Glu Leu
100 105 110

Gly Gln Leu Gln Asn Leu Asp Ser Leu Ile Leu Asn Asn Asn Ile Leu
115 120 125

Val Gly Glu Ile Pro Ala Gln Leu Ala Asn Cys Phe Ser Leu Asn Ile
130 135 140

Leu Asn Leu Ser His Asn Asn Phe Ser Gly His Val Pro Phe Ala Lys
145 150 155 160

Asn Phe Ser Lys Phe Pro Gly Glu Ser Phe Leu Gly Asn Pro Met Leu
165 170 175

Ser Val His Cys Lys Asp Ser Ser Cys Gly Asn Ser His Gly Ser Lys
180 185 190

Val Asn Thr Arg Thr Ala Ile Ala Cys Ile Ile Ser Gly Phe Val Ile
195 200 205

Leu Leu Cys Val Leu Leu Leu Ala Ile Tyr Lys Thr Lys Arg Pro Gln
210 215 220

Pro Pro Ile Lys Ala Ser Asp Lys Pro Gly Gln Gly Pro Pro Lys Ile
225 230 235 240

```
Val Leu Leu Gln Met Asp Met Ala Ile His Thr Tyr Asp Asp Ile Met
                245                 250                 255

Arg Leu Thr Glu Asn Leu Ser Glu Lys Tyr Ile Ile Gly Tyr Gly Ala
            260                 265                 270

Ser Ser Thr Val Tyr Lys Cys Val Leu Lys Ser Gly Lys Ala Ile Ala
        275                 280                 285

Val Lys Arg Leu Tyr Ser Gln Tyr Asn His Gly Ala Arg Glu Phe Glu
    290                 295                 300

Thr Glu Leu Glu Thr Val Gly Ser Ile Arg His Arg Asn Leu Val Ser
305                 310                 315                 320

Leu His Gly Phe Ser Leu Ser Pro Asn Gly Asn Leu Leu Phe Tyr Asp
                325                 330                 335

Tyr Met Glu Asn Gly Ser Leu Trp Asp Leu Leu His Gly Pro Ser Lys
            340                 345                 350

Lys Val Lys Leu Asp Trp Asp Thr Arg Leu Arg Ile Ala Val Gly Ala
        355                 360                 365

Ala Gln Gly Leu Ala Tyr Leu His His Asp Cys Asn Pro Arg Ile Val
    370                 375                 380

His Arg Asp Val Lys Ser Ser Asn Ile Leu Leu Asp Glu His Phe Glu
385                 390                 395                 400

Ala His Leu Ser Asp Phe Gly Ile Ala Lys Cys Val Pro Ala Ala Lys
                405                 410                 415
```

Thr His Ala Ser Thr Tyr Val Leu Gly Thr Ile Gly Tyr Ile Asp Pro
420 425 430

Glu Tyr Ala Arg Thr Ser Gln Leu Asn Glu Lys Ser Asp Val Tyr Ser
435 440 445

<210> 21
<211> 1273
<212> DNA
<213> partial maize ERECTA

<400> 21

cctggactct gtggatattg gcttggttct tcatgtcgtt ccactggcca ccgagacaaa 60

ccgccaatct caaaggctgc cataattggt gttgctgtgg gtggacttgt tatcctcctg 120

atgatcttag tagctgtatg caggccacac catccacctg cttttaaaga tgccactgta 180

agcaagccag tgagcaatgg tccacccaag ctggtgatcc ttcatatgaa catggctctt 240

catgtctttg atgatataat gaggatgact gagaacttga gtgagaaata catcattgga 300

tacggggcat caagtacagt ttataaatgt gttctaaaga attgcaaacc agtggcaata 360

aaaaagctgt atgcccacta ccctgcagag ccttaaggaa tttgaaactg agctcgagac 420

tgttggtagc atcaaacacc ggaatctagt cagccttgcc aagggtactc gttgtcacct 480

gttgggaacc tcctctttta tgattatatg gagagtggca gcttatggga tgttttacat 540

gaaggctcat ccaagaagaa caaacttgac tgggtgactc gcctacggat cgctcttggt 600

gcagctcaag gcctcgctta ccttcaccat gactgcagcc cacgaataat tcaccgggac 660

gtaaaatcaa agaatatact cctcgacaaa gattatgagg cccatcttac agacttcggc 720

atcgctaaga gcttatgtgt ctcgaagact cacacgtcaa cctacgtcat gggcactatt 780

ggttacattg atcccgagta cgcccgcacc tcccgcctca acgagaagtc tgatgtctac 840

agctacggca tcgttctgct ggagctgctg accggcaaga agccagtgga caacgagtgc 900

aatctccatc acttgatcct atcgaagacg gcgagcaacg aggtcatgga gacggtggac 960

cccgacgtgg gagacacctg caaggacctg ggcgaggtga agaagctgtt ccagctggcg 1020

ctcctctgca ccaagcggca gccctcggac cggccgacga tgcacgaggt ggtgcgcgtc 1080

cttgactgcc tggtgaaccc ggagccgccg ccgcagccgc agcagcagca gcagaagggc 1140

gcacgcgcac caccagctgc cgccgcagcc gtcgccgccg gcctacgtcg acgagtacgt 1200

cagcctgcgg ggcactggcg ccctctcctg cgccaactcg tccagcacct cggacgccga 1260

gctgttcctc aag 1273

<210> 22
<211> 100
<212> DNA
<213> partial maize ERECTA

<400> 22

cacaaaatgt cagtcaaact actccccctg caatcggcct cactcaaggc gcctcaccga 60

acgtctacgt cttcccctac accatgttct gcgagatggc 100

<210> 23
<211> 599
<212> DNA
<213> partial maize ERECTA

<220>
<221> misc_feature
<222> (529)..(529)
<223> not determined

<400> 23

tttttttttt tttttttttt tttttgagga ggaagctccg ctgctcttgc gttgcgtcac 60

```
atgactttt acagctaaca acaccctagc tactgagtcc catgttaatc tcctgcgctg    120
cgtcccacaa aatgtcagtc aaactactcc ctgcaatcgg cctcactcaa ggcgcctcac    180
cgaacgtcta cgtcttcccc tacaccatgt tctgcgagat ggcctcgccg aacttgagga    240
acagctcggc gtccgaggtg ctggacgagt tggcgcagga gagggcgccg gtgccccgca    300
ggctgacgta ctcgtcgacg taggccggcg gcgacggctg cggcggcagc tggtggtgcg    360
cgtgcgcctt ctgctgctgc tgctgcggct gcggcggcgg ctccgggttc accaggcagt    420
caaggacgcg caccacctcg tgcatcgtcg gccggtccga gggctgccgc ttggtgcaga    480
ggagcgccag ctggaacagc ttcttcacct cgcccaggtc cttgcaggng tctcccacgt    540
cggggtccac cgtctccatg acctcgttgc tcgccgtctt cgataggatc aaggatgga     599
```

<210> 24
<211> 436
<212> DNA
<213> partial maize ERECTA

<400> 24

```
tttttttttt tttttttga agaagctccg ctgctctcgc gttgcgtcac atgactttt      60
acagataaca ccaccctagc tactgagtcc catgttaatc tcctgcgctg cgttccacaa   120
aatgtcagcc aaactactcc ctgcaatcgg cctcactcaa ggcgcctcac cgaacgtcta   180
cgtcttcccc tacaccatgt tctgcgagat ggcctcgccg aacttgagga acagctcggc   240
gtccgaggtg ctggacgagt tggcgcagga gagggcgccg gtgccccgca ggctgacgta   300
ctcgtcgacg taggccggcg gggacggctg cggcggcagc tggtggtgcg cgtgcgcctt   360
ctgctgctgc tgctgcggct gcggcggggg ctccgggttc accaggcagt caaggacgcg   420
caccacctcg ggcatc                                                   436
```

<210> 25
<211> 509
<212> DNA
<213> partial maize ERECTA

<400> 25

```
ccaaagaaaa atggagaggg gggataaaga agatgaggaa gaagctccgc tgctcttgcg    60
ttgcgtcaca tgactttttta cagctaacaa caccctagct actgagtccc atgttaatct   120
cctgcgctgc gtcccacaaa atgtcagtca aactactccc tgcaatcggc ctcactcaag   180
gcgcctcacc gaacgtctac gtcttcccct acaccatgtt ctgcgagatg gcctcgccga   240
acttgaggaa cagctcggcg tccgaggtgc tggacgagtt ggcgcaggag agggcgccgg   300
tgccccgcag gctgacgtac tcgtcgacgt aggccggcgg cgacggctgc ggcggcagct   360
ggtggtgcgc gtgcgccttc tgctgctgct gctgcggctg cggcggcggc tccgggttca   420
ccaggcagtc aaggacgcgc accacctcgt gcatcgtcgg ccggtccgag ggctgccgct   480
tggtgcagag gagcgccagc tggaacagc                                      509
```

<210> 26
<211> 318
<212> DNA
<213> partial maize ERECTA

<400> 26

```
gatggatcaa tacagcctcc tagtaagtta gaccaccaaa gaaaaatggg gaggggggat    60
aaagaagagg aagaagctcc gctgctcttg cgtcacatga cttttttttac agctaacaac   120
accctagcta ctgagtccca tgttaatctc ctgcgctgcg tcccacaaaa tgtcagtcaa   180
actactcccc ctgcaatcgg cctcactcaa ggcgcctcac cgaacgtcta cgtcttcccc   240
tacaccatgt tctgcgagat ggcctcgccg aacttgagga acagctcggc gtccgaggtg   300
ctggacgagt tggcgcag                                                  318
```

<210> 27
<211> 103
<212> DNA
<213> partial maize ERECTA

<400> 27

agcaagccag tgagcaatgg tccacccaag ctggtgatcc ttcatatgaa catggctctt 60

catgtctttg atgatataat gaggatgact gagaacttga gtg 103

<210> 28
<211> 458
<212> DNA
<213> partial maize ERECTA

<400> 28

ataattcacc gggacgtaaa atcaaagaat atactcctcg acaaagatta tgaggcgcat 60

cttacagact tcggcatcgc taagagctta tgtgtctcga agactcacac gtcaacctac 120

gtcatgggca ctattggtac acttgatcct gagtacgccc gcacctcccg cctcaacgag 180

aagtctgatg tctacagcta cggcatcgtt ctgctggagc tgctgaccgg caagaagcca 240

gtggacaacg agtgcaatct ccatcacttg atcctatcga agacggcgag ccaacgaggt 300

catggagacg gtggaccccg acgtgggaga cacctgcaag gacctgggcg aggtgaagaa 360

gctgttccag ctggcgctcc tctgcaccaa gcggcagccc tcggaccggc cgacgatgca 420

cgaggtggtg cgcgtccttg actgcctggt gaacccgg 458

<210> 29
<211> 593
<212> DNA
<213> partial maize ERECTA

<400> 29

tttttttttt tttttttttt ttttttgagg aagaagctcc gctgctcttg cgttgcgtca 60

catgactttt tacagctaac aacaccctag ctactgagtc ccatgttaat ctcctgcgct 120

gcgtcccaca aaatgtcagt caaactactc cctgcaatcg gcctcatttt tttgttgtcc 180

tcaccgaacg tctacgtctt cccctacacc atgttctgcg agatggcctc gccgaacttg 240

aggaacagct cggcgtccga ggtgctggac gagttggcgc aggaaagggc gccggtgccc 300

cgcaggctga cgtactcgtc gacgtaggcc ggcggcgacg gctgcggcgg cagctggtgg 360

tgcgcgtgcg ccttctgctg ctgctgctgc ggctgcggcg gcggctccgg gttcaccagg 420

cagtcaagga cgcgcaccac ctcgtgcatc gtcggccggt ccgagggctg ccgcttggtg 480

cagaggagcg ccagctggaa cagcttcttc acctcgccca ggtccttgca ggtgtctccc 540

acgtcggggt ccaccggctc catgacctcg ttgctcgccg tcttcgatag gat 593

<210> 30
<211> 206
<212> DNA
<213> partial maize ERECTA

<400> 30

tcacaaaaga tcatcaagca gaggaacggg agagatgatg atggatcaat acagcctcct 60

agtaagttag accacaaaga aaaatgggga ggggggataa agaagaggaa gaagctccgc 120

tgctcttgcg tcacatgact ttttttacag ctaacaacac cctagctact gagtcccatg 180

ttaatctcct gcgctgcgtc ccacaa 206

<210> 31
<211> 534
<212> DNA
<213> partial maize ERECTA

<400> 31

caagcagagg aacgggagag atgatgatgg atcaatacag cctcctagta agttagacca 60

caaagaaaaa tggggagggg ggataaagaa gaggaagaag ctccgctgct cttgcgtcac 120

```
atgacttttt ttacagctaa caacacccta gctactgagt cccatgttaa tctcctgcgc    180
tgcgtcccac aaaatgtcag tcaaactact ccccctgcaa tcggcctcac tcaaggcgcc    240
tcaccgaacg tctacgtctt cccctacacc atgttctgcg agatggcctc gccgaacttg    300
aggaacagct cggcgtccga ggtgctggac gagttggcgc aggagagggc gccggtgccc    360
cgcaggctga cgtactcgtc gacgtaggcc ggcggcgacg gctgcggcgg cagctggtgg    420
tgcgcgtgcg gcttctgctg ctgctgctgc ggctgcggcg gcggctccgg gttcaccagg    480
cagtcaagga cgcgcaccac ctcgtgcatc gtcggccggt ccgagggctg ccgc          534
```

<210> 32
<211> 527
<212> DNA
<213> partial maize ERECTA

<400> 32

```
gaaagtcaca agatcataag gaagaggaac gggagagatg atgatggatc aatacagcct     60
cctagtaagt tagaccacca aagaaaaatg gagagggggg ataaagaaga tgaggaagaa    120
gctccgctgc tcttgcgttg cgtcacatga ctttttacag ctaacaacac cctagctact    180
gagtcccatg ttaatctcct gcgctgcgtc ccacaaaatg tcagtcaaac tactccctgc    240
aatcggcctc actcaaggcg cctcaccgaa cgtctacgtc ttcccctaca ccatgttctg    300
cgagatggcc tcgccgaact tgaggaacag ctcggcgtcc gaggtgctgg acgagttggc    360
gcaggaaagg gcgccggtgc cccgcaggct gacgtactcg tcgacgtagg ccggcggcga    420
cggctgcggc ggcagctggt ggtgcgcgtg cgccttctgc tgctgctgct gcggctgcgg    480
cggcggctcc gggttcacca ggcagtcaag gacgcgcacc acctcgt                  527
```

<210> 33
<211> 412
<212> DNA
<213> partial maize ERECTA

<400> 33

cttgcgttgc gtcacatgac tttttacagc taacaacacc ctagctactg agtcccatgt 60

taatctcctg cgctgcgtcc cacaaaatgt cagtcaaact actccctgca atcggcctca 120

ctcagggggc ctcaccgaac gtctacgtct tcccctacac caggttctgc gagatggcct 180

cgccgaactt gaggaacagc tcggcgtccg aggggctgga cgagttggcg caggaaaggg 240

cgccggggcc ccgcaggctg acgtactcgt cgacgtaggc cggcggcgac ggctgcggcg 300

gcagctgggg gtgcgcgtgc gccttctgct gctgctgctg cggttgcggc ggcggctccg 360

ggttcaccag gcagtcaagg acgcgcacca cctcgggcat cgtcggccgg tc 412

<210> 34
<211> 533
<212> DNA
<213> partial maize ERECTA

<400> 34

tcgagttttt tttttttttt ttttgatgat ggatcaatac agcctcctag taagttagac 60

caccaaagaa aaatggagag gggggataaa gaagatgagg aagaagctcc gctgctcttg 120

cgttgcgtca catgactttt tacagctaac aacaccctag ctactgagtc ccatgttaat 180

ctcctgcgct gcgtcccaca aaatgtcagt caaactactc cctgcaatcg gcctcactca 240

aggcgcctca ccgaacgtct acgtcttccc ctacaccatg ttctgcgaga tggcctcgcc 300

gaacttgagg aacagctcgg cgtccgaggt gctggacgag ttggcgcagg agagggcgcc 360

ggtgccccgc aggctgacgt actcgtcgac gtaggccggc ggcgacggct gcggcggcag 420

ctggtggtgc gcgtgcgcct tctgctgctg ctgctgcggc tgcggcggcg gctccgggtt 480

caccaggcag tcaaggacgc gcaccacctc gtgcatcgtc ggccggtccg agg 533

<210> 35
<211> 191
<212> DNA
<213> partial maize ERECTA

<400> 35

```
agcctcctag taagttagac caccaaagaa aaatggagag gggggataaa gaagatgagg      60

aagaagctcc gctgctcttg cgttgcgtca catgactttt tacagctaaa caacacccta     120

gctactgagt cccatggtaa tctcctgcgc tgcgtcccac aaaatgtcag tcaaactact     180

ccctgcaatc g                                                          191
```

<210> 36
<211> 683
<212> DNA
<213> partial maize ERECTA

<400> 36

```
gacgttggga acctcctctt ttatgcttta tggagagtgg cagcttatgg gatgttttac      60

atgaaggctc atccaagaag aacaaacttg actgggtgac tcgcctacgg atcgctcttg     120

gtgcagctca aggcctcgct taccttcacc atgactgcag cccacgaata attcaccggg     180

acgtaaaatc aaagaatata ctcctcgaca aagattatga ggcgcatctt acagacttcg     240

gcatcgctaa gagcttatgt gtctcgaaga ctcacacgtc aacctacgtc atgggcacta     300

ttggttacat tgatcctgag tacgcccgca cctcccgcct caacgagaag tctgatgtct     360

acagctacgg catcgttctg ctggagctgc tgaccggcaa gaagccagtg gacaacgagt     420

gcaatctcca tcacttgatc ctatcgaaga cggcgagcaa cgaggtcatg gagacggtgg     480

accccgacgt gggagacacc tgcaaggacc tgggcgaggt gaagaagctg ttccagctgg     540

cgctcctctg caccaagcgg cagccctcgg accggccgac gatgcacgag gtggtgcgcg     600

tccttgactg cctggtgaac ccggagccgc cgccgcagcc gcagcagcag cagcagaagg     660

cgcacgcgca ccaccagctg ccg                                             683
```

<210> 37
<211> 610
<212> DNA
<213> partial maize ERECTA

<400> 37

cttcggcatc gctaagagct tatgtgtctc gaagactcac acgtcaacct acgtcatggg 60

cactattggt tacattgatc ctgagtacgc ccgcacctcc cgcctcaacg agaagtctga 120

tgtctacagc tacggcatcg ttctgctgga gctgctgacc ggcaagaagc cagtggacaa 180

cgagtgcaat ctccatcact tgatcctatc gaagacggcg agcaacgagg tcatggagac 240

ggtggacccc gacgtgggag acacctgcaa ggacctgggc gaggtgaaga agctgttcca 300

gctggcgctc ctctgcacca agcggcagcc ctcggaccgg ccgacgatgc acgaggtggt 360

gcgcgtcctt gactgcctgg tgaacccgga gccgccgccg cagccgcagc agcagcagca 420

gaaggcgcac gcgcaccacc agctgccgcc gcagccgtcg ccgccggcct acgtcgacga 480

gtacgtcagc ctgcggggca ccggcgccct ctcctgcgcc aactcgtcca gcacctcgga 540

cgccgagctg ttcctcaagt tcggcgaggc catctcgcag aacatggtgt aggggaagac 600

gtagacgttc 610

<210> 38
<211> 208
<212> DNA
<213> partial maize ERECTA

<220>
<221> misc_feature
<222> (138)..(138)
<223> not determined

<400> 38

gcaagccagt gagcaatggt ccacccaagc tgggatcctt catatgaaca tggctcttca 60

tgtctttgat gatataatga ggatgactga gaacttgagt gagaaataca tcattggata 120

cggggcatca agtactgntt ataaatgtgt tctaaagaat tgcaaaccag tggcaataaa 180

aaagctgtat gcccactacc ctcagagc 208

<210> 39
<211> 634
<212> DNA
<213> partial maize ERECTA

<400> 39

```
gaccgggacg taaaatcaaa gaatatactc ctcgacaaag attatgaggc gcatcttaca      60
gacttcggca tcgctaagag cttatgtgtc tcgaagactc acacgtcaac ctacgtcatg     120
ggcactattg gttacattga tcctgagtac gcccgcacct cccgcctcaa cgagaagtct     180
gatgtctaca gctacggcat cgttctgctg gagctgctga ccggcaagaa gccagtggac     240
aacgagtgca atctccatca cttgatccta tcgaagacgg cgagcaacga ggtcatggag     300
acggtggacc ccgacgtggg agacacctgc aaggacctgg gcgaggtgaa gaagctgttc     360
cagctggcgc tcctctgcac caagcggcag ccctcggacc ggccgacgat gcacgaggtg     420
gtgcgcgtcc ttgactgcct ggtgaacccg gagccgccgc cgcagccgca gcagcagcag     480
cagaaggcgc acgcgcacca ccagctgccg ccgcagccgt cgccgccggc ctacgtcgac     540
gagtacgtca gcctgcgggg caccggcgcc ctctcctgcg ccaactcgtc cagcacctcg     600
gacgccgagc tgttcctcaa gttcggcgag gcca                                 634
```

<210> 40
<211> 558
<212> DNA
<213> partial maize ERECTA

<400> 40

actttgatgcc ccgtatccaa tgatgtattt ctcactcaag ttctcagtca tcctcattat 60

atcatcaaag acatgaagag ccatgttcat atgaaggatc accagcttgg gtggaccatt 120

gctcactggc ttgcttacag tggcatcttt aaaagcaggt ggatggtgtg gcctgcatac 180

agctactaag atcatcagga ggataacaag tccacccaca gcaacaccaa ttatggcagc 240

ctttgagatt ggcggtttgt ctcggtggcc agtggaacga catgaagaac caagccaata 300

tccacagagt ccaggattac ctaaaaagct gtcatgtgaa aaccgtgtga agttgttgtc 360

agtagggaca gcaccagcca aattattgta tgacacattt aagatattga ggctgaagca 420

gttcatcaga gaagagacat cgccagttat attgttgttt tccagtttta gcaacatcag 480

gttttgcagc attccaagtt cttgaggaat cagaccacca agatgattat aggataaatc 540

aatctccatg acacttct 558

<210> 41
<211> 429
<212> DNA
<213> partial maize ERECTA

<400> 41

tacttgatgc cccgtatcca atgatgtatt tctcactcaa gttctcagtc atcctcatta 60

tatcatcaaa gacatgaaga gccatgttca tatgaaggat caccagcttg ggtggaccat 120

tgctcactgg cttgcttaca gtggcatctt taaaagcagg tggatggtgt ggcctgcata 180

cagctactaa gatcatcagg aggataacaa gtccacccac agcaacacca attatggcag 240

cctttgagat tggcggtttg tctcggtggc cagtggaacg acatgaagaa ccaagccaat 300

atccacagag tccaggatta cctaaaaagc tgtcatgtga aaaccgtgtg aagttgttgt 360

cagtagggac agcaccagcc aaattattgt atgacacatt taagatattg aggctgaagc 420

agttcatca 429

<210> 42

<211> 556
<212> DNA
<213> partial maize ERECTA

<400> 42

```
acatgcaagt caacaggtta actggatcga taccaccaga gctaggaaat atgtcaacac    60
ttcattacct agaactgaat gataatcaac ttactgggtc aattccacca gagcttggaa   120
ggctaacagg cttgtttgac ctgaaccttg cgaataacca ccttgaagga ccaattcctg   180
acaacctaag ttcatgtgtg aatctcaata gcttcaatgc ttatggcaac aagttaaatg   240
gaaccattcc tcgttcgctg cggaaacttg aaagcatgac ctatttaaat ctttcatcaa   300
atttcataag tggctctatt cctattgagc tatcaaggat caacaatttg gacacgttgg   360
acttatcctg taacatgatg acgggtccaa ttccatcatc cattggcaac ctagagcatc   420
tattgaggct taacttgagc aagaatgatc tagttggatt catccctgcg gagtttggta   480
atttgagaag tgtcatggag attgatttat cctataatca tcttggtggt ctgattcctc   540
aagaacttgg aatgct                                                   556
```

<210> 43
<211> 683
<212> DNA
<213> partial maize ERECTA

<400> 43

```
gacgttggga acctcctctt ttatgcttta tggagagtgg cagcttatgg gatgttttac    60
atgaaggctc atccaagaag aacaaacttg actgggtgac tcgcctacgg atcgctcttg   120
gtgcagctca aggcctcgct taccttcacc atgactgcag cccacgaata attcaccggg   180
acgtaaaatc aaagaatata ctcctcgaca aagattatga ggcgcatctt acagacttcg   240
```

gcatcgctaa gagcttatgt gtctcgaaga ctcacacgtc aacctacgtc atgggcacta 300

ttggttacat tgatcctgag tacgcccgca cctcccgcct caacgagaag tctgatgtct 360

acagctacgg catcgttctg ctggagctgc tgaccggcaa gaagccagtg gacaacgagt 420

gcaatctcca tcacttgatc ctatcgaaga cggcgagcaa cgaggtcatg gagacggtgg 480

accccgacgt gggagacacc tgcaaggacc tgggcgaggt gaagaagctg ttccagctgg 540

cgctcctctg caccaagcgg cagccctcgg accggccgac gatgcacgag gtggtgcgcg 600

tccttgactg cctggtgaac ccggagccgc cgccgcagcc gcagcagcag cagcagaagg 660

cgcacgcgca ccaccagctg ccg 683

<210> 44
<211> 2315
<212> DNA
<213> maize ERECTA

<400> 44

acatgcaagt caacaggtta actggatcga taccaccaga gctaggaaat atgtcaacac 60

ttcattacct agaactgaat gataatcaac ttactgggtc aattccacca gagcttggaa 120

ggctaacagg cttgtttgac ctgaaccttg cgaataacca ccttgaagga ccaattcctg 180

acaacctaag ttcatgtgtg aatctcaata gcttcaatgc ttatggcaac aagttaaatg 240

gaaccattcc tcgttcgctg cggaaacttg aaagcatgac ctatttaaat ctttcatcaa 300

atttcataag tggctctatt cctattgagc tatcaaggat caacaatttg gacacgttgg 360

acttatcctg taacatgatg acgggtccaa ttccatcatc cattggcaac ctagagcatc 420

tattgaggct taacttgagc aagaatgatc tagttggatt catccctgcg gagtttggta 480

atttgagaag tgtcatggag attgatttat cctataatca tcttggtggt ctgattcctc 540

aagaacttgg aatgctgcaa aacctgatgt tgctaaaact ggaaaacaac aatataactg 600

gcgatgtctc ttctctgatg aactgcttca gcctcaatat cttaaatgtg tcatacaata 660

atttggctgg tgctgtccct actgacaaca acttcacacg gttttcacat gacagctttt 720

taggtaatcc tggactctgt ggatattggc ttggttcttc atgtcgttcc actggccacc 780

gagacaaacc gccaatctca aaggctgcca taattggtgt tgctgtgggt ggacttgtta 840

tcctcctgat gatcttagta gctgtatgca ggccacacca tccacctgct tttaaagatg 900

ccactgtaag caagccagtg agcaatggtc cacccaagct ggtgatcctt catatgaaca 960

tggctcttca tgtctttgat gatataatga ggatgactga gaacttgagt gagaaataca 1020

tcattggata cggggcatca agtactgttt ataaatgtgt tctaaagaat tgcaaaccag 1080

tggcaataaa aaagctgtat gcccactacc tgcagagcct taaggaattt gaaactgagc 1140

tcgagactgt tggtagcatc aaacaccgga atctagtcag cctgcaaggg tactcgttgt 1200

cacctgttgg gaacctcctc ttttatgctt atatggagag tggcagctta tgggatgttt 1260

tacatgaagg ctcatccaag aagaacaaac ttgactgggt gactcgccta cggatcgctc 1320

ttggtgcagc tcaaggcctc gcttaccttc accatgactg cagcccacga ataattcacc 1380

gggacgtaaa atcaaagaat atactcctcg acaaagatta tgaggcgcat cttacagact 1440

tcggcatcgc taagagctta tgtgtctcga agactcacac gtcaacctac gtcatgggca 1500

ctattggtta cattgatcct gagtacgccc gcacctcccg cctcaacgag aagtctgatg 1560

tctacagcta cggcatcgtt ctgctggagc tgctgaccgg caagaagcca gtggacaacg 1620

agtgcaatct ccatcacttg atcctatcga agacggcgag caacgaggtc atggagacgg 1680

tggaccccga cgtgggagac acctgcaagg acctgggcga ggtgaagaag ctgttccagc 1740

tggcgctcct ctgcaccaag cggcagccct cggaccggcc gacgatgcac gaggtggtgc 1800

gcgtccttga ctgcctggtg aacccggagc cgccgccgca gccgcagcag cagcagcaga 1860

aggcgcacgc gcaccaccag ctgccgccgc agccgtcgcc gccggcctac gtcgacgagt 1920

acgtcagcct gcggggcacc ggcgccctct cctgcgccaa ctcgtccagc acctcggacg 1980

ccgagctgtt cctcaagttc ggcgaggcca tctcgcagaa catggtgtag gggaagacgt 2040

agacgttcgg tgaggcgcct tgagtgaggc cgattgcagg gagtagtttg actgacattt 2100

tgtgggacgc agcgcaggag attaacatgg gactcagtag ctagggtgtt gttagctgta 2160

aaaagtcatg tgacgcaacg caagagcagc ggagcttctt cctcatcttc tttatccccc 2220

ctctccattt ttctttggtg gtctaactta ctaggaggct gtattgatcc atcatcatct 2280

ctcccgttcc tcttccttat gatcttgtga ctttc 2315

<210> 45
<211> 675
<212> PRT
<213> maize ERECTA

<400> 45

Met Gln Val Asn Arg Leu Thr Gly Ser Ile Pro Pro Glu Leu Gly Asn
1 5 10 15

Met Ser Thr Leu His Tyr Leu Glu Leu Asn Asp Asn Gln Leu Thr Gly
20 25 30

Ser Ile Pro Pro Glu Leu Gly Arg Leu Thr Gly Leu Phe Asp Leu Asn
35 40 45

Leu Ala Asn Asn His Leu Glu Gly Pro Ile Pro Asp Asn Leu Ser Ser
50 55 60

Cys Val Asn Leu Asn Ser Phe Asn Ala Tyr Gly Asn Lys Leu Asn Gly
65 70 75 80

```
Thr Ile Pro Arg Ser Leu Arg Lys Leu Glu Ser Met Thr Tyr Leu Asn
                85                  90                  95

Leu Ser Ser Asn Phe Ile Ser Gly Ser Ile Pro Ile Glu Leu Ser Arg
            100                 105                 110

Ile Asn Asn Leu Asp Thr Leu Asp Leu Ser Cys Asn Met Met Thr Gly
        115                 120                 125

Pro Ile Pro Ser Ser Ile Gly Asn Leu Glu His Leu Leu Arg Leu Asn
    130                 135                 140

Leu Ser Lys Asn Asp Leu Val Gly Phe Ile Pro Ala Glu Phe Gly Asn
145                 150                 155                 160

Leu Arg Ser Val Met Glu Ile Asp Leu Ser Tyr Asn His Leu Gly Gly
                165                 170                 175

Leu Ile Pro Gln Glu Leu Gly Met Leu Gln Asn Leu Met Leu Leu Lys
            180                 185                 190

Leu Glu Asn Asn Asn Ile Thr Gly Asp Val Ser Ser Leu Met Asn Cys
        195                 200                 205

Phe Ser Leu Asn Ile Leu Asn Val Ser Tyr Asn Asn Leu Ala Gly Ala
    210                 215                 220

Val Pro Thr Asp Asn Asn Phe Thr Arg Phe Ser His Asp Ser Phe Leu
225                 230                 235                 240

Gly Asn Pro Gly Leu Cys Gly Tyr Trp Leu Gly Ser Ser Cys Arg Ser
                245                 250                 255
```

Thr Gly His Arg Asp Lys Pro Pro Ile Ser Lys Ala Ala Ile Ile Gly
260 265 270

Val Ala Val Gly Gly Leu Val Ile Leu Leu Met Ile Leu Val Ala Val
275 280 285

Cys Arg Pro His His Pro Pro Ala Phe Lys Asp Ala Thr Val Ser Lys
290 295 300

Pro Val Ser Asn Gly Pro Pro Lys Leu Val Ile Leu His Met Asn Met
305 310 315 320

Ala Leu His Val Phe Asp Asp Ile Met Arg Met Thr Glu Asn Leu Ser
325 330 335

Glu Lys Tyr Ile Ile Gly Tyr Gly Ala Ser Ser Thr Val Tyr Lys Cys
340 345 350

Val Leu Lys Asn Cys Lys Pro Val Ala Ile Lys Lys Leu Tyr Ala His
355 360 365

Tyr Leu Gln Ser Leu Lys Glu Phe Glu Thr Glu Leu Glu Thr Val Gly
370 375 380

Ser Ile Lys His Arg Asn Leu Val Ser Leu Gln Gly Tyr Ser Leu Ser
385 390 395 400

Pro Val Gly Asn Leu Leu Phe Tyr Ala Tyr Met Glu Ser Gly Ser Leu
405 410 415

Trp Asp Val Leu His Glu Gly Ser Ser Lys Lys Asn Lys Leu Asp Trp

420 425 430

Val Thr Arg Leu Arg Ile Ala Leu Gly Ala Ala Gln Gly Leu Ala Tyr
435 440 445

Leu His His Asp Cys Ser Pro Arg Ile Ile His Arg Asp Val Lys Ser
450 455 460

Lys Asn Ile Leu Leu Asp Lys Asp Tyr Glu Ala His Leu Thr Asp Phe
465 470 475 480

Gly Ile Ala Lys Ser Leu Cys Val Ser Lys Thr His Thr Ser Thr Tyr
485 490 495

Val Met Gly Thr Ile Gly Tyr Ile Asp Pro Glu Tyr Ala Arg Thr Ser
500 505 510

Arg Leu Asn Glu Lys Ser Asp Val Tyr Ser Tyr Gly Ile Val Leu Leu
515 520 525

Glu Leu Leu Thr Gly Lys Lys Pro Val Asp Asn Glu Cys Asn Leu His
530 535 540

His Leu Ile Leu Ser Lys Thr Ala Ser Asn Glu Val Met Glu Thr Val
545 550 555 560

Asp Pro Asp Val Gly Asp Thr Cys Lys Asp Leu Gly Glu Val Lys Lys
565 570 575

Leu Phe Gln Leu Ala Leu Leu Cys Thr Lys Arg Gln Pro Ser Asp Arg
580 585 590

```
Pro Thr Met His Glu Val Val Arg Val Leu Asp Cys Leu Val Asn Pro
        595                 600                 605

Glu Pro Pro Pro Gln Pro Gln Gln Gln Gln Gln Lys Ala His Ala His
    610                 615                 620

His Gln Leu Pro Pro Gln Pro Ser Pro Pro Ala Tyr Val Asp Glu Tyr
625                 630                 635                 640

Val Ser Leu Arg Gly Thr Gly Ala Leu Ser Cys Ala Asn Ser Ser Ser
                645                 650                 655

Thr Ser Asp Ala Glu Leu Phe Leu Lys Phe Gly Glu Ala Ile Ser Gln
            660                 665                 670

Asn Met Val
        675
```